# EUROPEAN PATENT SPECIFICATION

(11) **EP 2 089 526 B1**
(45) Date of publication and mention of the grant of the patent: **13.03.2013**
(21) Application number: 07859279.7
(22) Date of filing: 08.11.2007
(51) Int. Cl.: C12N 15/82

(54) **A SET OF SEQUENCES FOR TARGETING EXPRESSION AND CONTROL OF THE POST-TRANSLATIONNAL MODIFICATIONS OF A RECOMBINANT POLYPEPTIDE**
AUF DIE EXPRESSION UND DIE KONTROLLE DER POSTTRANSLATIONALEN MODIFIKATIONEN EINES REKOMBINANTEN POLYPEPTIDS GERICHTETER SATZ VON SEQUENZEN
ENSEMBLE DE SÉQUENCES POUR CIBLAGE D'EXPRESSION ET CONTRÔLE DES MODIFICATIONS POST-TRADUCTION D'UN POLYPEPTIDE DE RECOMBINAISON

(30) Priority: 08.11.2006 US 857524 P
(43) Date of publication of application: 19.08.2009
(73) Proprietor: Centre National de la Recherche Scientifique (CNRS), 75794 Paris Cedex 16 (FR)
(72) Inventor: GOMORD, Véonique, 76000 Rouen (FR); SAINT-JORE-DUPAS, Claude, 76770 Malaunay (FR); BOULAFLOUS, Aurelia, 76821 Mont Saint Aignan Cédex (FR); KIEFFER-MEYER, Marie-Christine, 76130 Mont Saint Aignan (FR); FAYE, Loïc, 76160 Saint Jacques sur Darnetal (FR)
(74) Representative: Novagraaf Technologies
(86) International application number: PCT/IB2007/004234
(87) International publication number: WO 2008/056265

(56) References cited:
- WO-A-01/29242
- WO-A-01/81591
- WO-A-03/078637
- PAGNY S ET AL: "Structural requirements for Arabidopsis beta1,2-xylosyltransferase activity and targeting to the Golgi." PLANT JOURNAL, vol. 33, no. 1, January 2003 (2003-01), pages 189-203, XP002482104 ISSN: 0960-7412 cited in the application
- STRASSER RICHARD ET AL: "Molecular cloning and characterization of Arabidopsis thaliana Golgi alpha-mannosidase II, a key enzyme in the formation of complex N-glycans in plants" PLANT JOURNAL, vol. 45, no. 5, March 2006 (2006-03), pages 789-803, XP002482105 ISSN: 0960-7412 cited in the application
- SAINT-JORE-DUPAS C ET AL: "Protein localization in the plant Golgi apparatus and the trans-Golgi network." CMLS CELLULAR AND MOLECULAR LIFE SCIENCES, vol. 61, no. 2, January 2004 (2004-01), pages 159-171, XP002482106 ISSN: 1420-682X
- ESSL D ET AL: "The N-terminal 77 amino acids from tobacco N-acetylglucosaminyltransf erase I are sufficient to retain a reporter protein in the Golgi apparatus of Nicotiana benthamiana cells" FEBS LETTERS, ELSEVIER, AMSTERDAM, NL, vol. 453, no. 1-2, 18 June 1999 (1999-06-18), pages 169-173, XP004259823 ISSN: 0014-5793
- GOMORD VERONIQUE ET AL: "Protein retention and localization in the endoplasmic reticulum and the Golgi apparatus" BIOCHIMIE (PARIS), vol. 81, no. 6, June 1999 (1999-06), pages 607-618, XP002482107 ISSN: 0300-9084
- GOMORD V ET AL: "Posttranslational modification of therapeutic proteins in plants" CURRENT OPINION IN PLANT BIOLOGY, QUADRANT SUBSCRIPTION SERVICES, GB, vol. 7, no. 2, 1 April 2004 (2004-04-01), pages 171-181, XP002413134 ISSN: 1369-5266
- ALMQUIST KURT C ET AL: "Immunomodulation confers herbicide resistance in plants." PLANT BIOTECHNOLOGY JOURNAL MAY 2004, vol. 2, no. 3, May 2004 (2004-05), pages 189-197, XP002482108 ISSN: 1467-7652 cited in the application
- GOMORD ET AL: "Biopharmaceutical production in plants: problems, solutions and opportunities" TRENDS IN BIOTECHNOLOGY, ELSEVIER PUBLICATIONS, CAMBRIDGE, GB, vol. 23, no. 11, 1 November 2005 (2005-11-01), pages 559-565, XP005123745 ISSN: 0167-7799
- SAINT-JORE-DUPAS CLAUDE ET AL: "Plant N-glycan processing enzymes employ different targeting mechanisms for their spatial arrangement along the secretory pathway" PLANT CELL, vol. 18, no. 11, November 2006 (2006-11), pages 3182-3200, XP002482109 ISSN: 1040-4651

### SEQUENCE LISTING

<110> CENTRE NATIONAL DE LA RECHERCHE SCIENTIFIQUE (CNRS) GOMORD, Véronique SAINT-JORE-DUPAS, Claude BOULAFLOUS, Aurélia KIEFER-MEYER, Marie-Christine FAYE, Loïc
<120> A SET OF SEQUENCES FOR TARGETING EXPRESSION AND CONTROL OF THE POST-TRANSLATIONAL MODIFICATIONS OF A RECOMBINANT POLYPEPTIDE
<130> 51706/PCT
<150> US 60/857,524
   <151> 2006-11-08
<160> 132
<170> PatentIn version 3.3
<210> 1
   <211> 14
   <212> PRT
   <213> Artificial
<220>
   <223> peptidic signal (S1)
<400> 1
   Met Thr Ala Gly Ala Ser Arg Arg Ser Ala Arg Gly Arg Ile
   1 5 10
<210> 2
   <211> 10
   <212> PRT
   <213> Artificial
<220>
   <223> peptidic signal (S1)
<400> 2
   Met Ala Arg Gly Glu Arg Arg Arg Arg Ala
   1 5 10
<210> 3
   <211> 12
   <212> PRT
   <213> Artificial
<220>
   <223> peptidic signal (S1)
<400> 3
   Met Asn Asp Arg Arg Pro Gln Arg Lys Arg Pro Ala
   1 5 10
<210> 4
   <211> 50
   <212> PRT
   <213> Artificial
<220>
   <223> stem sequence S3
<400> 4
   Phe Gln Gly Glu His Lys Glu Ser Leu Tyr Trp Gly Thr Tyr Arg Pro
   1 5 10 15
   His Val Tyr Phe Gly Val Arg Ala Arg Thr Pro Leu Ser Leu Val Ala
   20 25 30
   Gly Leu Met Trp Leu Gly Val Lys Asp Glu Met Tyr Val Met Arg His
   35 40 45
   Phe Cys
   50
<210> 5
   <211> 50
   <212> PRT
   <213> Artificial
<220>
   <223> stem sequence S3
<400> 5
   Phe Gln Gly Asp His Lys Glu Ser Leu Tyr Trp Gly Thr Tyr Arg Pro
   1 5 10 15
   Asn Val Tyr Leu Gly Ile Arg Ala Arg Thr Pro Leu Ser Leu Ile Ala
   20 25 30
   Gly Ile Met Trp Ile Gly Ala Lys Asn Gly Gln Tyr Phe Leu Arg His
   35 40 45
   Val Cys
   50
<210> 6
   <211> 49
   <212> PRT
   <213> Artificial
<220>
   <223> stem sequence S3
<400> 6
   Glu Ser Asp Ala Ser Leu Leu Trp Gly Thr Tyr Arg Pro Gln Ile Tyr
   1 5 10 15
   Phe Gly Leu Arg Pro Arg Leu Pro Gly Ser Leu Leu Thr Gly Leu Ala
   20 25 30
   Trp Phe Gly Leu Gln Asp Tyr Ser Asp Phe Gln His Ile Arg His Gln
   35 40 45
   Cys
<210> 7
   <211> 47
   <212> PRT
   <213> Artificial
<220>
   <223> stem sequence S3
<400> 7
   Glu Arg Ser Asn Arg Leu Phe Trp Gly Thr Tyr Arg Pro Gly Ile Tyr
   1 5 10 15
   Phe Gly Met Lys His Arg Ser Pro Ile Ser Leu Leu Phe Gly Val Met
   20 25 30
   Trp Thr Val Gln Asp Ala Glu Asn Phe Ala Phe Arg His Ser Cys
   35 40 45
<210> 8
   <211> 150
   <212> PRT
   <213> Artificial
<220>
   <223> conjonction S1 and S3
<400> 8
   Met Thr Gly Ala Ser Arg Arg Ser Ala Arg Gly Arg Ile Lys Ser Ser
   1               5                   10                  15
   Ser Leu Ser Pro Gly Ser Asp Glu Gly Ser Ala Tyr Pro Pro Ser Ile
               20                  25                  30
   Arg Arg Gly Lys Gly Lys Glu Leu Val Ser Ile Gly Ala Phe Lys Thr
           35                  40                  45
   Asn Leu Lys Ile Leu Val Gly Leu Ile Ile Leu Gly Ile Ile Val Ile
   50 55 60
   Tyr Phe Val Ile Asn Arg Leu Val Arg His Gly Leu Leu Phe Asp Glu
   65 70 75 80
   Ser Gln Lys Pro Arg Val Ile Thr Pro Phe Pro Ala Pro Lys Val Met
   85 90 95
   Asp Leu Ser Met Phe Gln Gly Glu His Lys Glu Ser Leu Tyr Trp Gly
   100 105 110
   Thr Tyr Arg Pro His Val Tyr Phe Gly Val Arg Ala Arg Thr Pro Leu
   115 120 125
   Ser Leu Val Ala Gly Leu Met Trp Leu Gly Val Lys Asp Glu Met Tyr
   130 135 140
   Val Met Arg His Phe Cys
   145 150
<210> 9
   <211> 21
   <212> PRT
   <213> Artificial
<220>
   <223> transmembrane domain GS2
<220>
   <221> misc_feature
   <222> (1) .. (3)
   <223> Xaa can be any naturally occurring amino acid
<400> 9
   Xaa Xaa Xaa Leu Ala Leu Leu Phe Ile Val Phe Val Cys Val Ser Phe
   1 5 10 15
   Val Phe Trp Asp Arg
   20
<210> 10
   <211> 25
   <212> PRT
   <213> Artificial
<220>
   <223> transmembrane domain GS2
<220>
   <221> misc_feature
   <222> (1)..(2)
   <223> Xaa can be any naturally occurring amino acid
<400> 10
   Xaa Xaa Arg Tyr Leu Leu Ile Leu Ala Ala Val Ala Phe Ile Tyr Ile
   1 5 10 15
   Gln Met Arg Leu Phe Ala Thr Gln Ser
   20 25
<210> 11
   <211> 24
   <212> PRT
   <213> Artificial
<220>
   <223> transmembrane domain GS2
<220>
   <221> misc_feature
   <222> (1)..(3)
   <223> Xaa can be any naturally occurring amino acid
<220>
   <221> misc_feature
   <222> (22)..(24)
   <223> Xaa can be any naturally occurring amino acid
<400> 11
   Xaa Xaa Xaa Leu Gly Ile Leu Phe Ala Val Thr Leu Ser Ile Val Leu
   1 5 10 15
   Met Leu Val Ser Val Xaa Xaa Xaa
   20
<210> 12
   <211> 23
   <212> PRT
   <213> Artificial
<220>
   <223> transmembrane domain GS2
<220>
   <221> misc_feature
   <222> (1)..(2)
   <223> Xaa can be any naturally occurring amino acid
<400> 12
   Xaa Xaa Lys Ile Phe Leu Tyr Met Leu Leu Leu Asn Ser Leu Phe Leu
   1 5 10 15
   Ile Ile Tyr Phe Val Phe His
   20
<210> 13
   <211> 26
   <212> PRT
   <213> Artificial
<220>
   <223> transmembrane domain GS2
<220>
   <221> misc_feature
   <222> (1)..(3)
   <223> Xaa can be any naturally occurring amino acid
<400> 13
   Xaa Xaa Xaa Arg Lys Leu Ser Asn Leu Leu Pro Leu Cys Val Ala Leu
   1               5                   10                  15
   Val Val Ile Ala Glu Ile Gly Phe Leu Gly
               20                  25
<210> 14
   <211> 26
   <212> PRT
   <213> Artificial
<220>
   <223> transmembrane domain GS2
<220>
   <221> misc_feature
   <222> (1)..(3)
   <223> Xaa can be any naturally occurring amino acid
<400> 14
   Xaa Xaa Xaa Arg Lys Val Ser Thr Phe Leu Pro Ile Cys Val Ala Leu
   1               5                   10                  15
   Val Val Ile Ile Glu Ile Gly Phe Leu Cys
               20                  25
<210> 15
   <211> 29
   <212> PRT
   <213> Artificial
<220>
   <223> transmembrane domain GS2
<220>
   <221> misc_feature
   <222> (1)..(2)
   <223> Xaa can be any naturally occurring amino acid
<220>
   <221> misc_feature
   <222> (28)..(29)
   <223> Xaa can be any naturally occurring amino acid
<400> 15
   Xaa Xaa Phe Asn Thr Ile Thr Ile Thr Ile Met Ile Ala Phe Thr Phe
   1               5                   10                  15
   Phe Leu Leu Phe Leu Thr Gly Phe Leu Gln Phe Xaa Xaa
               20                  25
<210> 16
   <211> 29
   <212> PRT
   <213> Artificial
<220>
   <223> transmembrane domain GS2
<220>
   <221> misc_feature
   <222> (1) .. (2)
   <223> Xaa can be any naturally occurring amino acid
<400> 16
<210> 17
   <211> 29
   <212> PRT
   <213> Artificial
<220>
   <223> cytosolic tail GS1
<400> 17
   Met Ala Arg Gly Ser Arg Ser Val Gly Ser Ser Ser Ser Lys Trp Arg
   1               5                   10                  15
   Tyr Cys Asn Pro Ser Tyr Tyr Leu Lys Arg Pro Lys Arg
   20 25
<210> 18
   <211> 39
   <212> PRT
   <213> Artificial
<220>
   <223> cytosolic tail GS1·
<400> 18
   Met Gly Val Phe Ser Asn Leu Arg Gly Pro Arg Ala Gly Ala Thr His
   1 5 10 15
   Asp Glu Phe Pro Ala Thr Asn Gly Ser Pro Ser Ser Ser Ser Ser Pro
   20 25 30
   Ser Ser Ser Ile Lys Arg Lys
   35
<210> 19
   <211> 11
   <212> PRT
   <213> Artificial
<220>
   <223> cytosolic tail GS1
<400> 19
   Met Arg Gly Tyr Lys Phe Cys Cys Asp Phe Arg
   1 5 10
<210> 20
   <211> 39
   <212> PRT
   <213> Artificial
<220>
   <223> cytosolic tail GS1
<400> 20
   Met Gly Val Phe Ser Asn Leu Arg Gly Pro Lys Ile Gly Leu Thr His
   1               5                   10                  15
   Glu Glu Leu Pro Val Val Ala Asn Gly Ser Thr Ser Ser Ser Ser Ser
               20                  25                  30
   Pro Ser Ser Phe Lys Arg Lys
                       35
<210> 21
   <211> 12
   <212> PRT
   <213> Artificial
<220>
   <223> cytosolic tail GS1
<400> 21
   Met Leu Val Met Pro Gln Pro Pro Lys Pro Phe Asn
   1               5                   10
<210> 22
   <211> 29
   <212> PRT
   <213> Artificial
<220>
   <223> cytosolic tail GS1
<400> 22
   Met Ala Arg Gly Ser Arg Ser Val Gly Ser Ser Ser Ser Lys Trp Arg
   1               5                   10                  15
   Tyr Cys Asn Pro Ser Tyr Tyr Leu Lys Arg Pro Lys Arg
               20                  25
<210> 23
   <211> 17
   <212> PRT
   <213> Artificial
<220>
   <223> cytosolic tail GS1
<400> 23 Arg
<210> 24
   <211> 166
   <212> PRT
   <213> Artificial
<220>
   <223> stem sequence GS3
<400> 24
   Arg Ile Asn Leu Ala Arg Glu His Glu Val Glu Val Phe Lys Leu Asn
   1 5 10 15
   Glu Glu Val Ser Arg Leu Glu Gln Met Leu Glu Glu Leu Asn Gly Gly
   20 25 30
   Val Gly Asn Lys Pro Leu Lys Thr Leu Lys Asp Ala Pro Glu Asp Pro
   35 40 45
   Val Asp Lys Gln Arg Arg Gln Lys Val Lys Glu Ala Met Ile His Ala
   50 55 60
   Trp Ser Ser Tyr Glu Lys Tyr Ala Trp Gly Lys Asp Glu Leu Gln Pro
   65 70 75 80
   Arg Thr Lys Asp Gly Thr Asp Ser Phe Gly Gly Leu Gly Ala Thr Met
   85 90 95
   Val Asp Ser Leu Asp Thr Leu Tyr Ile Met Gly Leu Asp Glu Gln Phe
   100 105 110
   Gln Lys Ala Arg Glu Trp Val Ala Ser Ser Leu Asp Phe Asp Lys Asp
   115 120 125
   Tyr Asp Ala Ser Met Phe Glu Thr Thr Ile Arg Val Val Gly Gly Leu
   130 135 140
   Leu Ser Ala Tyr Asp Leu Ser Gly Asp Lys Met Phe Leu Glu Lys Ala
   145 150 155 160
   Lys Asp Ile Ala Asp Arg
   165
<210> 25
   <211> 280
   <212> PRT
   <213> Artificial
<220>
   <223> stem sequence GS3
<400> 25
   Thr Leu Phe His Phe Gly Val Pro Gly Pro Ile Ser Ser Arg Phe Leu
   1 5 10 15
   Thr Ser Arg Ser Asn Arg Ile Val Lys Pro Arg Lys Asn Ile Asn Arg
   20 25 30
   Arg Pro Leu Asn Asp Ser Asn Ser Gly Ala Val Val Asp Ile Thr Thr
   35 40 45
   Lys Asp Leu Tyr Asp Arg Ile Glu Phe Leu Asp Thr Asp Gly Gly Pro
   50 55 60
   Trp Lys Gln Gly Trp Arg Val Thr Tyr Lys Asp Asp Glu Trp Glu Lys
   65 70 75 80
   Glu Lys Leu Lys Ile Phe Val Val Pro His Ser His Asn Asp Pro Gly
   85 90 95
   Trp Lys Leu Thr Val Glu Glu Tyr Tyr Gln Arg Gln Ser Arg His Ile
   100 105 110
   Leu Asp Thr Ile Val Glu Thr Leu Ser Lys Asp Ser Arg Arg Lys Phe
   115 120 125
   Ile Trp Glu Glu Met Ser Tyr Leu Glu Arg Trp Trp Arg Asp Ala Ser
   130 135 140
   Pro Asn Lys Gln Glu Ala Leu Thr Lys Leu Val Lys Asp Gly Gln Leu
   145 150 155 160
   Glu Ile Val Gly Gly Gly Trp Val Met Asn Asp Glu Ala Asn Ser His
   165 170 175
   Tyr Phe Ala Ile Ile Glu Gln Ile Ala Glu Gly Asn Met Trp Leu Asn
   180 185 190
   Asp Thr Ile Gly Val Ile Pro Lys Asn Ser Trp Ala Ile Asp Pro Phe
   195 200 205
   Gly Tyr Ser Ser Thr Met Ala Tyr Leu Leu Arg Arg Met Gly Phe Glu
   210 215 220
   Asn Met Leu Ile Gln Arg Thr His Tyr Glu Leu Lys Lys Asp Leu Ala
   225 230 235 240
   Gln His Lys Asn Leu Glu Tyr Ile Trp Arg Gln Ser Trp Asp Ala Met
   245 250 255
   Glu Thr Thr Asp Ile Phe Val His Met Met Pro Phe Tyr Ser Tyr Asp
   260 265 270
   Ile Pro His Thr Cys Gly Pro Glu
   275 280
<210> 26
   <211> 183
   <212> PRT
   <213> Artificial
<220>
   <223> stem sequence GS3
<400> 26
   Gln Thr Gln Ser Gln Tyr Ala Asp Arg Leu Ser Ser Ala Ile Glu Ser
   1 5 10 15
   Glu Asn His Cys Thr Ser Gln Met Arg Gly Leu Ile Asp Glu Val Ser
   20 25 30
   Ile Lys Gln Ser Arg Ile Val Ala Leu Glu Asp Met Lys Asn Arg Gln
   35 40 45
   Asp Glu Glu Leu Val Gln Leu Lys Asp Leu Ile Gln Thr Phe Glu Ser
   50 55 60
   Ala Leu Leu Ser Pro Met Pro Val Ala Ala Val Val Val Met Ala Cys
   65 70 75 80
   Ser Arg Ala Asp Tyr Leu Glu Arg Thr Val Lys Ser Val Leu Thr Tyr
   85 90 95
   Gln Thr Pro Val Ala Ser Lys Tyr Pro Leu Phe Ile Ser Gln Asp Gly
   100 105 110
   Ser Asp Gln Ala Val Lys Ser Lys Ser Leu Ser Tyr Asn Gln Leu Thr
   115 120 125
   Tyr Met Gln Ile Asn Glu Asp Glu Gly Ser Phe Ser Pro Phe Gln His
   130 135 140
   Leu Asp Phe Glu Pro Val Val Thr Glu Arg Pro Gly Glu Leu Thr Ala
   145 150 155 160
   Tyr Tyr Lys Ile Ala Arg Lys Asp Trp Phe Leu Phe Ser Leu Arg Pro
   165 170 175
   Ser Ser Ser Ile Thr Glu Thr
   180
<210> 27
   <211> 174
   <212> PRT
   <213> Artificial
<220>
   <223> stem sequence GS3
<400> 27
<210> 28
   <211> 181
   <212> PRT
   <213> Artificial
<220>
   <223> stem sequence GS3
<400> 28
   His Ser Ser Ser Phe Ser Pro Glu Gln Ser Gln Pro Pro His Ile Tyr
   1 5 10 15
   His Val Ser Val Asn Asn Gln Ser Ala Ile Gln Lys Pro Trp Pro Ile
   20 25 30
   Leu Pro Ser Tyr Leu Pro Trp Thr Pro Pro Gln Arg Asn Leu Pro Thr
   35 40 45
   Gly Ser Cys Glu Gly Tyr Phe Gly Asn Gly Phe Thr Lys Arg Val Asp
   50 55 60
   Phe Leu Lys Pro Arg Ile Gly Gly Gly Gly Glu Gly Ser Trp Phe Arg
   65 70 75 80
   Cys Phe Tyr Ser Glu Thr Leu Gln Ser Ser Ile Cys Glu Gly Arg Asn
   85 90 95
   Leu Arg Met Val Pro Asp Arg Ile Val Met Ser Arg Gly Gly Glu Lys
   100 105 110
   Leu Glu Glu Val Met Gly Arg Lys Glu Glu Glu Glu Leu Pro Ala Phe
   115 120 125
   Arg Gln Gly Ala Phe Glu Val Ala Glu Glu Val Ser Ser Arg Leu Gly
   130 135 140
   Phe Lys Arg His Arg Arg Phe Gly Gly Gly Glu Gly Gly Ser Ala Val
   145 150 155 160
   Ser Arg Arg Leu Val Asn Asp Glu Met Leu Asn Glu Tyr Met Gln Glu
   165 170 175
   Gly Gly Ile Asp Arg
   180
<210> 29
   <211> 150
   <212> PRT
   <213> Artificial
<220>
   <223> stem sequence GS3
<400> 29
   Arg Leu Asp Asn Ala Ser Leu Val Asp Thr Leu Thr His Phe Phe Thr
   1               5                   10                  15
   Lys Ser Ser Ser Asp Leu Lys Val Gly Ser Gly Ile Glu Lys Cys Gln
               20                  25                  30
   Glu Trp Leu Glu Arg Val Asp Ser Val Thr Tyr Ser Arg Asp Phe Thr
           35                  40                  45
   Lys Asp Pro Ile Phe Ile Ser Gly Ser Asn Lys Asp Phe Lys Ser Cys
       50                  55                  60
   Ser Val Asp Cys Val Met Gly Phe Thr Ser Asp Lys Lys Pro Asp Ala
   65                  70                  75                  80
   Ala Phe Gly Leu Ser His Gln Pro Gly Thr Leu Ser Ile Ile Arg Ser
                   85                  90                  95
   Met Glu Ser Ala Gln Tyr Tyr Gln Glu Asn Asn Leu Ala Gln Ala Arg
               100                 105                 110
   Arg Lys Gly Tyr Asp Ile Val Met Thr Thr Ser Leu Ser Ser Asp Val
   115 120 125
   Pro Val Gly Tyr Phe Ser Trp Ala Glu Tyr Asp Ile Met Ala Pro Val
   130 135 140
   Gln Pro Lys Thr Glu Lys
   145 150
<210> 30
   <211> 205
   <212> PRT
   <213> Artificial
<220>
   <223> stem sequence GS3
<400> 30
   Leu Glu Phe Pro Ser Ala Ser Thr Ser Met Glu His Ser Ile Asp Pro
   1 5 10 15
   Glu Pro Lys Leu Ser Asp Ser Thr Ser Asp Pro Phe Ser Asp Val Leu
   20 25 30
   Val Ala Tyr Lys Lys Trp Asp Phe Glu Val Gly Cys Ala Arg Phe Arg
   35 40 45
   Glu Asn His Lys Asp Ala Ile Leu Gly Asn Val Ser Ser Gly Ser Leu
   50 55 60
   Gln Glu Phe Gly Cys Gly Lys Leu Lys Met Lys His Val Lys Val Leu
   65 70 75 80
   Val Lys Gly Trp Thr Trp Ile Pro Asp Asn Leu Glu Asn Leu Tyr Ser
   85 90 95
   Cys Arg Cys Gly Met Thr Cys Leu Trp Thr Lys Ser Ser Val Leu Ala
   100 105 110
   Asp Ser Pro Asp Ala Leu Leu Phe Glu Thr Thr Thr Pro Pro Leu Gln
   115 120 125
   Arg Arg Val Gly Asp Pro Leu Arg Val Tyr Met Glu Leu Glu Ala Gly
   130 135 140
   Arg Lys Arg Ser Gly Arg Glu Asp Ile Phe Ile Ser Tyr His Ala Lys
   145 150 155 160
   Asp Asp Val Gln Thr Thr Tyr Ala Gly Ser Leu Phe His Asn Asn Arg
   165 170 175
   Asn Tyr His Ile Ser Pro His Lys Asn Asn Asp Val Leu Val Tyr Trp
   180 185 190
   Ser Ser Ser Arg Cys Leu Pro His Arg Asp Arg Leu Ala
   195 200 205
<210> 31
   <211> 150
   <212> PRT
   <213> Artificial
<220>
   <223> stem sequence GS3
<400> 31
   Arg Leu Asp Lys Val Ala Leu Val Asp Thr Leu Thr Asp Phe Phe Thr
   1 5 10 15
   Gln Ser Pro Ser Leu Ser Gln Ser Pro Pro Ala Arg Ser Asp Arg Lys
   20 25 30
   Lys Ile Gly Leu Phe Thr Asp Arg Ser Cys Glu Glu Trp Leu Met Arg
   35 40 45
   Glu Asp Ser Val Thr Tyr Ser Arg Asp Phe Thr Lys Asp Pro Ile Phe
   50 55 60
   Ile Ser Gly Gly Glu Lys Asp Phe Gln Trp Cys Ser Val Asp Cys Thr
   65 70 75 80
   Phe Gly Asp Ser Ser Gly Lys Thr Pro Asp Ala Ala Phe Gly Leu Gly
   85 90 95
   Gln Lys Pro Gly Thr Leu Ser Ile Ile Arg Ser Met Glu Ser Ala Gln
   100 105 110
   Tyr Tyr Pro Glu Asn Asp Leu Ala Gln Ala Arg Arg Arg Gly Tyr Asp
   115 120 125
   Ile Val Met Thr Thr Ser Leu Ser Ser Asp Val Pro Val Gly Tyr Phe
   130 135 140
   Ser Trp Ala Glu Tyr Asp
   145 150
<210> 32
   <211> 49
   <212> PRT
   <213> Artificial
<220>
   <223> GS1-GS2-GS3
<400> 32
   Met Ala Arg Gly Ser Arg Ser Val Gly Ser Ser Ser Ser Lys Trp Arg
   1 5 10 15
   Tyr Cys Asn Pro Ser Tyr Tyr Leu Lys Arg Pro Lys Arg Leu Ala Leu
   20 25 30
   Leu Phe Ile Val Phe Val Cys Val Ser Phe Val Phe Trp Asp Arg Gln
   35 40 45
   Thr
<210> 33
   <211> 99
   <212> PRT
   <213> Artificial
<220>
   <223> GS1-GS2-GS3
<400> 33
   Met Ala Arg Gly Ser Arg Ser Val Gly Ser Ser Ser Ser Lys Trp Arg
   1 5 10 15
   Tyr Cys Asn Pro Ser Tyr Tyr Leu Lys Arg Pro Lys Arg Leu Ala Leu
   20 25 30
   Leu Phe Ile Val Phe Val Cys Val Ser Phe Val Phe Trp Asp Arg Gln
   35 40 45
   Thr Leu Val Arg Glu His Gln Val Glu Ile Ser Glu Leu Gln Lys Glu
   50 55 60
   Val Thr Asp Leu Lys Asn Leu Val Asp Asp Leu Asn Asn Lys Gln Gly
   65 70 75 80
   Gly Thr Ser Gly Lys Thr Asp Leu Gly Arg Lys Ala Thr Lys Ser Ser
   85 90 95
   Lys Asp Val
<210> 34
   <211> 22
   <212> PRT
   <213> Artificial
<220>
   <223> GS1-GS2-GS3
<400> 34
<210> 35
   <211> 68
   <212> PRT
   <213> Artificial
<220>
   <223> GS1-GS2-GS3
<400> 35
   Met Gly Val Phe Ser Asn Leu Arg Gly Pro Arg Ala Gly Ala Thr His
   1 5 10 15
   Asp Glu Phe Pro Ala Thr Asn Gly Ser Pro Ser Ser Ser Ser Ser Pro
   20 25 30
   Ser Ser Ser Ile Lys Arg Lys Leu Ser Asn Leu Leu Pro Leu Cys Val
   35 40 45
   Ala Leu Val Val Ile Ala Glu Ile Gly Phe Leu Gly Arg Leu Asp Lys
   50 55 60
   Val Ala Thr Ser
   65
<210> 36
   <211> 38
   <212> PRT
   <213> Artificial
<220>
   <223> GS1-GS2-GS3
<400> 36
   Met Arg Gly Tyr Lys Phe Cys Cys Asp Phe Arg Tyr Leu Leu Ile Leu
   1 5 10 15
   Ala Ala Val Ala Phe Ile Tyr Ile Gln Met Arg Leu Phe Ala Thr Gln
   20 25 30
   Ser Glu Tyr Ala Asp Arg
   35
<210> 37
   <211> 68
   <212> PRT
   <213> Artificial
<220>
   <223> GS1-GS2-GS3
<400> 37
   Met Gly Val Phe Ser Asn Leu Arg Gly Pro Lys Ile Gly Leu Thr His
   1 5 10 15
   Glu Glu Leu Pro Val Val Ala Asn Gly Ser Thr Ser Ser Ser Ser Ser
   20 25 30
   Pro Ser Ser Phe Lys Arg Lys Val Ser Thr Phe Leu Pro Ile Cys Val
   35 40 45
   Ala Leu Val Val Ile Ile Glu Ile Gly Phe Leu Cys Arg Leu Asp Asn
   50 55 60
   Ala Ser Thr Ser
   65
<210> 38
   <211> 41
   <212> PRT
   <213> Artificial
<220>
   <223> GS1-GS2-GS3
<400> 38
   Met Leu Val Met Pro Gln Pro Pro Lys Pro Phe Asn Thr Ile Thr Ile
   1 5 10 15
   Thr Ile Met Ile Ala Phe Thr Phe Phe Leu Leu Phe Leu Thr Gly Phe
   20 25 30
   Leu Gln Phe Pro Ser Ile Ser Pro Ser
   35 40
<210> 39
   <211> 106
   <212> PRT
   <213> Artificial
<220>
   <223> GS1-GS2-GS3
<400> 39
   Met Ala Arg Gly Ser Arg Ser Val Gly Ser Ser Ser Ser Lys Trp Arg
   1 5 10 15
   Tyr Cys Asn Pro Ser Tyr Tyr Leu Lys Arg Pro Lys Arg Leu Ala Leu
   20 25 30
   Leu Phe Ile Val Phe Val Cys Val Ser Phe Val Phe Trp Cys Val Ser
   35 40 45
   Phe Val Phe Trp Asp Arg Gln Thr Leu Val Arg Glu His Gln Val Glu
   50 55 60
   Ile Ser Glu Leu Gln Lys Glu Val Thr Asp Leu Lys Asn Leu Val Asp
   65 70 75 80
   Asp Leu Asn Asn Lys Gln Gly Gly Thr Ser Gly Lys Thr Asp Leu Gly
   85 90 95
   Arg Lys Ala Thr Lys Ser Ser Lys Asp Val
   100 105
<210> 40
   <211> 72
   <212> DNA
   <213> Artificial
<220>
   <223> primer
<400> 40
   gatccttggg aatgcttgct ctgctcttca tcgttttcgt ttgtgtctct ttcgttttct 60
   gggaccgtca aa 72
<210> 41
   <211> 72
   <212> DNA
   <213> Artificial
<220>
   <223> primer
<400> 41
   ctagtttgac ggtcccagaa aacgaaagag acacaaacga aaacgatgaa gagcagagca 60
   agcattccca ag 72
<210> 42
   <211> 81
   <212> DNA
   <213> Artificial
<220>
   <223> primer
<400> 42
   gatccttggg aatggctgct gctcttgctc tgctcttcat cgttttcgtt tgtgtctctt 60
   tcgttttctg ggaccgtcaa a 81
<210> 43
   <211> 81
   <212> DNA
   <213> Artificial
<220>
   <223> primer
<400> 43
   ctagtttgac ggtcccagaa aacgaaagag acacaaacga aaacgatgaa gagcagagca 60
   agagcagcag ccattcccaa g 81
<210> 44
   <211> 28
   <212> DNA
   <213> Artificial
<220>
   <223> primer
<400> 44
   ggtcactagt atcttcattt ccgagttg 28
<210> 45
   <211> 31
   <212> DNA
   <213> Artificial
<220>
   <223> primer
<400> 45
   ggtcactagt gcgatctgca tattctgact g 31
<210> 46
   <211> 31
   <212> DNA
   <213> Artificial
<220>
   <223> primer
<400> 46
   aacgtctaga atgagagggt acaagttttg c 31
<210> 47
   <211> 29
   <212> DNA
   <213> Artificial
<220>
   <223> primer
<400> 47
   cggggtaccc catgaccgga gctagccgt 29
<210> 48
   <211> 24
   <212> DNA
   <213> Artificial
<220>
   <223> primer
<400> 48
   gactagaaaa ggagtgataa ccct 24
<210> 49
   <211> 35
   <212> DNA
   <213> Artificial
<220>
   <223> primer
<400> 49
   cggggtaccc catgaaatca tcatcattat ctccc 35
<210> 50
   <211> 51
   <212> PRT
   <213> Artificial
<220>
   <223> cytosolic tail sequence (GCSI ER signal)
<400> 50
   Met Thr Gly Ala Ser Arg Arg Ser Ala Arg Gly Arg Ile Lys Ser Ser
   1 5 10 15
   Ser Leu Ser Pro Gly Ser Asp Glu Gly Ser Ala Tyr Pro Pro Ser Ile
   20 25 30
   Arg Arg Gly Lys Gly Lys Glu Leu Val Ser Ile Gly Ala Phe Lys Thr
   35 40 45
   Asn Leu Lys
   50
<210> 51
   <211> 41
   <212> PRT
   <213> Artificial
<220>
   <223> cytosolic tail sequence (human GCSI ER targeting)
<400> 51
   Met Ala Arg Gly Glu Arg Arg Arg Arg Ala Val Pro Ala Glu Gly Val
   1 5 10 15
   Arg Thr Ala Glu Arg Ala Ala Arg Gly Gly Pro Gly Arg Arg Asp Gly
   20 25 30
   Arg Gly Gly Gly Pro Arg Ser Thr Ala
   35 40
<210> 52
   <211> 39
   <212> PRT
   <213> Artificial
<220>
   <223> cytosolic tail sequence
<400> 52
   Met Ala Arg Gly Glu Arg Arg Arg Arg Ala Ala Ala Ala Glu Gly Ala
   1 5 10 15
   Arg Pro Leu Glu Arg Ala Arg Ala Ala Gly Arg Arg Asp Gly Arg Ala
   20 25 30
   Gly Gly Ala Arg Gly Ser Ala
   35
<210> 53
   <211> 36
   <212> PRT
   <213> Artificial
<220>
   <223> cytosolic tail sequence
<400> 53
   Met Ala Arg Gly Glu Arg Arg Arg Arg Gly Ala Pro Val Asp Gly Ala
   1 5 10 15
   Arg Thr Ala Glu Arg Ala Ala Arg Gly Gly Pro Ala Arg Arg Gly Gly
   20 25 30
   Glu Ala Arg Gly
   35
<210> 54
   <211> 42
   <212> PRT
   <213> Artificial
<220>
   <223> cytosolic tail sequence
<400> 54
   Met Ala Ala Arg Thr Arg Ile Ala Asp Ser Gly Gly Gly Ala Arg Ser
   1 5 10 15
   Arg Glu Thr Lys Thr Lys Pro Lys Ser Gly Asn Gly Ala Gln Ser Arg
   20 25 30
   Asn Asn Glu Thr Gln Ser Ser Ser Lys Asn
   35 40
<210> 55
   <211> 39
   <212> PRT
   <213> Artificial
<220>
   <223> cytosolic tail sequence
<400> 55
   Met Gly Arg Arg Arg Lys Arg Val Ala Thr Gly Asp Gly Val Pro Ser
   1 5 10 15
   Pro Arg Lys Glu Glu Lys Ala Pro Ala Pro Pro Arg Lys Glu Lys Lys
   20 25 30
   Lys Lys Thr Asp Ile Gly Lys
   35
<210> 56
   <211> 37
   <212> PRT
   <213> Artificial
<220>
   <223> cytosolic tail sequence
<400> 56
   Met Ala Arg Gln Arg Arg Thr Gln Gly Ala Ala Asp Pro Asn Lys Gly
   1 5 10 15
   Thr Asn Ser Ser Ser Ser Asn Gly Ser Asn Ser Thr Asn Asn Arg Ser
   20 25 30
   Ser Lys Ser Thr Ser
   35
<210> 57
   <211> 51
   <212> PRT
   <213> Artificial
<220>
   <223> cytosolic tail sequence
<400> 57
   Met Ala Lys Lys Lys Val Pro Arg Glu Lys Asn His Ser Gly Gly Thr
   1 5 10 15
   Thr Arg Arg Thr Ser Glu Ser Ser Ser Asn Asn His Ala Asp Ser Lys
   20 25 30
   Arg Gln Ile Arg Ile Lys Leu Asn Glu Lys Arg Lys Arg Gln Glu Pro
   35 40 45
   Gly Ser Lys
   50
<210> 58
   <211> 46
   <212> PRT
   <213> Artificial
<220>
   <223> cytosolic tail sequence
<400> 58
   Met His Arg Glu His Glu Glu Met His Gln Pro Ser Arg Arg Arg Arg
   1 5 10 15
   Pro Pro Arg Glu Val Glu Arg Pro Ser Ala Thr Ile Arg Tyr Glu Pro
   20 25 30
   Val Ala Glu Pro Glu Pro Trp Cys Ser Phe Cys Ser Trp Asp
   35 40 45
<210> 59
   <211> 62
   <212> PRT
   <213> Artificial
<220>
   <223> cytosolic tail sequence
<400> 59
   Met Ser Gly Gly Gly Gly Ser Ser Ser Val Arg Arg Pro Val Ala Ala
   1 5 10 15
   Ala Arg Ser Arg Ser Gly Pro Glu Pro Asp Ala Arg Arg Ala Ala Ala
   20 25 30
   Ala Ala Ala Ala Ala Ala Ala Ala Ala Ala Arg Arg Arg Gly Arg Gly
   35 40 45
   Asp His Gly Pro Leu Arg Leu Met Glu Val Ser Pro Arg Asn
   50 55 60
<210> 60
   <211> 11
   <212> PRT
   <213> Artificial
<220>
   <223> cytosolic tail sequence
<400> 60
   Met Ala Pro Pro Pro Pro Arg Gln Pro Arg Gln
   1 5 10
<210> 61
   <211> 42
   <212> PRT
   <213> Artificial
<220>
   <223> cytosolic tail sequence
<400> 61
   Met Ser Ile Leu Asn Ile Ser Ile Thr Val Leu Cys Ile Ala Ile Ala
   1 5 10 15
   Thr Trp Phe Ser Tyr Lys Gly Tyr Leu Glu Thr Arg Val Asn Thr Pro
   20 25 30
   Tyr Asp Ile Lys Lys Leu Val Thr Ile Ser
   35 40
<210> 62
   <211> 18
   <212> PRT
   <213> Artificial
<220>
   <223> cytosolic domain
<400> 62
   Met Thr Gly Ala Ser Arg Arg Ser Ala Arg Gly Arg Ile Lys Ser Ser
   1 5 10 15
   Ser Leu
<210> 63
   <211> 7
   <212> PRT
   <213> Artificial
<220>
   <223> cytosolic domain - SEQ ID NO: 64 - SEQ ID NO: 2
<400> 63
   Met Ile Lys Ser Ser Ser Leu
   1 5
<210> 64
   <211> 16
   <212> PRT
   <213> Artificial
<220>
   <223> elongated cytosolic domain - SEQ ID NO: 2
<400> 64
   Met Ala Arg Gly Glu Arg Arg Arg Arg Ala Ile Lys Ser Ser Ser Leu
   1 5 10 15
<210> 65
   <211> 14
   <212> PRT
   <213> Artificial
<220>
   <223> cytosolic domain
<400> 65
   Met Thr Ala Gly Ala Ser Arg Arg Ser Ala Arg Gly Arg Ile
   1 5 10
<210> 66
   <211> 18
   <212> PRT
   <213> Artificial
<220>
   <223> cytosolic domain
<400> 66
   Met Thr Gly Ala Ser Ala Ala Ser Ala Ala Gly Ala Ile Lys Ser Ser
   1 5 10 15
   Ser Leu
<210> 67
   <211> 18
   <212> PRT
   <213> Artificial
<220>
   <223> cytosolic domain
<400> 67
   Met Thr Gly Ala Ser Leu Leu Ser Ala Leu Gly Leu Ile Lys Ser Ser
   1 5 10 15
   Ser Leu
<210> 68
   <211> 18
   <212> PRT
   <213> Artificial
<220>
   <223> cytosolic domain
<400> 68
   Met Thr Gly Ala Ser Leu Leu Ser Ala Arg Gly Arg Ile Lys Ser Ser
   1 5 10 15
   Ser Leu
<210> 69
   <211> 18
   <212> PRT
   <213> Artificial
<220>
   <223> cytosolic domain
<400> 69
   Met Thr Gly Ala Ser Arg Arg Ser Ala Leu Gly Leu Ile Lys Ser Ser
   1 5 10 15
   Ser Leu
<210> 70
   <211> 4
   <212> PRT
   <213> Artificial
<220>
   <223> arginine motif sequence
<220>
   <221> misc_feature
   <222> (2)..(3)
   <223> Xaa can be any naturally occurring amino acid
<400> 70
   Arg Xaa Xaa Arg
   1
<210> 71
<400> 71
   000
<210> 72
   <211> 4
   <212> PRT
   <213> Artificial
<220>
   <223> arginine motif sequence
<220>
   <221> misc_feature
   <222> (2)..(3)
   <223> Xaa can be any naturally occurring amino acid
<400> 72
   Arg Xaa Xaa Lys
   1
<210> 73
   <211> 5
   <212> PRT
   <213> Artificial
<220>
   <223> arginine motif sequence
<220>
   <221> misc_feature
   <222> (2)..(4)
   <223> Xaa can be any naturally occurring amino acid
<400> 73
   Arg Xaa Xaa Xaa Lys
   1               5
<210> 74
   <211> 4
   <212> PRT
   <213> Artificial
<220>
   <223> arginine motif sequence
<220>
   <221> misc_feature
   <222> (2) .. (3)
   <223> Xaa can be any naturally occurring amino acid
<400> 74
   Lys Xaa Xaa Arg
   1
<210> 75
   <211> 5
   <212> PRT
   <213> Artificial
<220>
   <223> arginine motif sequence
<220>
   <221> misc_feature
   <222> (2)..(4)
   <223> Xaa can be any naturally occurring amino acid
<400> 75
   Lys Xaa Xaa Xaa Arg
   1               5
<210> 76
   <211> 7
   <212> PRT
   <213> Artificial
<220>
   <223> arginine motif sequence
<220>
   <221> misc_feature
   <222> (3)..(4)
   <223> Xaa can be any naturally occurring amino acid
<220>
   <221> misc_feature
   <222> (6)..(6)
   <223> Xaa can be any naturally occurring amino acid
<400> 76
   Arg Arg Xaa Xaa Arg Xaa Arg
   1 5
<210> 77
   <211> 7
   <212> PRT
   <213> Artificial
<220>
   <223> arginine motif sequence
<220>
   <221> misc_feature
   <222> (3)..(4)
   <223> Xaa can be any naturally occurring amino acid
<220>
   <221> misc_feature
   <222> (6)..(6)
   <223> Xaa can be any naturally occurring amino acid
<400> 77
   Arg Lys Xaa Xaa Arg Xaa Arg
   1 5
<210> 78
   <211> 7
   <212> PRT
   <213> Artificial
<220>
   <223> arginine motif sequence
<220>
   <221> misc_feature
   <222> (3)..(4)
   <223> Xaa can be any naturally occurring amino acid
<220>
   <221> misc_feature
   <222> (6) .. (6)
   <223> Xaa can be any naturally occurring amino acid
<400> 78
   Arg Arg Xaa Xaa Arg Xaa Lys
   1               5
<210> 79
   <211> 7
   <212> PRT
   <213> Artificial
<220>
   <223> arginine motif sequence
<220>
   <221> misc_feature
   <222> (3)..(4)
   <223> Xaa can be any naturally occurring amino acid
<220>
   <221> misc_feature
   <222> (6) .. (6)
   <223> Xaa can be any naturally occurring amino acid
<400> 79
   Arg Arg Xaa Xaa Lys Xaa Arg
   1               5
<210> 80
   <211> 7
   <212> PRT
   <213> Artificial
<220>
   <223> arginine motif sequence
<220>
   <221> misc_feature
   <222> (3)..(4)
   <223> Xaa can be any naturally occurring amino acid
<220>
   <221> misc_feature
   <222> (6)..(6)
   <223> Xaa can be any naturally occurring amino acid
<400> 80
   Lys Arg Xaa Xaa Arg Xaa Arg
   1               5
<210> 81
   <211> 7
   <212> PRT
   <213> Artificial
<220>
   <223> arginine motif sequence
<220>
   <221> misc_feature
   <222> (3)..(4)
   <223> Xaa can be any naturally occurring amino acid
<220>
   <221> misc_feature
   <222> (6)..(6)
   <223> Xaa can be any naturally occurring amino acid
<400> 81
   Lys Lys Xaa Xaa Arg Xaa Arg
   1 5
<210> 82
   <211> 7
   <212> PRT
   <213> Artificial
<220>
   <223> arginine motif sequence
<220>
   <221> misc_feature
   <222> (3) .. (4)
   <223> Xaa can be any naturally occurring amino acid
<220>
   <221> misc_feature
   <222> (6)..(6)
   <223> Xaa can be any naturally occurring amino acid
<400> 82
   Arg Arg Xaa Xaa Lys Xaa Lys
   1 5
<210> 83
   <211> 7
   <212> PRT
   <213> Artificial
<220>
   <223> arginine motif sequence
<220>
   <221> misc_feature
   <222> (3)..(4)
   <223> Xaa can be any naturally occurring amino acid
<220>
   <221> misc_feature
   <222> (6)..(6)
   <223> Xaa can be any naturally occurring amino acid
<400> 83
   Arg Lys Xaa Xaa Lys Xaa Arg
   1               5
<210> 84
   <211> 7
   <212> PRT
   <213> Artificial
<220>
   <223> arginine motif sequence
<220>
   <221> misc_feature
   <222> (3)..(4)
   <223> Xaa can be any naturally occurring amino acid
<220>
   <221> misc_feature
   <222> (6)..(6)
   <223> Xaa can be any naturally occurring amino acid
<400> 84
   Arg Lys Xaa Xaa Arg Xaa Lys
   1               5
<210> 85
   <211> 4
   <212> PRT
   <213> Artificial
<220>
   <223> arginine motif sequence
<400> 85
   Arg Arg Arg Arg
   1
<210> 86
   <211> 4
   <212> PRT
   <213> Artificial
<220>
   <223> arginine motif sequence
<400> 86
   Arg Lys Arg Arg
   1
<210> 87
   <211> 4
   <212> PRT
   <213> Artificial
<220>
   <223> arginine motif sequence
<400> 87
   Arg Arg Lys Arg
   1
<210> 88
   <211> 4
   <212> PRT
   <213> Artificial
<220>
   <223> arginine motif sequence
<400> 88
   Arg Arg Arg Lys
   1
<210> 89
   <211> 4
   <212> PRT
   <213> Artificial
<220>
   <223> arginine motif sequence
<400> 89
   Arg Arg Lys Lys
   1
<210> 90
   <211> 4
   <212> PRT
   <213> Artificial
<220>
   <223> arginine motif sequence
<400> 90
   Arg Lys Lys Arg
   1
<210> 91
   <211> 4
   <212> PRT
   <213> Artificial
<220>
   <223> arginine motif sequence
<400> 91
   Lys Lys Arg Arg
   1
<210> 92
   <211> 38
   <212> DNA
   <213> Artificial
<220>
   <223> primer
<400> 92
   gactctagag cgggaagatg aggcttcggg agccgctc 38
<210> 93
   <211> 58
   <212> DNA
   <213> Artificial
<220>
   <223> primer
<400> 93
   aaggcctacg ctacttgtca tcgtcatctt tgtagtcgca cggtgtcccg aagtccac 58
<210> 94
   <211> 35
   <212> DNA
   <213> Artificial
<220>
   <223> primer
<400> 94
   atcgaaatcg cacgatgaga gggtacaagt tttgc 35
<210> 95
   <211> 35
   <212> DNA
   <213> Artificial
<220>
   <223> primer
<400> 95
   cccatgatgc gatctgcata ttctgactgt gtcgc 35
<210> 96
   <211> 29
   <212> DNA
   <213> Artificial
<220>
   <223> primer
<400> 96
   ggactagtgc actgtcgctg cccgcctgc 29
<210> 97
   <211> 58
   <212> DNA
   <213> Artificial
<220>
   <223> primer
<400> 97
   aaggcctacg ctacttgtca tcgtcatctt tgtagtcgca cggtgtcccg aagtccac 58
<210> 98
   <211> 18
   <212> PRT
   <213> Artificial
<220>
   <223> cytosolic domain
<400> 98
   Met Thr Gly Ala Ser Leu Arg Ser Ala Arg Gly Leu Ile Lys Ser Ser
   1               5                   10                  15
   Ser Leu
<210> 99
   <211> 18
   <212> PRT
   <213> Artificial
<220>
   <223> cytosolic domain
<400> 99
   Met Thr Gly Ala Ser Leu Arg Ser Ala Leu Gly Arg Ile Lys Ser Ser
   1 5 10 15
   Ser Leu
<210> 100
   <211> 18
   <212> PRT
   <213> Artificial
<220>
   <223> cytosolic domain
<400> 100
   Met Thr Gly Ala Ser Arg Leu Ser Ala Arg Gly Leu Ile Lys Ser Ser
   1 5 10 15
   Ser Leu
<210> 101
   <211> 18
   <212> PRT
   <213> Artificial
<220>
   <223> cytosolic domain
<400> 101
   Met Thr Gly Ala Ser Arg Leu Ser Ala Leu Gly Arg Ile Lys Ser Ser
   1 5 10 15
   Ser Leu
<210> 102
   <211> 39
   <212> DNA
   <213> Artificial
<220>
   <223> nucleic sequence encoding SEQ ID N∞1
<400> 102
   atgaccggag ctagccgtcg gagcgcgcgt ggtcgaatc 39
<210> 103
   <211> 30
   <212> DNA
   <213> Artificial
<220>
   <223> nucleic sequence encoding SEQ ID N∞2
<400> 103
   atggctcggg gcgagcggcg gcgccgcgca 30
<210> 104
   <211> 36
   <212> DNA
   <213> Artificial
<220>
   <223> nucleic sequence encoding SEQ ID N∞3
<400> 104
   atgaatgatc gtagaccgca aaggaaacgc ccagca 36
<210> 105
   <211> 147
   <212> DNA
   <213> Artificial
<220>
   <223> nucleic sequence encoding SEQ ID N∞4
<400> 105
   tttcagggcg agcacaaaga gagcttatac tggggaactt atcgtcctca tgtttatttt 60
<210> 106
   <211> 150
   <212> DNA
   <213> Artificial
<220>
   <223> nucleic sequence encoding SEQ ID N∞5
<400> 106
   ttccaaggag atcacaagga aagcttgtat tggggtacct acaggccgaa tgtatatctt 60
   ggaattcgtg caagaacccc gttgtctcta attgctggga taatgtggat tggtgcaaag 120
   aatgggcaat actttcttcg tcatgtttgc 150
<210> 107
   <211> 148
   <212> DNA
   <213> Artificial
<220>
   <223> nucleic sequence encoding SEQ ID N∞6
<400> 107
   tgaatctgac gcatcgctgc tctggggaac ataccgtcca caaatttact ttggccttcg 60
   tcctcgtctt cccggatccc tcttaaccgg cctcgcttgg tttggcctac aagattacag 120
   cgactttcag catattcgac atcagtgc 148
<210> 108
   <211> 142
   <212> DNA
   <213> Artificial
<220>
   <223> nucleic sequence encoding SEQ ID N∞7
<400> 108
   agaacgatct aatcgtctct tctggggtac ttatcgtcct ggaatttatt tcggtatgaa 60
   acacagaagt cccatatctc ttttgtttgg agttatgtgg acagttcaag acgcagaaaa 120
   ctttgccttc cgtcactcat gt 142
<210> 109
   <211> 480
   <212> DNA
   <213> Artificial
<220>
   <223> nucleic sequence encoding SEQ ID N∞8
<400> 109
   atgaccggag ctagccgtcg gagcgcgcgt ggtcgaatca aatcatcatc attatctccc 60
   ggctccgatg aaggaagcgc ttatcctccg agtatccgcc gcggcaaagg caaggaactt 120
   gtatcgatcg gtgctttcaa gacgaatctc aagatattgg ttgggttaat catattaggg 180
   attatagtaa tctacttcgt aatcaatcgg ctagttcgtc acgggttgtt gtttgatgag 240
   tcccagaagc ctagggttat cactcctttt cctgctccga aagtcatgga tttgtcaatg 300
   tttcagggcg agcacaaaga gagcttatac tggggaactt atcgtcctca tgtttatttt 360
   ggagttcgag ctagaactcc attgtccttg gtagctggct tgatgtggct tggtgtcaaa     420
   gatgagatgt atgttatgcg gcatttctgt gaaaactctg atgatttaag tacatttggc 480
<210> 110
   <211> 54
   <212> DNA
   <213> Artificial
<220>
   <223> nucleic sequence encoding SEQ ID N∞9
<400> 110
   cttgctctgc tcttcatcgt tttcgtttgt gtctctttcg ttttctggga ccgt 54
<210> 111
   <211> 69
   <212> DNA
   <213> Artificial
<220>
   <223> nucleic sequence encoding SEQ ID N∞10
<400> 111
   cggtacctcc tcatcttggc tgctgtcgcc ttcatctaca tacagatgcg gctttttgcg 60
   acacagtca 69
<210> 112
   <211> 54
   <212> DNA
   <213> Artificial
<220>
   <223> nucleic sequence encoding SEQ ID N∞11
<400> 112
   ttagggattc tatttgctgt tactttgtct atagttctga tgttggtgtc tgta 54
<210> 113
   <211> 60
   <212> DNA
   <213> Artificial
<220>
   <223> nucleic sequence encoding SEQ ID N∞12
<400> 113
   atttttctgt atatgttact tctcaactct ctctttctca tcatctactt cgtttttcac 60
<210> 114
   <211> 69
   <212> DNA
   <213> Artificial
<220>
   <223> nucleic sequence encoding SEQ ID N∞13
<400> 114
   cgaaaattat cgaatttgtt accactctgc gttgctctgg tagttatcgc tgagatcggg 60
   tttctgggt 69
<210> 115
   <211> 69
   <212> DNA
   <213> Artificial
<220>
   <223> nucleic sequence encoding SEQ ID N∞14
<400> 115
   cgtaaagtct cgaccttttt gccaatctgc gtggctcttg tcgtcattat cgagatcggg 60
   ttcctctgt 69
<210> 116
   <211> 75
   <212> DNA
   <213> Artificial
<220>
   <223> nucleic sequence encoding SEQ ID N∞15
<400> 116
   ttcaacacaa tcaccatcac aatcatgata gctttcacct tcttcctcct cttcctcacc      60
   ggtttcctcc aattc 75
<210> 117
   <211> 60
   <212> DNA
   <213> Artificial
<220>
   <223> nucleic sequence encoding SEQ ID N∞16
<400> 117
   aagcgtcttg ctctgctctt catcgttttc gtttgtgtct ctttcgtttt ctgggaccgt 60
<210> 118
   <211> 87
   <212> DNA
   <213> Artificial
<220>
   <223> nucleic sequence encoding SEQ ID N∞17
<400> 118
   atggcgagag ggagcagatc agtgggtagc agcagcagca aatggaggta ctgcaaccct 60
   tcctattact tgaagcgccc aaagcgt 87
<210> 119
   <211> 117
   <212> DNA
   <213> Artificial
<220>
   <223> nucleic sequence encoding SEQ ID N∞18
<400> 119
   atgggtgttt tctcgaatct tcgaggaccc agagccggag ctacccacga tgaatttccg 60
   gcgaccaatg gctctccttc gtcttcttct tctccatctt catcaatcaa gcgaaaa 117
<210> 120
   <211> 32
   <212> DNA
   <213> Artificial
<220>
   <223> nucleic sequence encoding SEQ ID N∞19
<400> 120
   atgagagggt acaagttttg ctgtgatttc cg 32
<210> 121
   <211> 117
   <212> DNA
   <213> Artificial
<220>
   <223> nucleic sequence encoding SEQ ID N∞20
<400> 121
   atgggtgttt tctccaatct tcgaggtcct aaaattggat tgacccatga agaattgcct 60
   gtagtagcca atggctctac ttcttcttct tcgtctcctt cctctttcaa gcgtaaa 117
<210> 122
   <211> 35
   <212> DNA
   <213> Artificial
<220>
   <223> nucleic sequence encoding SEQ ID N∞21
<400> 122
   atgcttgtaa tgccacagcc acccaaaccc ttcaa 35
<210> 123
   <211> 87
   <212> DNA
   <213> Artificial
<220>
   <223> nucleic sequence encoding SEQ ID N∞22
<400> 123
   atggcgagag ggagcagatc agtgggtagc agcagcagca aatggaggta ctgcaaccct      60
   tcctattact tgaagcgccc aaagcgt 87
<210> 124
   <211> 50
   <212> DNA
   <213> Artificial
<220>
   <223> nucleic sequence encoding SEQ ID N∞23
<400> 124
   atggcaaatc tttggaagaa gcagagattg agagatacag gtttatgtcg 50
<210> 125
   <211> 499
   <212> DNA
   <213> Artificial
<220>
   <223> nucleic sequence encoding SEQ ID N∞24
<400> 125
   tcgtattaat cttgcccgag aacatgaggt tgaagttttt aagctaaatg aagaagtttc 60
   acggttggag cagatgttag aagagcttaa tggtggtgtt ggcaataagc ctttgaagac 120
   tctgaaggat gccccagaag atccagttga taaacagcga aggcagaaag taaaagaggc 180
   aatgatccat gcttggagct cttatgaaaa gtatgcatgg gggaaagatg agcttcagcc 240
   tcggacaaaa gatggcactg atagctttgg tggccttgga gcaactatgg tagattcttt 300
   agatacactc tatataatgg gtctagatga gcagtttcaa aaagccagag agtgggttgc 360
   aagctcattg gatttcgaca aggattatga cgccagtatg tttgagacaa ccataagagt 420
   tgtaggcgga cttcttagtg cgtatgatct ttctggggac aaaatgttcc ttgaaaaggc 480
   taaggatatt gcagacaga 499
<210> 126
   <211> 837
   <212> DNA
   <213> Artificial
<220>
   <223> nucleic sequence encoding SEQ ID N∞25
<400> 126
   actctcttcc acttcggcgt accaggaccg atctcctcac gattccttac ctccagatcc      60
   aatcggatcg tcaagccacg gaagaatatt aatcgccgac ccttaaacga ttccaattca     120
   ggcgccgtcg ttgatatcac aactaaagat ctatacgata ggattgagtt tcttgataca     180
   gatggtggtc catggaaaca aggttggaga gttacgtata aagacgatga gtgggagaaa     240
   gagaagctca aaatcttcgt tgttcctcat tctcataacg atcctggttg gaaattgact     300
   gtagaggagt attatcagag acaatccaga catattcttg acaccattgt tgagacttta     360
   tctaaggatt caagaagaaa gtttatatgg gaggagatgt catatctgga gagatggtgg     420
   agagacgctt cacctaataa acaagaagct ttgactaaat tggttaagga tgggcagcta     480
   gagattgttg gaggtggctg ggttatgaat gatgaggcta attcacatta ttttgccata     540
   attgaacaga tagcagaggg taatatgtgg ctgaatgaca caattggggt tattcctaag     600
   aattcttggg ctatagatcc ctttggctat tcatcaacca tggcttatct tctccggcgt     660
   atgggttttg aaaacatgct tattcaaagg actcattacg agctcaagaa agaccttgcc     720
   cagcataaga atcttgaata tatttggcgt cagagctggg atgctatgga aaccacagat     780
   atctttgttc atatgatgcc gttttattca tacgatatcc cacacacttg tggacca        837
<210> 127
   <211> 550
   <212> DNA
   <213> Artificial
<220>
   <223> nucleic sequence encoding SEQ ID N∞26
<400> 127
   ccagacgcaa tcacagtatg cagatcgcct cagttccgct atcgaatctg agaaccattg      60
   cactagtcaa atgcgaggcc tcatagatga agttagcatc aaacagtcgc ggattgttgc     120
   cctcgaagat atgaagaacc gccaggacga agaacttgtg cagcttaagg atctaatcca     180
   gacgtttgaa agtgccttac tatcacctat gcctgtggct gctgtagtgg ttatggcctg     240
   cagtcgtgca gactatcttg aaaggactgt taaatcagtt ttaacatatc aaactcccgt     300
   tgcttcaaaa tatcctctat ttatatctca ggatggatct gatcaagctg tcaagagcaa     360
   gtcattgagc tataatcaat taacatatat gcagataaat gaggatgaag gctcgttttc     420
   cccttttcag cacttggatt ttgaaccagt ggtcactgaa aggcctggcg aactgactgc     480
   gtactacaag attgcacgta aggactggtt tctcttttct ctgcgtccct ccagctccat     540
   tactgaaaca                                                            550
<210> 128
   <211> 523
   <212> DNA
   <213> Artificial
<220>
   <223> nucleic sequence encoding SEQ ID N∞27
<400> 128
   tagaactgct ttgaatggtt cttctattga cgatgatttg gatggtttag ataaagattt      60
   ggaagctaag cttaatgctt cattgctaag tgtagctaga ggtaatagaa tgtctctaag     120
   gttgcataga aggaaccatt tttcgcctag aaatacggat ctgttcccgg atttggcaaa     180
   agatcgtgtg gttatcgtct tgtatgtgca taatcgggct cagtattttc gagtcacagt     240
   ggaaagtttg tcgaaggtta aaggtataag tgagacattg ttgattgtta gtcatgatgg     300
   ttactttgaa gagatgaata ggattgtgga gagtattaag ttttgtcaag tgaaacagat     360
   tttctcgcct tattcgcctc atatatatcg tactagcttc ccgggtgtga ccctgaatga     420
   ttgtaagaac aagggtgatg aggcaaaggg gcattgtgaa ggtaatcctg atcagtatgg     480
   gaatcatcgg tctccgaaga ttgtatcttt gaagcatcac tgg                       523
<210> 129
   <211> 544
   <212> DNA
   <213> Artificial
<220>
   <223> nucleic sequence encoding SEQ ID N∞28
<400> 129
   tcactcatcg tcgttttcac cggagcagtc acagcctcct catatatacc acgtttcagt      60
   gaataaccaa tcggcgattc agaaaccgtg gccgatctta ccttcttacc tcccatggac     120
   gccgccgcag aggaatctac caactggctc ctgcgaaggt tacttcggga atggatttac     180
   aaagagagtt gacttcctta agccgaggat tggaggagga ggagaaggaa gctggttccg     240
   atgtttttac agtgagacat tacagagttc gatttgtgaa ggaaggaatc tgagaatggt     300
   tccggatcgg attgttatgt cgagaggagg tgagaagtta gaggaagtta tggggaggaa     360
   agaggaggag gagcttcctg cgtttcgaca aggtgcgttt gaggtagcgg aagaggtttc     420
   ttcacggtta ggttttaaga gacaccgtcg ttttggtgga ggagaaggag gtagtgcggt     480
   ttctcggcgg ctggtgaatg atgagatgtt gaatgaatat atgcaagaag gtggaattga     540
   taga                                                                  544
<210> 130
   <211> 450
   <212> DNA
   <213> Artificial
<220>
   <223> nucleic sequence encoding SEQ ID N∞29
<400> 130
   cggctcgata acgcttcttt ggtcgatacg ttaacccatt ttttcaccaa gtcgtcgtcc 60
   gatttgaaag ttgggtcagg aatagagaaa tgccaggagt ggttagagag agtggattca 120
   gttacttatt ctagagattt cactaaagat ccgattttta tctctggtag taacaaggac 180
   ttcaaatcgt gctctgttga ttgtgtaatg ggattcactt cagataagaa acctgatgcg 240
   gcttttggat taagtcatca acctggaaca ctcagtataa tccgttccat ggaatcagca 300
   cagtattacc aagagaataa tcttgctcaa gcacgacgga aaggttatga tattgtgatg 360
   acaactagtc tgtcatcaga tgttcctgtt gggtattttt catgggcgga atatgatatt 420
   atggctccag tgcaaccaaa aacagagaaa 450
<210> 131
   <211> 616
   <212> DNA
   <213> Artificial
<220>
   <223> nucleic sequence encoding SEQ ID N∞30
<400> 131
   tttagaattc ccatctgctt caacttcgat ggagcattca atagacccgg aaccgaaatt      60
   gtctgattca acatcggatc cattcagtga tgttcttgta gcttataaaa aatgggactt     120
   tgaagtgggt tgtgctcggt ttagggagaa tcataaagat gcgattttgg gtaatgttag     180
   ttcgggttct ttacaagaat ttggttgtgg taagcttaag atgaagcatg ttaaggtatt     240
   ggttaaaggg tggacttgga ttccggataa tttggaaaat ttgtattctt gtcgttgtgg     300
   gatgacttgt ttgtggacta aatcatcggt tttggctgat tcacctgatg ctttgttgtt     360
   cgagacaaca actcctccac tacagagacg tgttggagat ccgctccgtg tgtacatgga     420
   gctagaggcc ggaagaaaac gctcaggccg tgaagatata ttcatcagct accatgcgaa     480
   agacgatgtc caaacaactt acgccggttc gctctttcat aataacagaa actatcacat     540
   ctctccacat aaaaacaatg atgttctggt gtattggtct tcctcaagat gcctccctca     600
   cagagatcgt ctagca                                                     616
<210> 132
   <211> 450
   <212> DNA
   <213> Artificial
<220>
   <223> nucleic sequence encoding SEQ ID N∞31
<400> 132
   cggctcgata aagtcgcttt ggttgatacg ttgactgatt tcttcaccca gtctccgtca      60
   ctctcgcagt ctccaccggc gagatccgat cggaagaaga tcggattatt tactgatagg     120
   agctgcgagg agtggttgat gagagaagat tcagttactt actctagaga ttttactaaa     180
   gatccaattt ttatctctgg tggtgaaaag gactttcaat ggtgttctgt ggattgtaca     240
   tttggagata gttcagggaa aacaccagat gctgcgtttg gattaggtca gaaacctgga     300
   actcttagta taatacgttc catggaatca gcacagtatt atccagaaaa tgatcttgca     360
   caggcacgac ggagaggtta tgatatagtg atgaccacta gtctatcatc agatgttcct     420
   gttggatatt tttcgtgggc ggagtatgat                                      450

## Description

### Field of the invention

The present invention relates to the field of recombinant proteins production and is more particularly related to methods for producing recombinant polypeptides that are post-translationally modified in the endoplasmic reticulum (ER) and/or the Golgi apparatus (GA). The present invention provides tools useful for controlling the post-translational modifications of recombinant polypeptides and more generally DNA manipulation tools for plant genetic modification. The present invention also provides processes for producing a recombinant polypeptide involving these tools.

The tools of the present invention include targeting signals allowing the sorting of recombinant polypeptides during their synthesis in a host cell to specific sub-cellular compartments and allowing also a specific designing of said recombinant polypeptides within said sub-cellular compartments. The present invention allows advantageously, for example, an increase of the yield of production of recombinant polypeptides, a limitation or prevention of immunogenicity of the recombinant polypeptides and obtaining therapeutically active recombinant polypeptides that are the exact copy of their natural counterpart.

The present invention relates particularly to the field of reorientation of plants made pharmaceuticals (PMP).

The present invention relates also to the field of immune targeting of plant made pharmaceutical using protein interaction with Single Chain variable fragment (ScFv) fused with targeting signals.

The present invention relates also to the field of reorientation of plant enzymes involved in the post-translational modifications of plant made pharmaceuticals

The present invention relates also to the field of reorientation of heterologous enzymes involved in the post-translational modifications of plant made pharmaceuticals

**Prior Art**

Recombinant DNA technology has enabled the production of heterologous recombinant proteins in host systems. The majority of the early work was directed toward the expression of recombinant therapeutic proteins in prokaryote hosts, mainly in *Escherichia coli.* The advantages of prokaryotes as a production system are the ease with which they can be manipulated genetically, their rapid growth and high expression level of recombinant proteins and the possibility of a large-scale fermentation.

However several post-translational modifications (PTMs), including signal peptide cleavage, propeptide processing, protein folding, disulfide bond formation and glycosylation, might not be carried out in prokaryotes. As a result, complex therapeutic proteins produced in prokaryotes are not always properly folded or processed to provide the desired degree of biological activity. Consequently, microbial expression systems have generally been used for the expression of relatively simple therapeutic proteins, such as insulin, interferon or human growth hormone, which do not require folding or extensive posttranslational processing to be biologically active.

Owing to the limitations of prokaryotes for the production of therapeutic proteins, the biotechnology industry has directed its efforts toward eukaryote hosts, such as mammalian cell cultures, yeast, fungi, insect cells, and transgenic animals. These production systems may suffer, however, of different disadvantages: such as expensive fermentation, low yields, secretion problems, high operating costs, difficulties in scaling up to large volumes and potential contamination by viruses or prions. (Gomord et al. 2004 (ref. 23)):

Plant cells and plant suspension-cultured cells can represent a good alternative : advantageous costs, no pahogenic contamination against human. ( Gomord et al. 2004 (ref 23))

But one of the major problems of all eukaryotic cells is inappropriate Post Transcriptionnal Modifications (PTMs).

Indeed, the vast majority of therapeutic proteins undergo several PTMs, which are the final steps in which genetic information from a gene directs the formation of a functional gene product.

In the present specification, the term "PTM" covers covalent modifications that yield derivatives of individual amino-acid residues for example glycosylation, phosphorylation, methylation, ADP-ribosylation, oxidation and glycation; proteolytic processing by reactions involving the polypeptide backbone; and non-enzymatic modifications, such as deamidation, racemization and spontaneous changes in protein conformation.

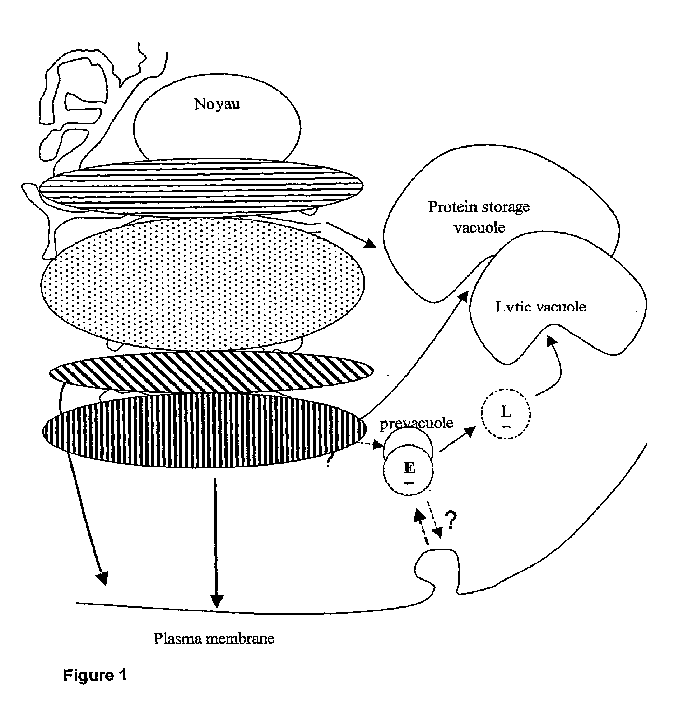

Most of the PTMs depend on the presence of the endomembrane system in the eukaryotic cells. The secretory pathway is composed of the endoplasmic reticulum (ER), the Golgi apparatus (GA), the tonoplast, the lysosomal compartments, the plasma membrane and the extracellular medium as represented in annexed figure 1. Recent studies show that the early compartments (ER and GA) are probably constituted of a membrane continuum (see figure 1). However, the detailed study of the protein transport in the ER, between the ER and the GA and in the Golgi shows that the proteins are subcompartimented in four domains enzymatically distincts in the plant cells (see figure 1: domains blue, yellow, green and orange).

Most therapeutic proteins, including blood proteins, cytokines, immunoglobulins, structural proteins, (growth) hormones, vaccines, enzymes and lysosomal proteins, are co-translationally inserted in the lumen of the ER, and then transported *via* the GA to the lysosomal compartment, the extracellular matrix or the blood stream. Most modifications of therapeutic proteins occur in the secretory pathway but in particular in its early compartments (ER and GA, see Gomord and Faye , 2004 (ref 21).

For example, coagulation factor IX is a vitamin-K-dependent glycoprotein synthesized as a precursor molecule of 461 amino acids in the ER. To get a biologically active factor IX, this precursor undergoes extensive posttranslational modifications in the ER and in the GA, including the cleavage of a signal peptide and a propeptide, disulfide bridge formation, γ-carboxylation of the first 12 glutamic acid residues, partial β-hydroxylation of aspartate 64, N-linked glycosylation at Asparagine (Asn) 157 and Asn 167, O-linked glycosylation at Serine (Ser) 63, Ser 61, Threonine (Thr) 159, Thr 169, Thr 172 and Thr 179, sulfation of Tyrosine (Tyr) 155, and phosphorylation of Ser 158. This is one the most complex maturations of a therapeutic protein ever observed.

More generally, most therapeutic proteins require at least proteolytic cleavage(s) and glycosylation for their bioactivity, pharmacokinetics, stability and solubility.

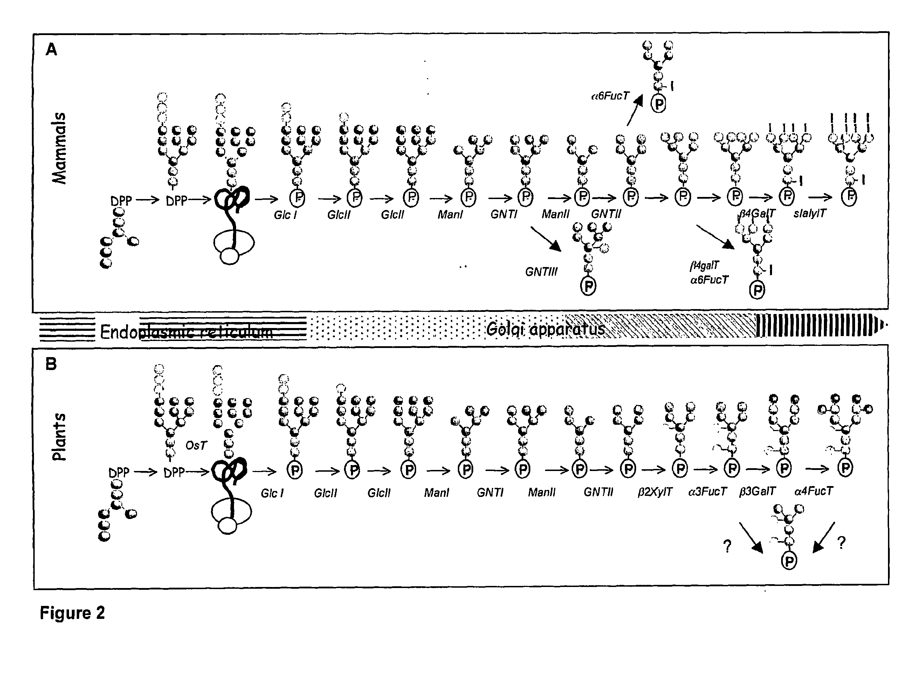

Eukaryotic cells are able to realize most of these modifications. However, these maturations are more generally specific of the host systems. Moreover, post-translational modifications are different from mammalian cell to plant cell. In plants, as in other eukaryotic cells, N-glycosylation starts in the ER, with the cotranslational addition of an oligosaccharide precursor (Glc3Man9GlcNAc2) to specific asparagine residue constitutive of potential N-glycosylation sequences, Asn-X-Ser/Thr. Once transferred on the nascent protein, and while the secreted glycoprotein is transported along the secretory pathway, the oligosaccharide precursor undergoes several maturations involving the removal or addition of sugar residues in the ER and the GA as shown schematically in annexed figure 2. It is only in the late GA that plant and mammalian N-glycan maturation differs, which results in the absence of alpha-(1,6)- linked fucose, beta (1,4)-linked galactose and sialic acids and the presence of bisecting beta-(1,2)-xylose and core alpha-(1,3)-fucose in the N-glycans of PMPs (see annexed figure 2).

In this context, it is very important to be able to control the co-and post-translational maturations in the heterologous expression system.

The structural analysis of plant ER-resident proteins has shown that they bear mainly high-mannose-type N-glycans (Navazio *et al.,* 1997 (ref 41), 1998 (**ref** 42); Pagny *et al.,* 2000 (**ref** 49). These oligosaccharide structures are common to plants and mammals, and therefore are not immunogenic. This observation has suggested a strategy to prevent the association of immunogenic residues such beta-(1,2)-xylose or alpha-(1,3)-fucose to plant-made pharmaceuticals (PMPs) N-glycans. This strategy consists in the storage of recombinant proteins within the ER, i.e., upstream of Golgi cisternae, where immunogenic glyco-epitopes are added to complex plant N-glycans. It was first shown that the addition of H/KDEL sequences at the C-terminal end of a recombinant soluble protein is sufficient for its retention in the plant ER (Gomord *et al.,* 1997 (**ref**. 24), 1999 (**ref**. 22), Saint-Jore-Dupas et al., et 2004 (**ref**. 57)).

Using the same strategy, we have fused a H/KDEL-ER retention sequence to both heavy and light chains of the antibody of two different antibodies. These antibodies present exclusively non immunogenic high-mannose-type N-glycans (Sriraman et al., 2004 (ref. 59); Petrucelli et al., 2006 (**ref**. 51)), indicating a very efficient recycling based on glycan maturation limited to enzymes located in the ER and *cis*-Golgi, such as alpha-mannosidase I (Nebenfuhr *et al*., 1999 (**ref**. 43)). Therefore, preventing the association of immunogenic N-glycans to PMPs through the fusion to ER retention signals is possible.

In contrast, very little is known about the molecular signals responsible for maintaining membrane-bound proteins in the plant ER.

Indeed, only few studies have provided a role of C-terminal dilysine motif (Barrieu and Chrispeels, 1999 (**ref**. 3); Benghezal et al, 2000 (**ref**. 4); McCartney et al., 2004 (**ref**. 35); Reyes et al, 2006 (**ref**. 53)), the length of the Trans-Membrane Domain (TMD) (Brandizzi et al., 2002a (**ref**. 9) or an aromatic amino-acid-enriched ER retrieval signal (McCartney et al., 2004 (**ref**. 35)). The alpha-glucosidase I is the first enzyme involved in the maturation of the N-linked oligosaccharide precursor by removing specifically the distal alpha-(1,2)-linked glucose residue from the oligosaccharide precursor just after its transfer "en block" on the nascent glycoprotein (see Figure 2). The function and consequently the location of this type II membrane protein in the ER is essential. Indeed, in mammalian cells, its defect in neonate induce severe generalised hypotonia and dysmorphic features to fatal outcome at age 74 days (De Praeter et *al.,* 2000 (**ref.** 14)).

Besides glycosylation, proteins travelling downstream the secretory pathway typically undergo specific proteolytic processing, such as targeting signal and regulatory peptides cleavage. As for N-glycosylation, recent evidence in the literature suggests that proteolytic maturations in the secretory pathway is similar in plant and mammalian cells. These maturations are essential for processing of both endogenous and recombinant proteins, but they also make high-yield production of stable, integral polypeptides a challenging task. These proteolytic maturations also depend on the subcellular compartment where the protein is accumulated (Faye et *al.,* 2005 (**ref**. 17.)).

Many plant proteins transported through the secretory pathway are first synthesized as pre-proproteins (also called "not post-translationally modified" in the following description), including an N-terminal cleavable signal peptide - or pre-region - directing the nascent polypeptide chain to the endoplasmic reticulum, and a regulatory pro(poly)peptide - or proregion - involved in the stabilization, targeting, inhibition and/or folding of the mature protein before its translocation to and processing at the final cellular destination. After removal of their signal peptide by a signal peptidase, proteins are released into the ER lumen to be properly assembled and folded, and then translocated to the GA, and eventually further downstream to the different compartments of the secretory system.

Many plant proteins leave the ER as proproteins, with the proregion being proteolytically cleaved downstream along their route through the secretory pathway. It is the case of many vacuolar proteins bearing a C- or N-terminal cleavable sorting signal, which are removed during or after their transport to the vacuole by specific proteases. Asn-specific cysteine proteinases found in vacuoles and cell walls, in particular, would be involved in the processing of several secreted proteins, including seed storage proteins such as 2S albumins and 11S globulins, and proteins with antimicrobial or antifeedant/antidigestive activity such as pathogenesis-related proteins, chitinases, glucanases, lectins and wound-inducible proteinase inhibitors. In mammalian cells, numerous secretory proteins are first synthesized as inactive proprotein precursors, which are then processed post-translationally by soluble- or membrane bound proteases while moving through the secretory pathway.

In many cases, activation of protein precursors by limited proteolysis is carried out by subtilisin-like proprotein convertases, a family of enzymes structurally similar to bacterial subtilisins and yeast kexin. Mammalian proprotein convertases, including notably furin and dibasic-specific kexin-like proteases, typically cleave protein precursors at the consensus sequence (Arg/Lys)-Xn-(Arg/Lys), where X is any amino acid except Cys, and n=0, 2, 4 or 6. In vivo, these enzymes usually found in the trans-Golgi network convert a variety of protein precursors to mature proteins, thereby directly or indirectly contributing to the fine control of important processes such as zymogen activation, gene expression, cell cycle, programmed cell death, intracellular protein targeting, and endocrine/neural functions.

In practice, several examples have been provided illustrating the correct processing of animal proproteins to their biologically active form in plants. An interesting example was provided for human procollagen, shown to be converted to the mature protein after cleavage of its C- and N-terminal propeptidein tobacco cells Lienard et al., 2006 (ref. 32).

As shown by recent studies on the protein processing enzymes of Solanaceae, functional homologues of mammalian processing convertases are implicated in protein maturation along the plant cell's secretory pathway. A kex2p-like protease activity was shown to occur in transgenic tobacco lines able to correctly process the viral antifungal, KP6 killer protoxin. This Golgi-resident, kex2p-like convertase was then shown to exhibit substrate specificity characteristic of yeast kex2p, in contrast with an extracellular proprotein convertase from tomato, LeSBT1, exhibiting distinct specificity, like the mammalian convertase SKI-1 belonging to the same subfamily of processing enzymes (Jansik et al, 2000 (**ref**. 31); Rautengarden et al., 2005 (**ref.** 52)).

More recently, functional homologues of yeast and mammalian CAAX proteases processing the C-terminal tetrapeptidic, CAAX motif of proteins undergoing lipid modification were found in Arabidopsis, again confirming the occurrence of well conserved proteolytic processes along the secretory pathway of eukaryotic cells (Bracha et al, 2002 (**ref**. 7)).

From a practical viewpoint, these conserved processes at the cellular level, along with the overall conserved nature of N-glycosylation, strongly point out the potential of plant systems for the expression and correct processing of various proteins of biological or therapeutic relevance requiring complex post-translational modifications.

In plant, alpha-glucosidase I is primordial in the accumulation of storage proteins, the formation of protein bodies, cell differentiation and cell wall disruptions during *Arabidopsis thaliana* embryo development. Without alpha-glucosidase I activity *Arabidopsis thaliana* seed development is blocked at the heart stage (Boisson et *al.,* 2001 (**ref**. 6)) and cell wall biosynthesis is strongly affected (Gillmor et *al.,* 2002 (**ref.** 19)).

There is still a need in the art of means for producing proteins by genetic recombination, in particular in eukaryotic host, particularly in plant cells, in order to produce designed or engineered post-transcriptionnaly modified recombinant proteins, for example to express in hosts heterologous proteins substantially identical to their natural counterpart and having as less a possible immunogenic properties so as to be usable as therapeutic substances, in particular for humans.

The inventors of the present invention have identified two independent types of signals conferring ER residency on *Arabidopsis thaliana* glucosidase I. Using various deletions or mutants of this glucosidase fused to GFP, they have shown that full length of *A. thaliana* alpha-glucosidase I (hereafter named GCSI) is strictly accumulated in the ER, and contains Arg-based motifs located in the cytosolic tail sufficient for targeting reporter to the ER. However, these functional Arg-based signals are not required to localize the full-length GCSI in this compartments and a second signal has been identified in the stem of this membrane bound ER enzyme.

The inventors of the present invention have identified three independent types of signals conferring GA residency on *Arabidopsis thaliana* glycosyltransferases.

### Description of the invention

The purpose of the present invention is precisely to provide efficient tools responding to this need. The tools of the present invention are in the form of targeting or retention signals and processes involving these targeting signals. The present invention is generally directed to the modification of the post-translational maturation of recombinant polypeptides by different ways using these targeting or retention signals.

Based on their numerous studies on transport and localization of plant or human enzymes, for example of glycosidases and glycosyltransferases, the inventors have identified different peptidic signals specifically involved in the distribution of the membrane proteins between the ER and the GA, in particular in plant cells.

Accordingly, a first aspect of the present invention is to provide particular peptidic signals that allow retention of recombinant polypeptides in specific cell sub-compartment, in particular in plant cells. The sequences and structures of these peptidic signals are described below. The peptidic signals of the present invention are selected from the group consisting SEQ IDn°1, SEQ ID n°4 and SEQ ID n° 8. Hereafter, these peptidic signals may be referred to as "retention signal sequence" or "signal sequence" or "targeting signals" or "peptidic signal".

The retention signal sequences of the present invention specifically target a recombinant polypeptide to the ER and/or the GA compartment membranes, in particular of plant cells. These sequences allow retention of the recombinant polypeptides in the ER and/or in different sub-compartments of the GA or under a membrane bound form in the ER and/or different sub-compartments of the GA. By "target" or "targeting" it is meant that a polypeptide fused with a peptidic signal of the present invention will be localized, i.e. confined, in the ER and/or GA because of this peptific signal.

Maturation and stability of a recombinant polypeptide depend directly on the compartment where the polypeptide is accumulated. For instance, the present inventors have shown that retention of recombinant polypeptides in the ER increases their stability and prevents their N-glycan maturation. They have also shown that expression of a soluble recombinant polypeptide as a membrane polypeptide also increases its stability. Finally, they show that retention of a recombinant polypeptide in production of said recombinant polypeptide and may prevents immunogenicity due to glycan maturation.

The present inventors fused appropriate signals of the present invention to different recombinant polypeptides for different goals achieved and exposed hereafter. Accordingly, another aspect of the present invention is to provide recombinant polypeptides comprising a peptidic signal according to the present invention and a polypeptide, said peptidic signal being fused to the polypeptide. The fusion of the peptidic signal of the present invention may be at the C-terminal or N-terminal extremity of the polypeptide. In other words, said peptidic signal may be linked to the C-terminal or N-terminal end of the polypeptide or protein.

According to the present invention, the recombinant polypeptide may be any recombinant polypeptide having an interest in pharmaceutical or agri-food industry. The "recombinant polypeptide" may also be named "recombinant protein" or "peptide X" or "target protein" in the present specification. However, when the methods of the present invention are disclosed, it is preferred to use "recombinant polypeptide" for designating the recombinant polypeptide to be produced, for example for a pharmaceutical use; and to use "recombinant protein" for designating the a protein involved (see explanations below in the methods description) in the maturation process, i.e. post-translational modification, of the recombinant polypeptide.

According to the present invention, the polypeptide, also named herein "target polypeptide" may be all membrane therapeutical polypeptide, all soluble therapeutical polypeptide that may be expressed as a membrane protein or not, all antibodies and fragments thereof.

For example, the recombinant polypeptide may be selected from the group comprising an enzyme, an antibody or part thereof, a reporter protein, a nucleotide transporter and a therapeutically active polypeptide. Preferably, the recombinant polypeptide is a soluble polypeptide or protein.

Examples of therapeutically active proteins that may be produced with the present invention are: vaccines, allergens, enzymes, blood proteins, hormone, antibodies, antibody-derived fragments. Preferably, the therapeutically active polypeptide is soluble. "Therapeutical polypeptide" or "therapeutically active polypeptide" have the same meaning in the present description and claims [or the soluble part of a membrane bound proteins].

According to the present invention, when the recombinant polypeptide is an enzyme, said enzyme may be a plant or an animal enzyme. According to the present invention, said enzyme may be selected for example from the group comprising glycosidase, glycosyltransferases, protease, kinase, decarboxylase, epimerase, nucleotide-sugar transporter, for example UDP-sugar transporter, GDP-sugar transporter or CMP-Sugar transporter, amidation enzymes and more generally any maturation enzyme present or not in the ER and/or GA of an host cell, for example a plant cell. Glycosidase may for example be those involved in the N- or O-glycosylation. Glycosyltransferases may for example be those involved in the N- or O-glycosylation. Examples of enzymes are cited in the following table

**Table of enzymes**

| | |
|---|---|
| N-glycosylation | Human Beta (1,4) galactosylation |
| | Yeast Mannosyltransferase |
| | OCH1p |
| | Human GNT III |
| O-glycosylation | N-acetylglucosaminyl transferase galactosyltransferase |
| Proteolytic cleavage | Serine proteases |
| | Cyteine proteases |
| amidation | Oxygenese lyase |
| Phosphorylation | phosphorylase |
| Gamma-carboxylation | Gamma carboxylase |
| Proteoglycan modif | Glycotransferase |
| | Glycosidase |
| Sulfation | sulfatase |
| hydroxylation | hydroxylase |
| acetylation | acetylase |
| Cell wall polysaccharides modif. | Glycotransferase |
| | Glycosidase |

By "maturation enzyme" it is referred to any enzyme that participate to the maturation of a recombinant protein in a host cell.

According to the present invention, whatever the protein is, it may be fused with a storage protein or proteins stored in the protein bodies, for example a protein fused to the ZERA®. This is interesting for the recovering of the recombinant polypeptide after production in a host cell. This point is discussed below in the light of the description of the processes of the present invention.

A further aspect of the present invention is to provide the nucleic acid sequences encoding an amino acid sequence selected from the group consisting of SEQ ID N°1, SEQ ID N°4 and SEQ ID N°8. Examples of nucleic acid sequences encoding SEQ ID NOs: 1-31 are given in the annexed listing sequences under references SEQ ID NOs: 102-132. According to the degenerescence of the genetic code, the skill person will easily deduce other suitable nucleic acid sequences that encode also SEQ ID NOs: 1-31.

A further aspect of the present invention is to provide the nucleic acid sequences encoding the recombinant polypeptide according to the present invention.

A further aspect of the present invention is to provide nucleic acid vectors comprising a nucleic acid sequence according the present invention. The nucleic acid vector of the present invention comprises a nucleic acid sequence coding for a peptidic signal of the present invention, wherein said nucleic acid is introduced in the vector in frame with the nucleic acid sequence coding for a polypeptide or protein to produce a recombinant polypeptide or protein containing the "retention signal sequence" at one (or both) extremity (extremities) of said polypeptide.

Any known and suitable method may be use to construct these nucleic acids and nucleic acid vectors. For example, the methods disclosed in (Gomord et al., 1997 (ref. 24) and 1998 (ref. 25), Pagny et al, 2000 (ref. 49) and 2003 (ref. 50), Saint-Jore-Dupas et al 2006 (ref. 58)) may advantageously be used.

A further aspect of the present invention is to provide a plant cell comprising at least one peptidic signal of the present invention and/or at least one recombinant polypeptide of the present invention (i.e. including a peptidic signal of the present invention) and/or at least one nucleic acid sequences encoding a recombinant polypeptide of the present invention and/or at least one nucleic acid vector of the present invention. According to the present invention, said recombinant polypeptide may be a homologous or a heterologous polypeptide. Recombinant polypeptides may be as defined above.

Any known and suitable method may be use to obtain said plant cells. For example, the methods disclosed in Gomord et al., 1997 (**ref**. 24), 1998 (**ref**. 25) , Pagny et al, 2000 (**ref**. 49) and 2003 (**ref**. 50), Saint-Jore-Dupas et al 2006 (**ref**. 58), Saint-Jore et al., 2002 (**ref**. 56) may advantageously be used.

A further aspect of the present invention is to provide a plant comprising at least one peptidic signal of the present invention and/or at least one recombinant protein of the present invention (i.e. including a peptidic signal of the present invention) and/or at least one nucleic acid sequences encoding a recombinant protein of the present invention and/or at least one nucleic acid vector of the present invention. According to the present invention, said recombinant protein may be a homologous or a heterologous protein. Recombinant proteins are as above-defined.

Any known and suitable method may be use to obtain said plant. For example, the methods disclosed in Saint-JoreDupas et al 2006 (Ref 58), Saint-Jore et al, 2002 (**ref**. 56) may advantageously be used.

According to the present invention, the plant may be any suitable plant that allows the production of a recombinant protein. For example, the plant may selected from the group comprising Alfalfa, Arabidospsis thaliana, Nicotiana tabacum, Glycine max, Lycopersicon esculentum, Solanum tuberosum, oriza sativa, zea maize, moss (physcomitrella patens), Lemna minor, Algae (ostreococcus tauri, phaelodactylum), chlamydomonas reinhardtii.

According to the present invention, the plant cell may be issued or derived from any of these plants.

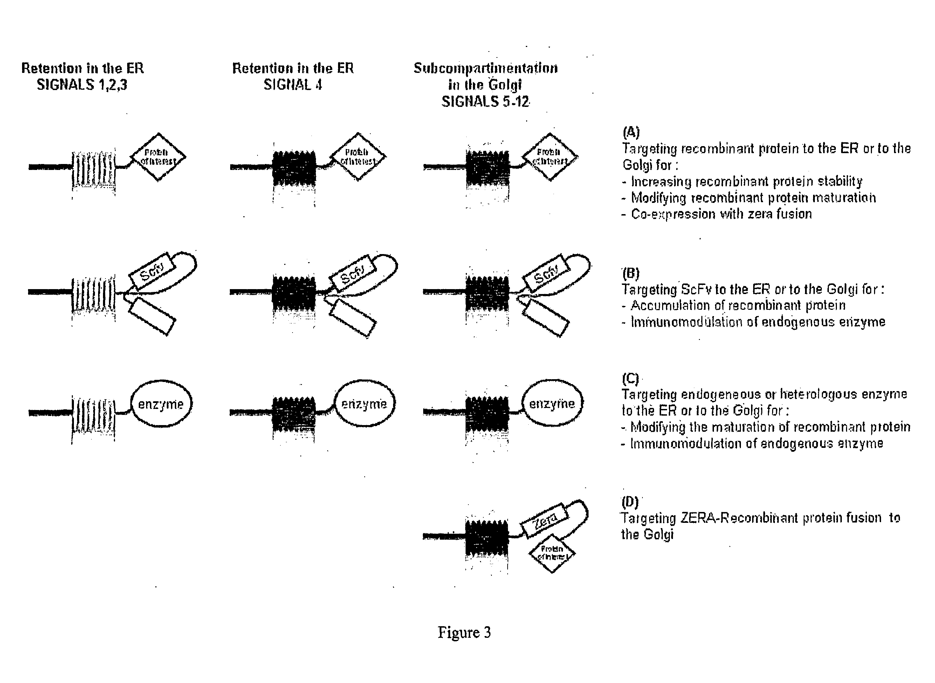

The present inventors provide further a first method for producing a post-translationally modified heterologous polypeptide in host cells that have been transformed with a vector design for the targeted expression of said recombinant polypeptide. This first method is represented schematically in annexed figure 3, line (A).

The present invention has also for subject a method for producing a post-translationally modified polypeptide comprising the steps of:
- transfecting or transforming a plant cell with at least one nucleic acid vector according to the present invention, said vector encoding a recombinant protein which is the polypeptide before being post-translationally modified or a recombinant protein different to said polypeptide;
- growing the transfected plant cell; and
- harvesting the post-translationally modified polypeptide;
   wherein, when said recombinant protein is different to said polypeptide, the method also comprises a step of transfecting said plant cell with at least one nucleic acid vector encoding said polypeptide.

According to the present invention, the process may further comprise a step of screening the cells before the step of growing the transfected or transformed cells. Any method of screening known by the skilled person may be used. For example, by direct selection with a microscope, by electrophoresis SDS page, by immunodetection, by membrane transfert, etc. An example of a method usfull for carring out the screening is disclosed for example in document Gomord et al. 1997 (Ref 24). This step of screening allows selecting the cells that have been transfected or transformed according to the present invention. This step leads to a better yield relating to the mofified polypeptide.

For the present disclosure of the process of the invention, as mentioned above, it is preferred to use "recombinant polypeptide" for designating the recombinant polypeptide to be produced, for example for a pharmaceutical use; and to use "recombinant protein" for designating a protein involved (see explanations below in the second method description) in the maturation process, i.e. post-translational modification, of the recombinant polypeptide.

By this method, the use of the nucleic acid vector encoding the peptidic signal of the present invention fused with the recombinant polypeptide allows the retention of the recombinant polypeptide freely or under a membrane bound form in the ER and/or different sub-compartments of the GA. Part of the heterogeneity observed on recombinant polypeptides with the prior art methods occurs during the transport and maturation through the Golgi. The retention of the recombinant polypeptides in the ER or in the ER-derived protein bodies or in the early Golgi compartments by the use of the peptidic signal of the present invention may reduce this heterogeneity.

A major difficulty is to produce a recombinant protein, which is the exact copy of its natural counterpart. The polypeptide post-translational maturations differ not only from an expression system to the other but also from an organ to the other in the same expression system and from one sub-cellular compartment to the other in a cell constitutive of the same organ.

The addition of a peptidic signal to target a PMP to a specific compartment according to the first method of the present invention may solve this difficulty but this structural modification of the recombinant protein leads of course to a non-native protein. Advantageously, the peptidic signals of the present invention is a real tool allowing further to control the maturation of the recombinant polypeptide, by engineering or designing the ER and GA medium of the host cells. This control allows producing recombinant polypeptides that are more stable within the host cell, that are less immunogenic and that tend to exact copies of their natural counterparts. This is very important for the production of therapeutically active proteins, in particular by plants, usable in human therapy.

The present invention provides several solutions to design the ER and GA environment. These solutions may be used alone or together (combination). Examples of these solutions are schematically represented in annexed figure 3, lines (B) and (C). In all of these solutions, the host cell is transfected or transformed with:
- at least one vector encoding the peptidic signal of the present invention fused with a recombinant protein that is different to the recombinant polypeptide to be produced, and
- with at least one nucleic acid vector encoding said recombinant polypeptide.

Accordingly, a further aspect of the present invention is to provide a second method for producing a post-translationally modified recombinant polypeptide comprising the steps of:
- transfecting or transforming a cell with at least one nucleic acid vector according to the present invention, said vector encoding a recombinant protein which is different to said polypeptide;
- transfecting or transforming said cell with at least one nucleic acid vector encoding said polypeptide;
- growing the transfected cell; and
- harvesting the post-translationally modified polypeptide.

According to the present invention, the process may further comprise a step of screening the cells before the step of growing the transfected or transformed cells. Any method of screening known by the skilled person may be used. Screening methods may be those disclosed above. This step of screening allows selecting the cells that have been transfected or transformed according to the present invention. This step leads to a better yield relating to the mofified polypeptide.

In this second method, the recombinant protein translated in the host cells comprises the peptidic signal of the present invention and this recombinant protein is different to polypeptide to be produced.

In this second method, the recombinant protein play a role in the modulation of the post-translational modification of the recombinant polypeptide to be produced, i.e. it is involved in the maturation process, i.e. post-translational modification, of the recombinant polypeptide.

This role depends on the nature of the selected recombinant protein. The skilled person will easily understand how to select the recombinant protein in order to achieve what he wishes to achieve by using the present invention through the reading of the present description and examples.

According to the present invention, this recombinant protein may be, for example, an enzyme and/or an antibody or part thereof.

According to this second method of the present invention, when the recombinant protein is an antibody or part thereof, it may be such as recognizing and binding specifically the polypeptide to be produced and/or an antibodies or part thereof recognizing and binding specifically with an enzyme involved in the post-translational modification of said polypeptide.

By this method, the antibody or part thereof fused with the peptide signal of the present invention will be localized in the ER and/or GA of the host cell. The role of the recombinant protein is here to capture the recombinant polypeptide in the RE and/or GA. So the instant invention provides a method for producing a post-translationally modified heterologous polypeptide by expressing antibody or antibody fragment in the host cells that have been transformed with an expression vector comprising nucleic acid sequences encoding :
- an ER/GA targeted antibody or antibody fragment, and
- the polypeptide to be produced.

In the context of an antibody or part thereof recognizing and binding specifically said polypeptide to be produced, the present invention allows advantageously to retain a native (not modified by a tag addition consisting of the peptidic signal of the present invention) recombinant protein in the ER and/or the GA of host cells via its binding to a specific antibody or antibody fragment retained in one of these compartments by fusing said antibody or fragment thereof with a peptide signal according to the present invention. For this purpose, ER and/or GA retention peptide signals according to the present invention have been fused to antibodies with specific affinity for the recombinant polypeptide with the ultimate goal to control or to modulate the maturation of recombinant protein.

In the context of an antibody or part thereof recognizing and binding specifically with an enzyme involved in the post-translational modification of said polypeptide, the antibody or part thereof preferably modulates said enzyme. Here, an application of the present invention is to specifically use inactivating antibodies to the ER and/or GA to inactivate specific enzymes located in these compartments. For this purpose, ER and/or GA retention peptide signals according to the present invention have been fused to antibodies with specific affinity for the catalytic domain of ER or GA enzymes with the ultimate goal to inactivate endogenous enzyme to control or to modulate the maturation of recombinant protein. The role of the recombinant protein is here to capture an enzyme involved in the maturation of the recombinant polypeptide. The present invention allows therefore to "immuno-modulate" endogeneous enzymes within the host cell.

According to the present invention, the second method may further be used in order to :
1) to complement the host cell with heterologous enzymes, by using at least one vector according to the present invention encoding a heterologous enzyme fused to a peptidic signal of the present invention
   and/ or
2) to relocalize endogeneous enzymes for optimizing the production capacity of host cells by using at least one vector according to the present invention encoding said endogenous enzyme fused to a peptidic signal of the present invention.

The capacity of a heterologous or homologous enzyme to modify the maturation of recombinant protein will depend on its localization in the cell. Then, in order to modify the enzymatic equipment of a host cell, it is preferable to target the appropriate enzyme in the "good" compartment. So the present invention provides a method for producing a post-translationally modified heterologous polypeptide by expressing maturation enzymes in the host cells that have been transformed with (an) expression vector(s) containing nucleic acid sequences encoding:
1) a recombinant protein that is a targeted maturation enzyme, i.e. a maturation enzyme fused with a peptide signal of the present invention, and
2) the recombinant polypeptide to be produced.

Accordingly, said recombinant protein may be an endogenous or heterologous enzyme involved in the post-translational modification of said polypeptide. In this example, the present invention allows modifying the enzymatic equipment of the ER and/or the GA in the host cell, in particular in plant cell, according to two separate but juxtaposable strategies: (a) by reorientation of an endogeneous enzyme to ER and/or GA, and (b) by targeting an heterologous enzyme to ER/GA which can be located in different sub-cellular compartments. This modification of the enzymatic equipment of the ER and/or the GA in the host cell is a tool allowing modifying (i.e. designing) the post-translational maturation of the recombinant polypeptide. For example, this modification may allow to improve the stability and/or to control the immunogenicity of the produced recombinant polypeptide. The role of the recombinant protein is here to capture homologous or heterologous enzymes involved in the maturation of the recombinant polypeptide in the ER and/or GA.

The present invention allows therefore advantageously getting a recombinant polypeptide that is a non-immunogenic glycoprotein but also a recombinant polypeptide that is a homogeneous glycoproteins.

According to the present invention, the polypeptide may be any polypeptide needed to be produced by genetic recombination, in particular in plant cells or whole plants. The polypeptide that may be produced with the present invention are for example those cited above in the disclosure of the recombinant polypeptide of the present invention.

According to the present invention, the first and second method may be used simultaneously. In this case, in the second method, the vector nucleic acid vector encoding said polypeptide is also a nucleic acid vector according to the present invention, i.e. encoding the polypeptide fused with the peptidic signal of the present invention. With this embodiment of the present invention, one may target the recombinant polypeptide for maturation for localization in the ER and/or GA and, in the same time design the ER and/or GA for the maturation, i.e. post-translational modification of the recombinant polypeptide. The polypeptide may be as defined above. For example, it may be a therapeutically active protein.

As disclosed herein, according to the present invention, the post-translational modification of the polypeptide is advantageously carried out in the ER and/or GA compartment membranes.

The present inventors are the very first ones to provide such powerful tools for producing designed recombinant polypeptides in a host cell, particularly in a plant cell.

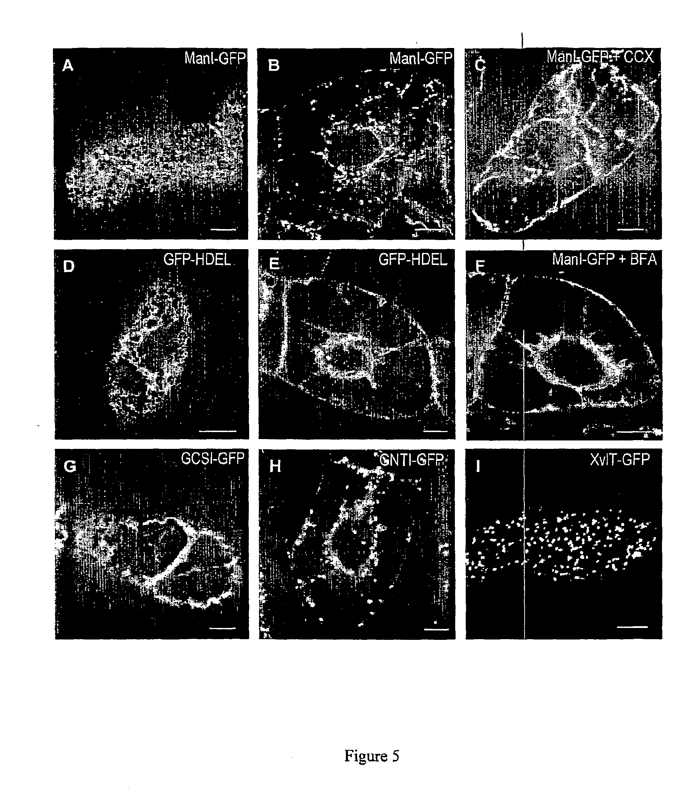

Whatever the method of the present invention that is used (first and/or second method), the polypeptide may be co-expressed with a storage protein. This storage protein may be for example a protein fused to ZERA® or any other suitable protein. Annexed figure 3, line (D) represent schematically the targeting of ZERA®-recombinant protein fusion to the Golgi. For example, protein bodies may be initiated from the ER membrane by a ZERA®-recombinant protein expression in the host cells (see annexed figure 5A). Recombinant glycoproteins accumulated in the ER (see examples below) harbour exclusively high mannose type N-glycan. The present invention provides the peptide signals that allow advantageously combining ER homogeneity and glycan modification, for example for a pharmaceutical glycoprotein, stored in the ER after fusion with a ER retention signal.

Usable methods for obtaining the vectors used in the methods of the present invention are disclosed above.

Usable methods for tranfecting or transforming cells, in particular plant cells are disclosed above.

According to the present invention, the cells are preferably plant cells, for example as defined above, e.g. plant cells are cells issued from a plant selected from the group comprising *Medicago sativa, Arabidopsis thaliana, Nicotiana tabacum, Glycine max, Lycopersicon esculentum, Solanum tuberosum Oriza sativa, Zea mays, Physcomitrella patens, Lemna minor, Ostreococcus tauri, Phaelodactylum.*

Usable methods for growing the transfected cell, or plants obtained there from, and harvesting the post-translationally modified polypeptide are those known by the skilled person in the art. For example, the methods disclosed in lienard et al., 2006 (Ref 32) may advantageously be used.

Others advantages may appear to one skill in the art from the followings non-limiting examples illustrated by annexed figures.

### Brief description of the figures

Figure 1 is a schematic representation of plant cell secretory pathway. The domains of the ER and Golgi continuum are indicated as follows:

▤ ER

▩ ER/cis Golgi

▧ medial Golgi

▥ late Golgi

Figure 2 shows transfert and processing of N-linked glycans on the protein, in the endoplasmic reticulum and Golgi apparatus of plant and mammalian cells. A precursor oligosaccharide assembled onto a lipid carrier is transferred on specific Asn residues constitutive of the nascent polypeptide. The N-glycan is then trimmed off with removal of glucosyl and most mannosyl residues. Differences in the processing of plant and mammalian complex N-glycans are late Golgi maturation events. The ER and Golgi domains are indicated on main arrow as described in Fig 1

Figure 3 shows potential applications of different signals (in dark) in the targeting of recombinant protein (line A), of antibodies or part thereof (ScFv) (line B), of Enzymes (line C) or of Zera®-Fusion (line D).

Figure 4 is a schematic representation of analyzed fusion proteins:
- GCSI-GFP: full length *Arabidopsis thaliana* GCSI fused to GFP,
- GCS90-GFP: the first 90 N-terminal aa of GCSI fused to GFP,
- Δ13GCS90-GFP: GCS90-GFP minus the first 13 N-terminal aa,
- Manl-GFP: full length *Glycine max* Manl fused to GFP,
- Δ19CTMan-GFP: ManI-GFP minus the first 19 N-terminal aa,
- Man99-GFP and Man49-GFP: the first 99 and 49 aa of ManI, respectively, fused to GFP,
- Δ19CTMan49-GFP: Man49-GFP minus the first 19 N-terminal aa,
- ΔCTMan49-GFP: the whole CT was deleted from Man49-GFP,
- MAAAMan49-GFP: the CT of Man49-GFP was replaced by an artificial CT containing 3 Ala residues,
- ManTMD23-GFP and Man99TMD23-GFP: ManI-GFP and Man99-GFP respectively, where the TMD was lengthened from 16 to 23 aa,
- GNTI-GFP: full length *Nicotiana tabacum* GNTI fused to GFP,
- GNT38-GFP: the first 38 N-terminal aa of GNTI fused to GFP,
- XylT-GFP: full length *A. thaliana* XylT fused to GFP,
- XylT35-GFP: the first 35 aa of XylT fused to GFP,
- ST52-mRFP: the first 52 aa of a rat α-2,6-sialyltransferase fused to mRFP,
- GFP-HDEL: a GFP version containing the sporamine signal peptide and a C-terminal HDEL ER retention sequence.

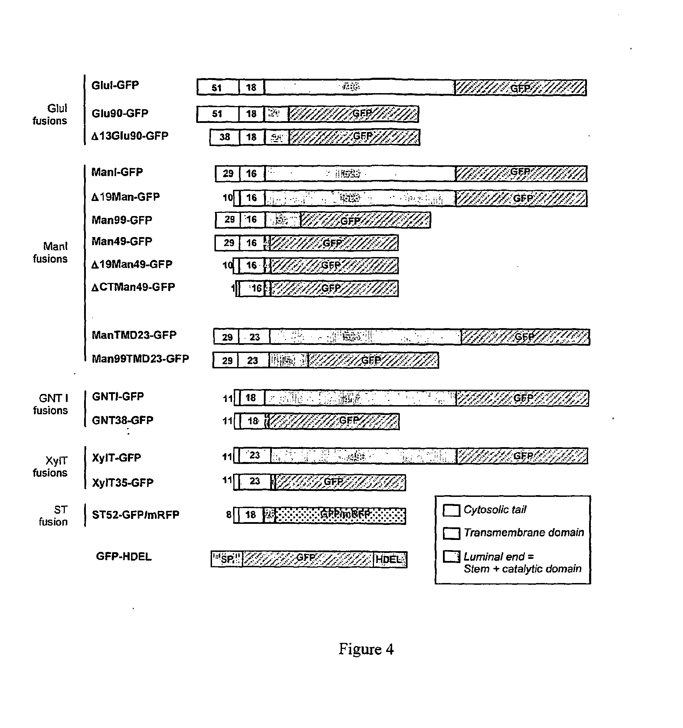

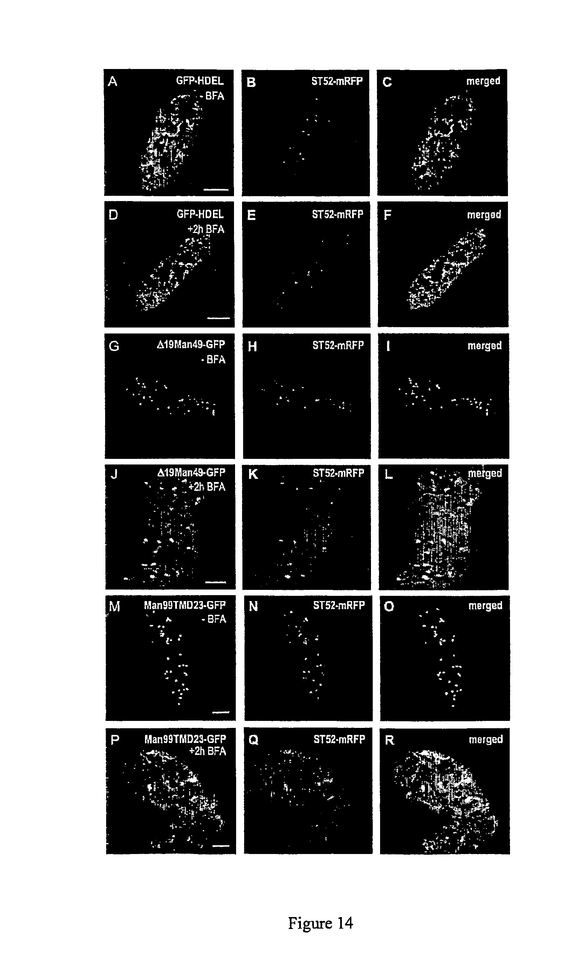

Figure 5 shows localization into the Golgi and/or the ER of a series of GFP fusions to four different members of the N-glycan processing machinery (α-glucosidase I, mannosidase I, N-acetylglucosaminyltransferase I, β-1,2-xylosyltransferase, figure 4) and to the C-terminal HDEL ER retention sequence, after stable expression (3-4 days) in tobacco BY-2 cells, using confocal laser scanning microscopy:
(A,B) ManI-GFP,
(C) ManI-GFP (after a 2h treatment with the protein synthesis inhibitor cycloheximide),
(D,E) GFP-HDEL,
(F) ManI-GFP [after a 2h treatment with BFA (50mg.mL-1)],
(G) GCSI-GFP,
(H) GNTI-GFP,
(I) XylT-GFP,
All bars = 8 µm.

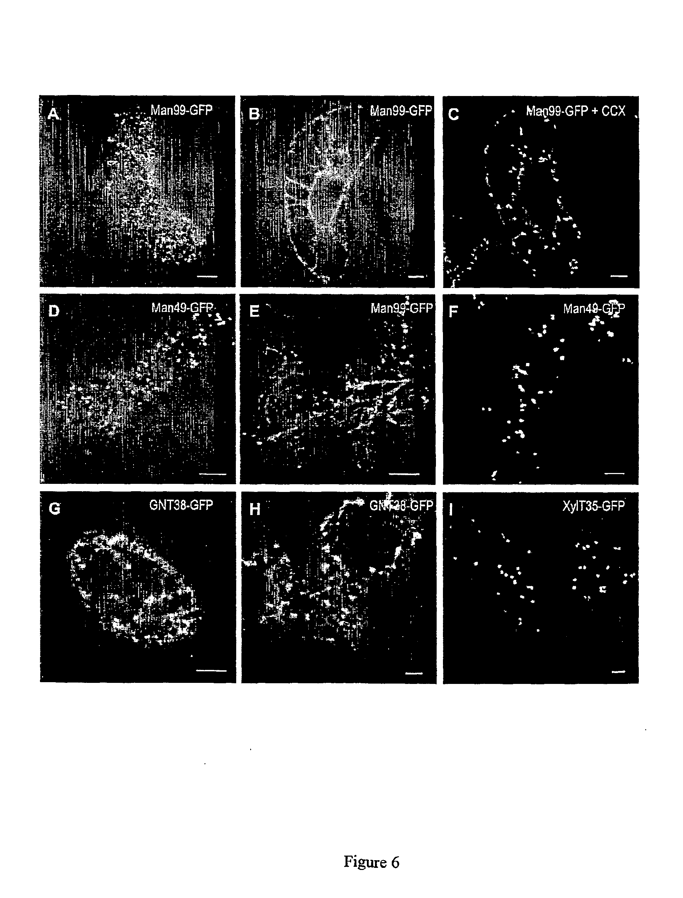

Figure 6 shows localization into the Golgi and/or the ER of a series of GFP fusions to truncated members of the N-glycan processing machinery (Man99, Man49, GNT38, XylT35, figure 4), after stable expression (3-4 days) in tobacco BY-2 cells or transient expression (5 days) in tobacco leaf epidermal cells by leaf infiltration, using confocal laser scanning microscopy:
(A,B) BY-2 suspension cultured cells expression of Man99-GFP,
(C) BY-2 suspension cultured cells expression of Man99-GFP (after a 2h treatment with the protein synthesis inhibitor cycloheximide),
(D) BY-2 suspension cultured cells expression of Man49-GFP, (E-F) Nicotiana leaf epidermal cells expression of Man99-GFP and Man49-GFP, respectively,
(G) BY-2 suspension cultured cells expression of GNT38-GFP (G),
(H) Nicotiana leaf epidermal cells of GNT38-GFP, and
(I) Nicotiana leaf epidermal cells expression of XylT35-GFP.
   All bars = 8 µm.

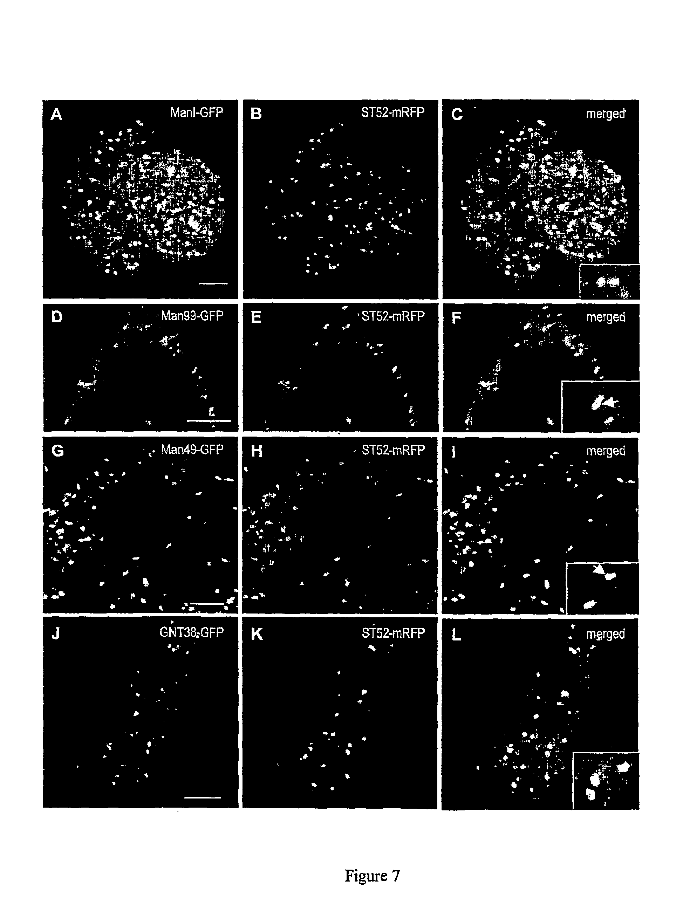

Figure 7 shows localization into the Golgi stacks of a series of GFP fusions to ManI, Man99, Man49 or GNT38 (figure 4) and the trans Golgi marker ST52-mRFP, after stable co-expression (3-4 days) in tobacco BY-2 cells of one or other of these GFP fusions and ST52-mRFP, using confocal laser scanning microscopy:
(A-C) ManI-GFP (A) and ST52-mRFP (B) co-expression in BY-2 suspension-cultured tobacco cells,
(D-F) Man99-GFP (D) and ST52-mRFP (E) co-expression in BY-2 suspension-cultured tobacco cells,
(G-I) Man49-GFP (G) and ST52-mRFP (H) co-expression in BY-2 suspension-cultured tobacco cells,
(J-L) GNT38-GFP (J) and ST52-mRFP (K) co-expression in BY-2 suspension-cultured tobacco cells, and
(C, F, I and L) corresponding merged expression GFP fusions + ST52-mRFP.

Inserts: magnification of selected Golgi stacks (X2,2).

Note that stacks often appear "tri-coloured" (arrows in F and I) with the GFP fusions on one side (green), the ST52-RFP on the other side (red) and a region of overlap between them (yellow).

A-I: Bars = 8 µm; J-L: Bar = 16 µm.

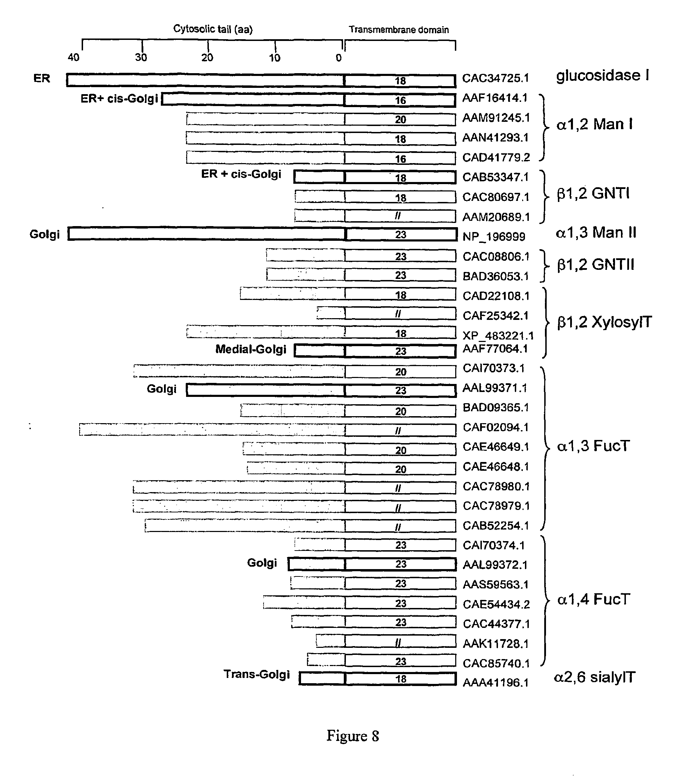

Figure 8 shows a comparison of cytosolic tail and TMD length for plant N-glycosylation enzymes:
(A) Processing of N-linked glycans in the endoplasmic reticulum (ER) and Golgi apparatus of plant cells.
(B) Cytosolic tail and transmembrane domain length of N-glycan processing enzymes that have been cloned from different plant species. Accession numbers are indicated on the right of each schematic representation.

For each membrane protein, the position and the size of the transmembrane domain were estimated from the TmHMM_v2 software (http://www.cbs.dtu.dk/services/TMHMM/).

For some of proteins, the probability to define the position of the TMD is below to 50% (//).

Boxes outlined in bold correspond to N-glycan processing enzymes whose intracellular localization has been studied to date by confocal and/or electronic microscopy.

GCSI: glucosidase I, α1,2 ManI: α1,2-mannosidase I, β1,2 GNTI: , β1,2-N-acetylglucosaminyltransferase I, α1,3 ManII: α1,3-mannosidase II. β1,2 GNTII: β1,2-acetylglucosaminyltransferase II, β1,2 XylosylT: β1,2-xylosyltransferase, α1,3 FucT: α1,3-fucosyltransferase, α1,4 FucT: α1,4-fucosyltransferase, α2,6 sialylT: α2,6-sialyltransferase.

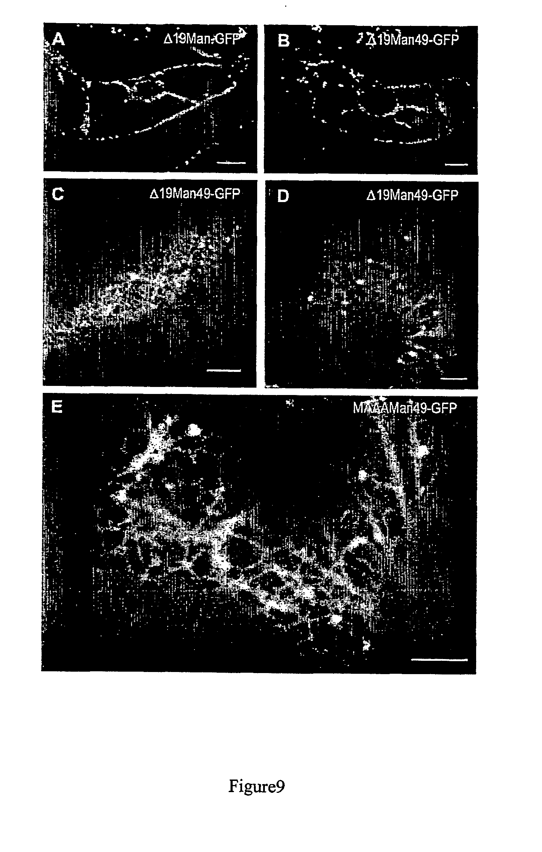

Figure 9 shows localization into the Golgi and the ER of a series of GFP fusions to truncated forms of Manl (Δ19Man, Δ19Man49, figure 3) and to MAAAMan49, after stable expression (3-4 days) in tobacco BY-2 cells or transient expression (5 days) in tobacco leaf epidermal cells by leaf infiltration, using confocal laser scanning microscopy:
(A) BY-2 suspension cultured cells expression of Δ19Man-GFP, (B, C) BY-2 suspension cultured cells expression of Δ19Man49-GFP,
(D) leaf epidermal cells expression of Δ19Man49-GFP,
(E) leaf epidermal cells expression of MAAAMan49-GFP.

A-C: Bars = 16 µm; D, E: Bars = 8 µm

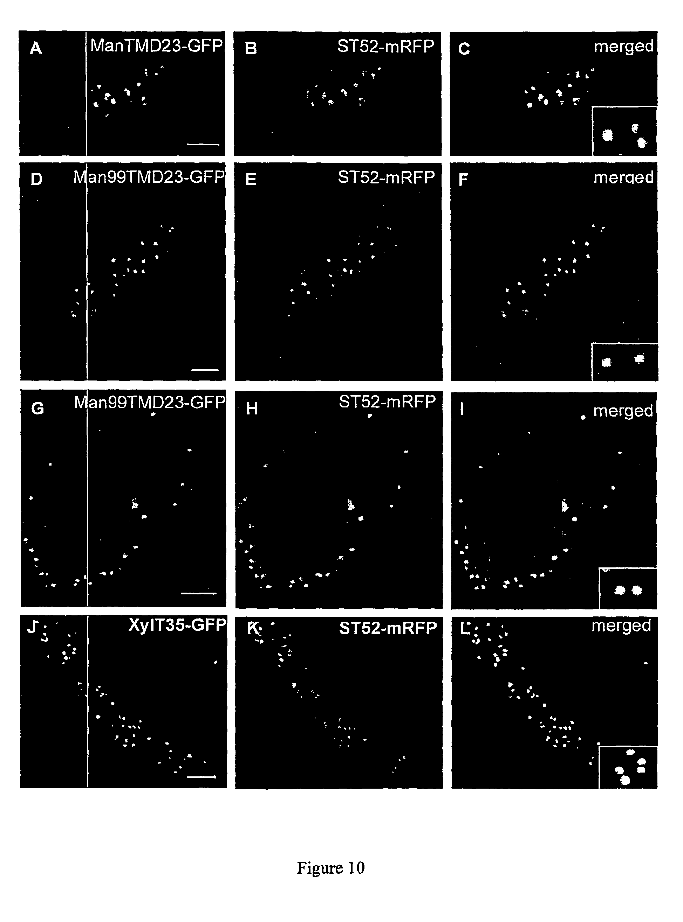

Figure 10 shows localization into the Golgi compartments of a series of GFP fusions to ManTMD23, Man99TMD23 or XylT35 (figure 3) and the trans Golgi marker ST52-mRFP, after stable co-expression (3-4. days) in tobacco BY-2 cells of one or other of these GFP fusions and ST52-mRFP, using confocal laser scanning microscopy:
(A-C) ManTMD23-GFP (A) and ST52-mRFP (B) co-expression in BY-2 suspension-cultured tobacco cells,
(D-F), (G-I) Man99TMD23-GFP (D, G) and ST52-mRFP (E, H) co-expression in BY-2 suspension-cultured tobacco cells,
(J-L) XylT35-GFP (J) and ST52-mRFP (K) co-expression in BY-2 suspension-cultured tobacco cells,
(C, F, I and L) corresponding merged expression GFP fusions + ST52-mRFP.

Inserts: magnification (x2,2)

All bars = 8 µm.

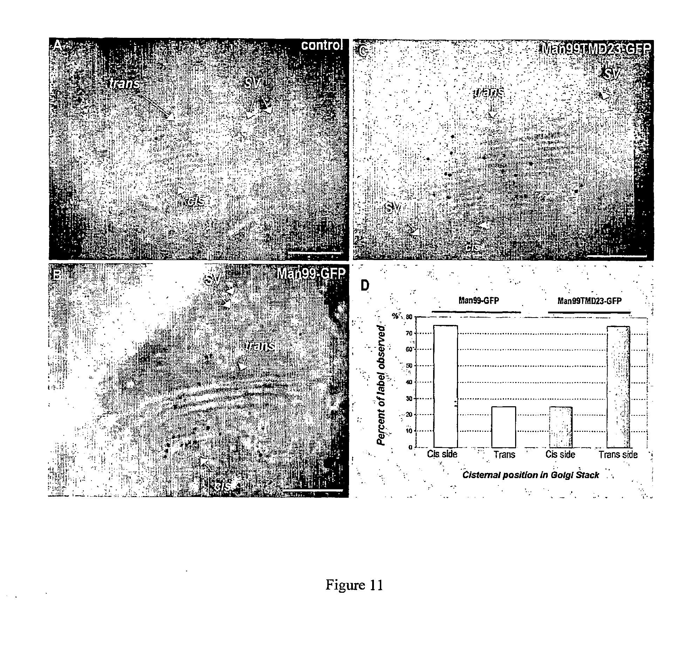

Figure 11 shows localization in the Golgi apparatus of GFP fusions to Man99 and Man99TMD23, using electron microscopy coupled to immunogold-labeling with polyclonal anti-GFP antibodies from suspension-cultured BY2 tobacco cells:
(A) wild type BY2 tobacco cells,
(B) BY2 cells expressing Man99-GFP,
(C) BY2 cells expressing Man99TMD23-GFP,
(D) Expression of the distribution of fusion proteins in the Golgi as a percent of label observed for 15 individual stacks analyzed in different cells, counting gold particles on the *cis*-side and *trans-side* after having divided Golgi in two domains.

SV= secretory vesicle.

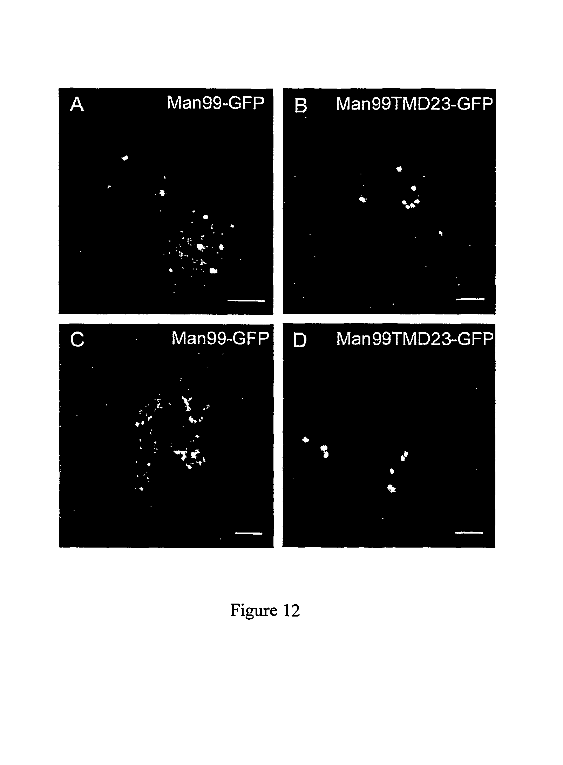

Figure 12 shows localization into the Golgi and the ER of a series of GFP fusions to members of the N-glycan processing machinery (ManI, XylT), truncated forms thereof (Man99TMD23, Man99, , figure 4) after transient expression (5 days) in leaf epidermal cells by leaf infiltration, using confocal laser scanning microscopy:
(A) Glycine max leaf epidermal cells expression of Man99-GFP,
(B) Glycine max leaf epidermal cells expression of Man99TMD23-GFP,
(C) Lycopersicum esculentum leaf epidermal cells expression of Man99-GFP,
(D) Lycopersicum esculentum leaf epidermal cells expression of Man99TMD23-GFP,

All bars = 16 µm.

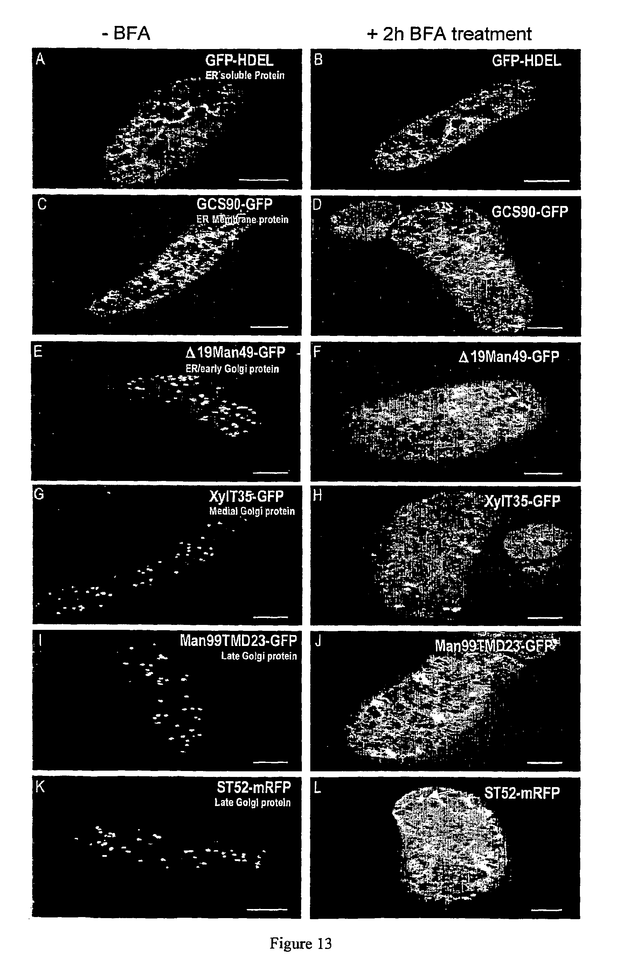

Figure 13 shows effects of a 2h treatment with BFA on ER and/or Golgi proteins in BY-2 cells expressing a soluble ER marker (GFP-HDEL, A-B), a membrane ER marker (Glu90-GFP, C-D), an ER and early Golgi marker (Δ19Man49-GFP, E-F), a medial Golgi marker (XyIT35-GFP, G-H), or late Golgi markers (Man99TMD23-GFP, ST52-mRFP, I-L), using confocal laser scanning microscopy.

Note in all cases, the ER network often turns into fenestrated sheets of fluorescence.

All bars = 8µm.

Figure 14 shows effects of a 2h treatment with BFA (50mg.mL⁻¹) on the simultaneous redistribution of both early and late Golgi markers into the ER and into the Golgi clusters, using confocal laser scanning microscopy, from:
(A-F) HDEL-GFP (A, B) and ST52-mRFP (B, E) co-expression in BY-2 suspension-cultured tobacco cells in absence or presence of BFA, respectively,
(G-L) Δ19Man49-GFP (G, J) and ST52-mRFP (H, K) co-expression in BY-2 suspension-cultured tobacco cells in absence or presence of BFA, respectively,
(M-R) Man99TMD23-GFP (M, P) and ST52-mRFP (N, Q) co-expression in BY-2 suspension-cultured tobacco cells in absence or presence of BFA, respectively,
(C, F, I, L, O, R) corresponding merged expression GFP fusions + ST-mRFP in absence or presence of BFA.

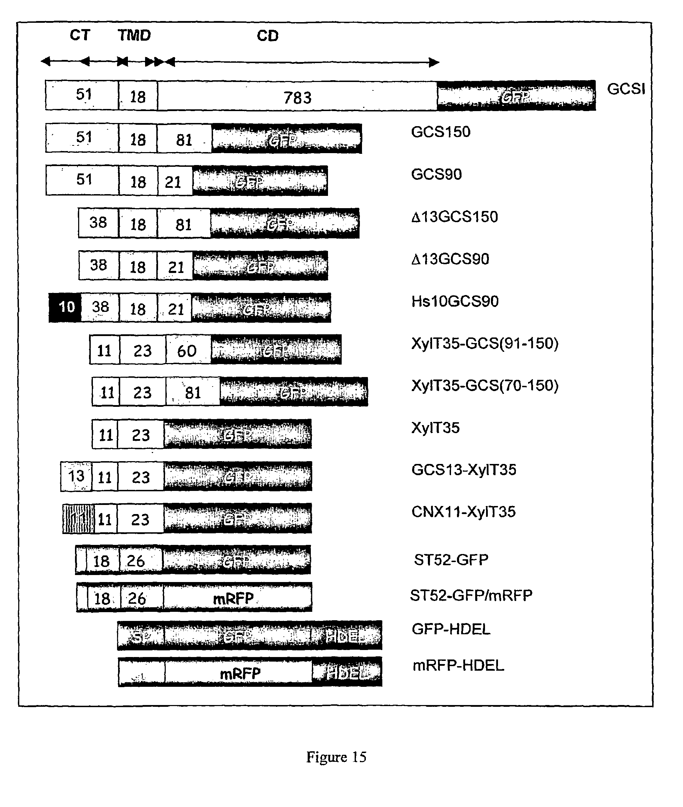

Figure 15 is a schematic representation of the constructs we used in this study: GCSI: full length A. *thaliana* α-glucosidase I fused to GFP / GCS150: the first 150 amino acids of GCSI fused to GFP / GCS90: the first 90 amino acids of GCSI fused to GFP / Δ13GCS150: the 13 first N-terminal amino acids (MTGASRRSARGRI-SEQ ID N°1) were deleted from GCS150 / Δ13GCS90: the 13 first N-terminal amino acids were deleted from GCS90/ Hs10-Δ13GCS90: the 10 first N-terminal amino acids of Homo sapiens GCSI (MARGERRRRA-SEQ ID N°2) were fused at the N-terminus of Δ13GCS90 / XYLT35: the first 35 amino acids of *A. thaliana* β-1,2-xylosyltransferase fused to GFP / XYLT35-GCS(91-150): the first 35 amino acids of XYLT were fused to the first 60 amino acids of luminal domain (a.a. 91 to 150) of GCSI and fused to GFP / XYLT35-GCS(70-150): the first 35 amino acids of XYLT were fused to the first 80 amino acids of luminal domain (a.a. 70 to 150) of GCSI fused to GFP / GCS13-XYLT35: the 13 first N-terminal amino acids of GCSI were fused to XYLT35 /CNX11-XYLT35: the last 11 amino acids (NDRRPQRKRPA-SEQ ID N°3) of A. *thaliana* calnexin (CNX) were fused to the N-terminus of XYLT35 /ST52-GFP/mRFP: the first 52 amino acids of a rat α-2,6-sialyltransferase (ST) were fused to GFP or mRFP/ GFP/mRFP-HDEL: GFP or mRFP under the control of the sporamine signal peptide and the HDEL ER retention sequence CT: cytosolic tail ; TMD: transmembrane domain ; CD: catalytic domain.

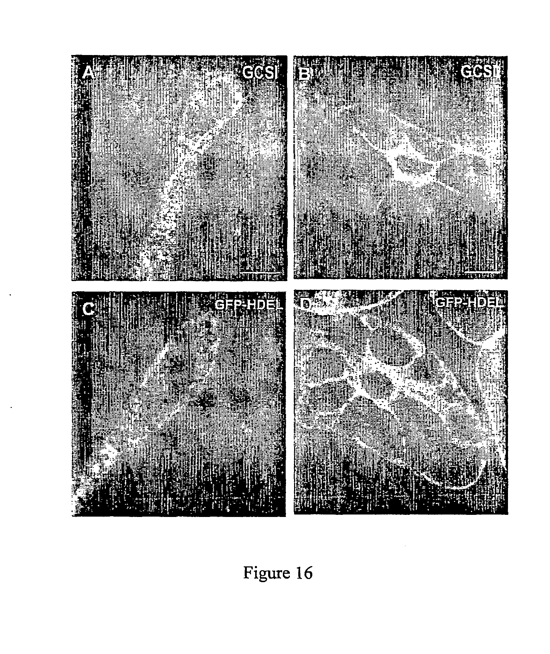

Figure 16 shows that GCSI accumulates strictly in the ER: Transgenic BY-2 tobacco cell lines were observed 3-4 days after sub-culturing. Cortical (A,C) and medial optical sections (B, D) show GCSI is located in the ER (A,B) and the pattern staining is similar to that of GFP-HDEL (C, D).

Bars = 8 µm

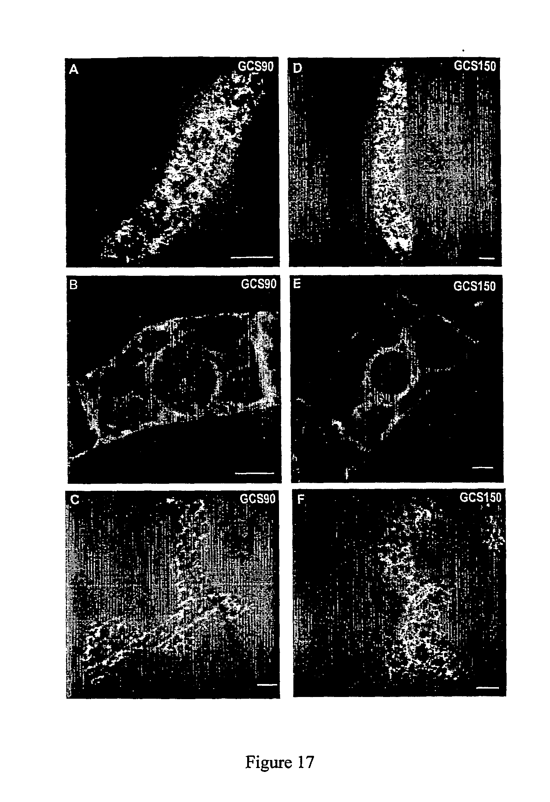

Figure 17 shows that the N-terminal domains are sufficient to localize GCSI in the ER (GCS150 and GCS90 accumulate in the ER in BY-2 cells (A, B and D ,E respectively) like the full length construct GCSI. Their targeting is identical in *Nicotiana tabacum* leaf epidermal cells (C and F respectively). Bars = 8 µm

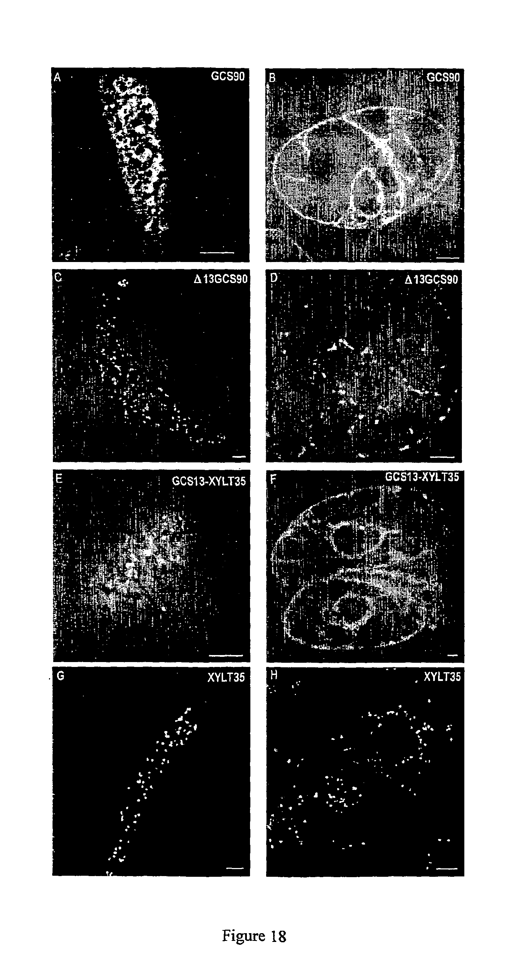

Figure 18 shows that the N-terminal sequence of 13 amino acids contains ER localization information. Whereas GCS90 accumulates in the ER in BY-2 cells (A, B), Δ13GCS90 is located in the Golgi (C, D). Whereas XYLT35 is a Golgi protein (E, F), GCS13-XYLT35 is found in the ER and in the Golgi (G, H).

Bars = 8µm

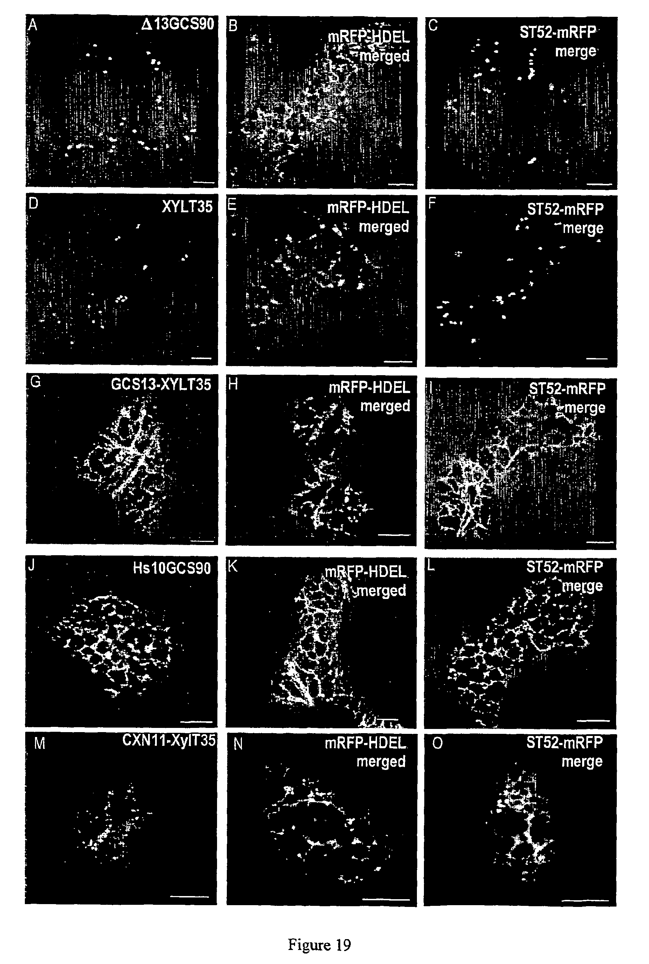

Figure 19 shows that the Arg-rich ER targeting sequence is conserved between kingdoms *Nicotiana tabacum* leaf epidermal cells expressing Δ13GCS90, XYLT35, GCS13-XYLT35, Hs10-GCS90 or CNX11-XYT35 alone (A, D, G, J, M), or with mRFP-HDEL (B, E, H, K, N), or with ST52-mRFP (C, F, I, L, O). As in BY-2 cells (Figure 4), Δ13GCS90 (A-C) is exclusively in the Golgi whereas GCS90 was in the ER and Δ13GCS90 perfectly co-localises with ST-mRFP (C, F). The micrographs are identical when Δ13GCS90 (A-C) is compared to XYLT35 (D-F). When GCS13-XYLT35 (G) is co-expressed with mRFP-HDEL, the ER appears in yellow and the Golgi remains green (H) whereas with ST52-mRFP the Golgi is yellow and the ER is green (I) showing GCS13-XYLT35 has a dual location in the ER and in the Golgi. Interestingly, the first 13 aa. of GCS90 can be replaced with the first 10 aa. of the human GCSI (J-L) : the Hs10-GCS90 chimeric protein is in the ER only (J) and co-localises with mRFP-HDEL (K) but not with ST52-mRFP (L). This suggests signals are conserved between kingdoms. In the same way, when CNX11-XYL35 (M), is co-expressed with mRFP-HDEL, the ER appears in yellow and the Golgi remains green (N) whereas with ST52-mRFP the Golgi is yellow and the ER is green (O) showing GCS13-XYLT35 also has a dual location in the ER and in the Golgi.

Bars = 8 µm

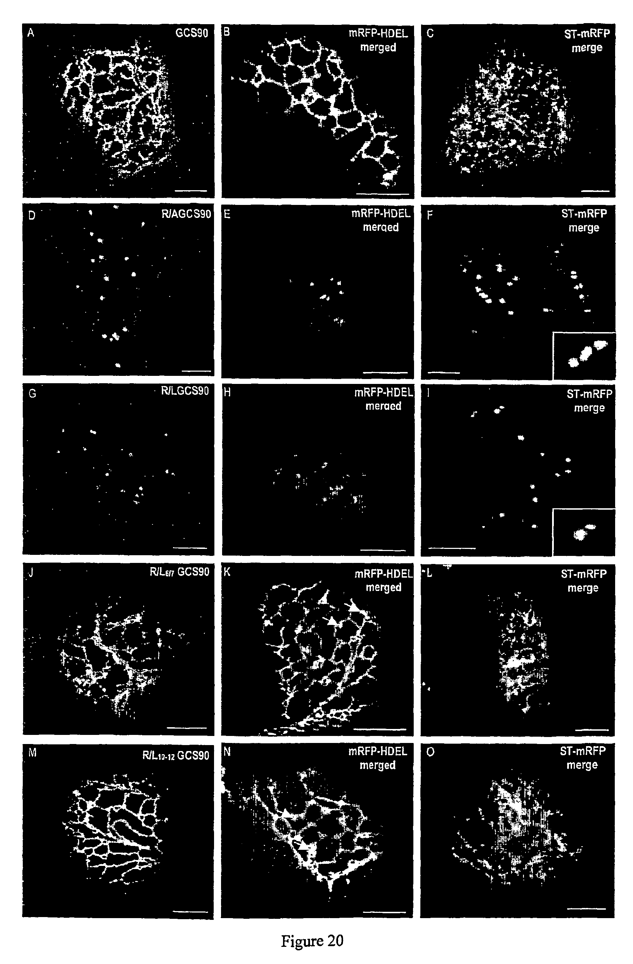

Figure 20 shows that the N-terminal arginine contains ER localization information *Nicotiana tabacum* leaf epidermal cells expressing GFP fusions alone (left panel), or with mRFP-HDEL (middle panel) or with ST52-mRFP (right panel). GCS90 (A) perfectly co-localises with mRFP-HDEL (B, ER in yellow) but not with ST52-mRFP (C, ER in green, Golgi in red). When the four Arg in position 6, 7, 10 and 12 are substituted with Ala (D-F) or Leu (G-I), R/LGCS90 or R/AGCS90 accumulates exclusively in the Golgi as illustrated with the yellow spots observed on the micrographs F and I. When Arg are mutated by pairs, R/L6-7GCS90 (J- L) or R/L10-12GCS90 (M-O) or R/L6-L12 (P-R) are located to the ER and to mini-spots that do not contain the ER soluble protein mRFP-HDEL (K, N, Q) and are closely associated to Golgi stacks (L, O, R) but remain independent. These data show an RR, RXR or RXXR motif at its N-terminus confers ER targeting to GSC90.

Bars = 8 µm

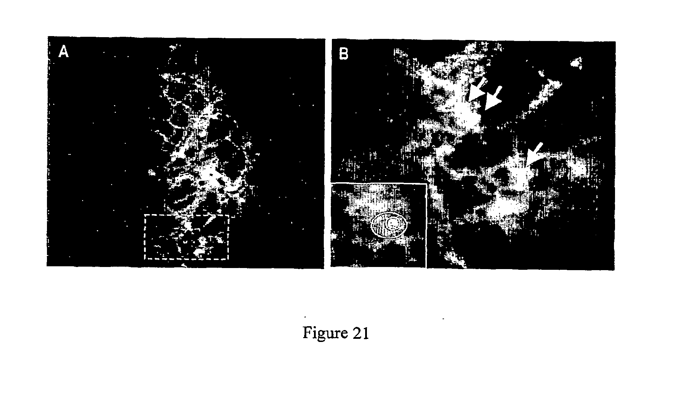

Figure 21 shows that R/L6-7 are closely associate to the Golgi. When R/L6-7GCS90 are co-expressed with mRFP-HDEL and ST52-mRFP, the fluorescent structures appear in yellow showing their very close association with the Golgi.

Bars = 8 µm

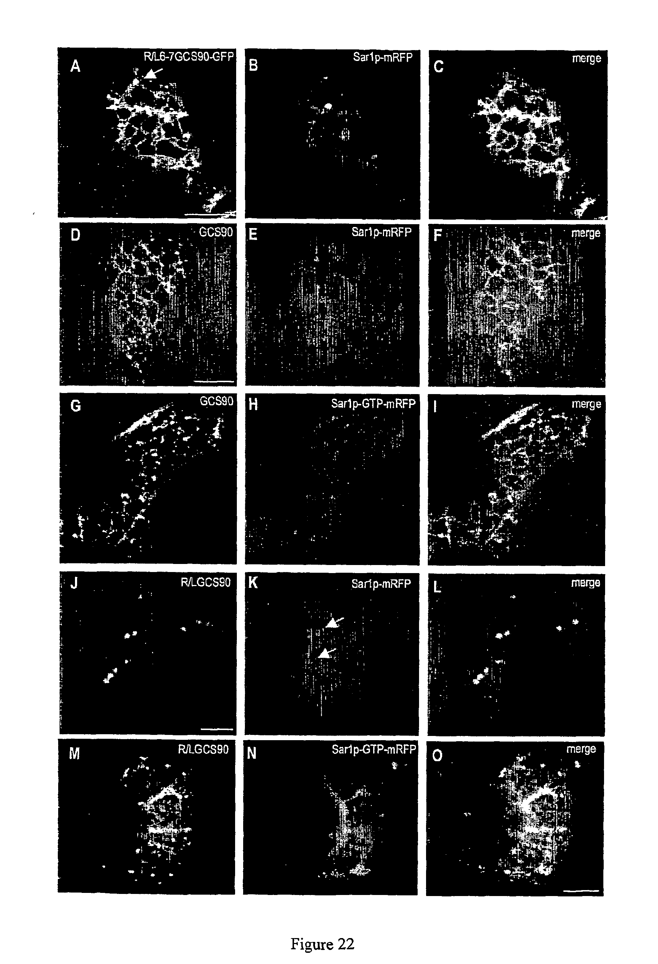

Figure 22 shows that *Nicotiana tabacum* leaf epidermal cells expressing GFP-fusions with Sar1p-mRFP (panels B, E and K) or Sar1p-GTP-mRFP (panels H and N). When Arg are mutated by pairs, R/L6-7GCS90 (A-C) or GCS90 (D-I) are co-expressed with mutated or not mutated Sar1p, no labelling modification was observed for GFP-fusions. In contrast, the co-expression of R/LGCS90 with Sar1p -GTP (M-O) blocks the transport of R/LGCS90 in the ER while the co-expresion with Sar1p doesn't modify the pattern of R/LGCS90.

Bars = 8 µm

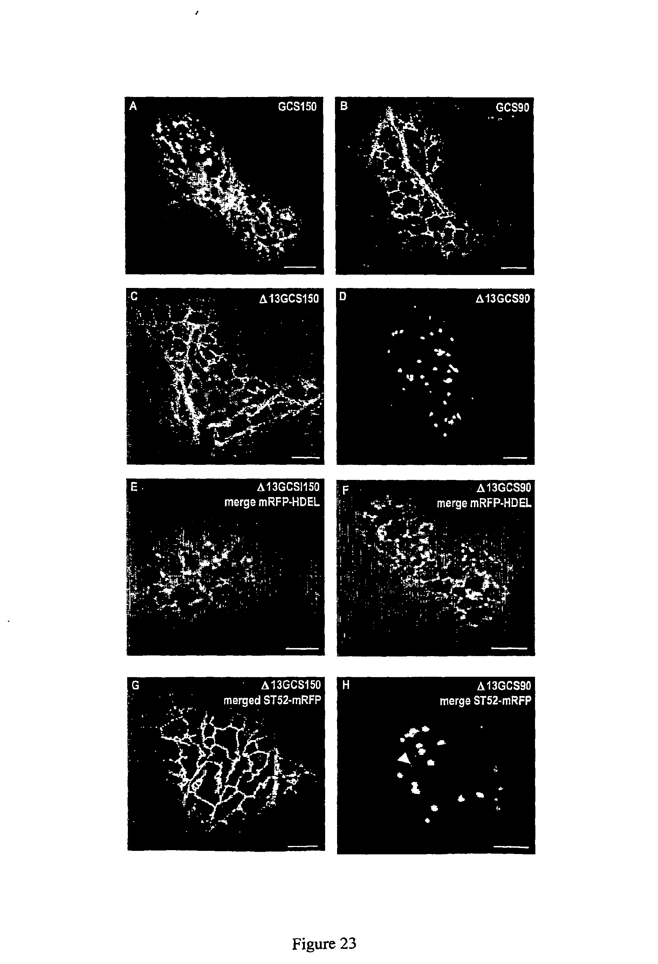

Figure 23 shows that the N-terminal arginine motifs are not the key determinant for ER retention of *At*GCSI. ER is visualized in transient GCS150-GFP (A), GCS90-GFP (B), and Δ13GCS150-GFP (C) transformed *Nicotiana tabacum* leaf epidermal cells, cortical section. ER localization of Δ13GCS150-GFP is confirmed by co-expression with mRFP-HDEL (E) and ST52-mRFP (G), cortical sections. The first 13 aa of this type II membrane protein seem to be not implicated in GCS150-GFP ER targeting in spite of di-arginine motif. (D) (F) (H) Golgi is visualized in transient Δ13GCS90-GFP transformed *Nicotiana tabacum* leaf epidermal cells (D), and in co-expressing mRFP-HDEL (F) or ST52-mRFP (H), cortical sections. The first 13 aa are essentials in ER targeting for GCS90-GFP contrary to GCS150-GFP. Amino acids flanking the luminal domain may contain ER targeting information. Bars: 8µm

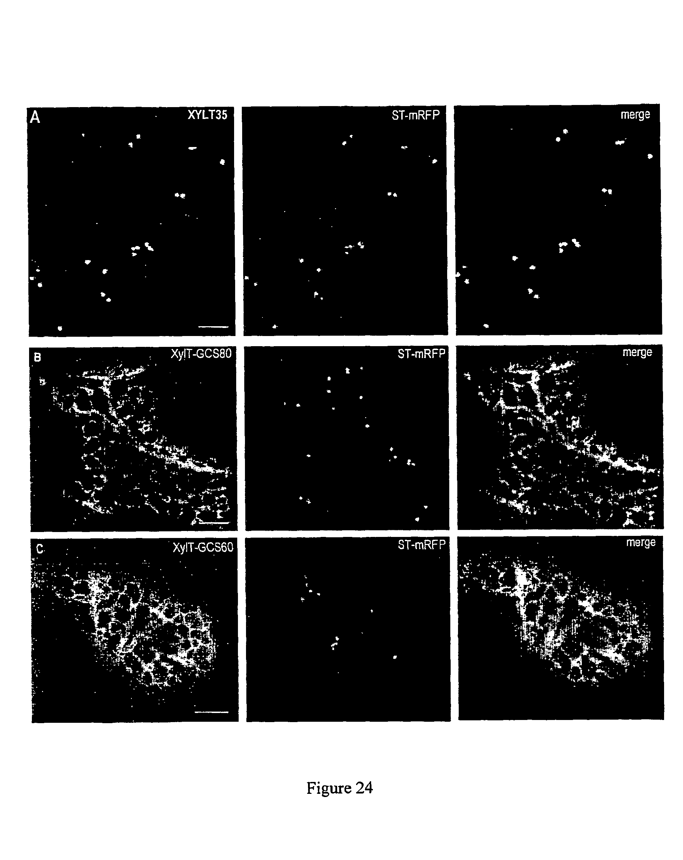

Figure 24 shows that the luminal targeting determinant is sufficient to retain XYLT35 in the ER:
(A) Golgi visualized in transient XYLT35-GFP transformed Nicotiana tabacum leaf epidermal cells, cortical sections.
(B) (C) ER and Golgi visualized transiently in *Nicotiana tabacum* leaf epidermal cells co-expressing XYLT35-GCS60 (B) XYLT35-GCS80 (C) with ST52-mRFP, cortical sections. Luminal domains of AtGCSI relocate the majority of XYLT35 in the ER. Bars= 8µm

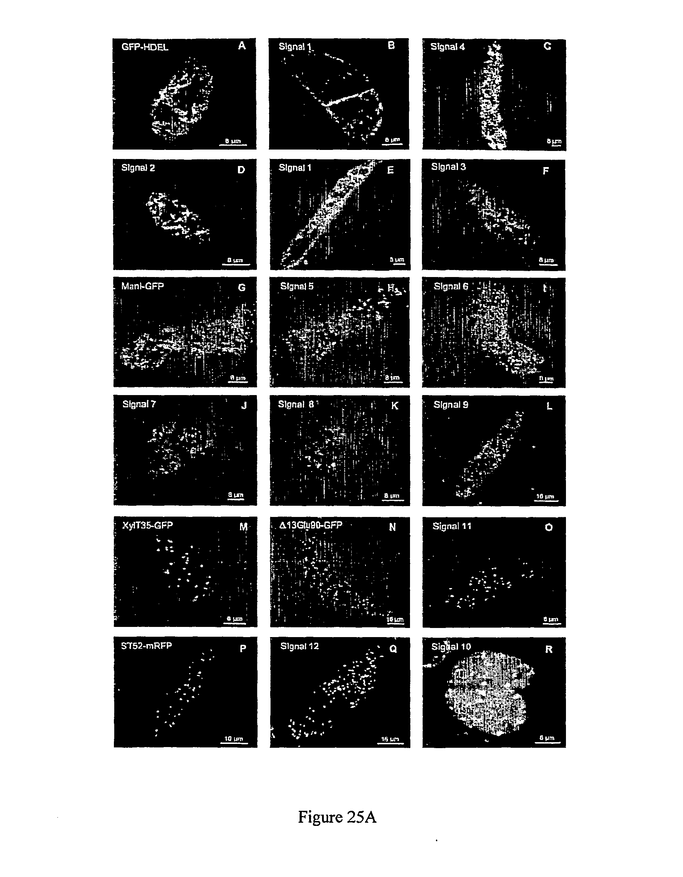

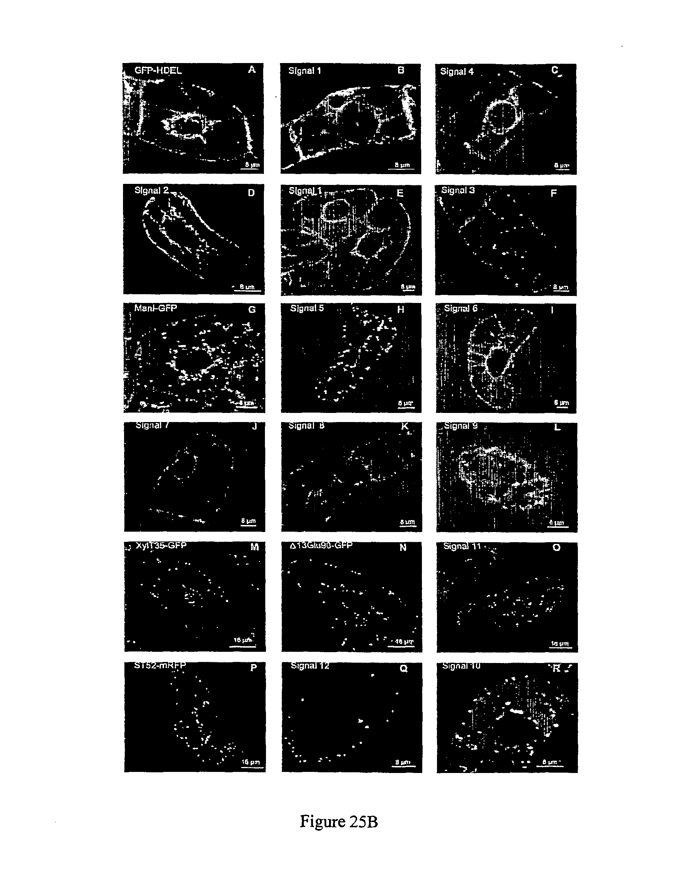

Figure 25 shows localisation analysis of GFP fusion in BY-2 cells by confocal microscopy; Cortical (A) or transversal (B) View. Signals recently identified in our group have been fused to GFP and the localisation of the recombinant protein has been analyzed after stable expression in BY-2 tobacco cells. Fusion between GFP and signals 1, 2, 3 or 4 highlighted the ER like the GFP-HDEL fusion (panels A-F). Fusion between GFP and signals 5, 6, 7, 8 or 9 highlighted the ER but also aggregates assimilated to Golgi clusters, like the control ManI-GFP (panels G-L). Fusion between GFP-and- signals 11-12 highlighted only aggregates assimilated to Golgi clusters, like the control XylT35-GFP and D13Glu90-GFP (panels M-O). Finally, fusion between GFP and signal highlighted aggregates assimilated to Golgi clusters but also the lysosomal compartment (panel R).

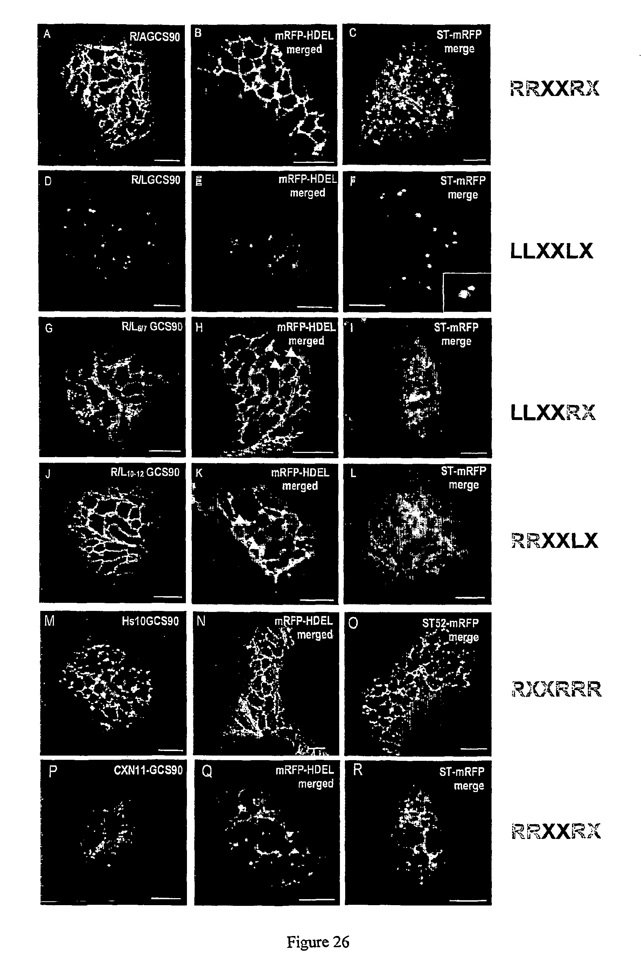

Figure 26 shows the localization of recombinant proteins harboring a di-arg motif. Recombinant protein harboring one or two di-arg motifs colocalized with the mRFP-HDEL ER maker (A-C; G-R) while the mutations of arginine are responsible of the co-localisation of the recombinant protein with ST52 -mRFP Golgi marker ( D-F).

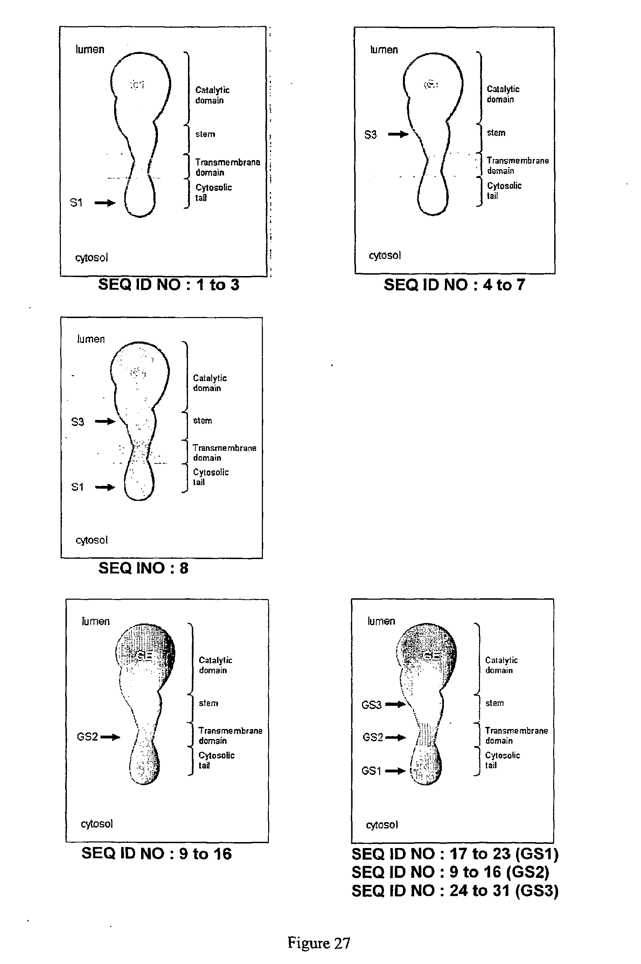

Figure 27 shows localization of targeting signals on the type I or type II membrane protein.

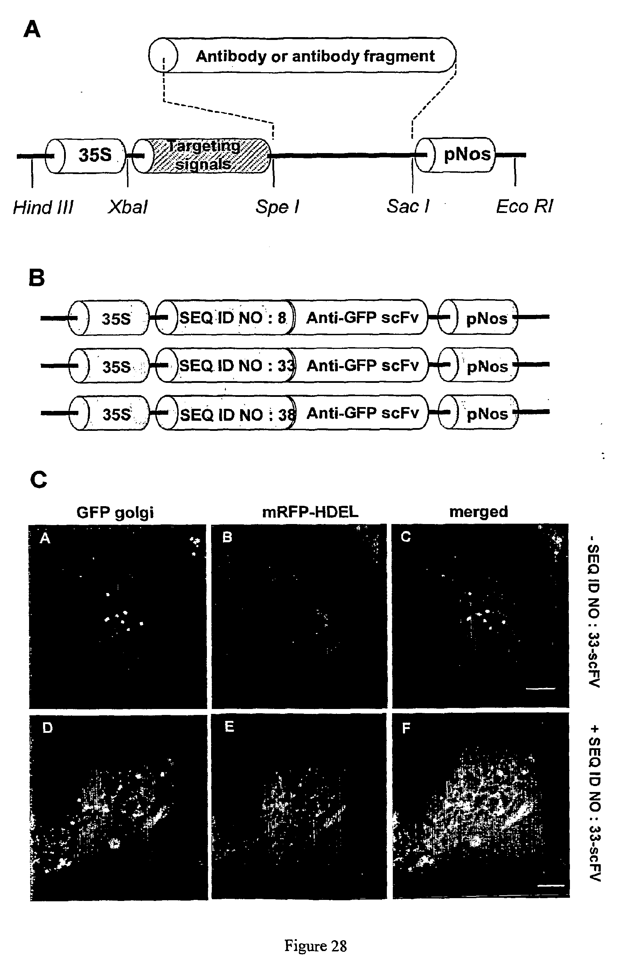

Figure 28, illustrates that an antibody or an antibody fragment, specific for a membrane protein and fused with one of the targeting sequence described here has the capacity to target the membrane protein in a different compartment of the secretory pathway. Here a GFP localized in the Golgi is used for illustration. Construction of plasmids and some examples of plasmids used for targeted expression of a scFv are presented panels A and B. Here (panel C) a membrane protein localized in the Golgi (GFP-golgi) when expressed alone is reoriented to an ER/Golgi compartment when co-expressed in a same plant cell with a GFP-specific scFv fused with SEQ ID NO: 33.

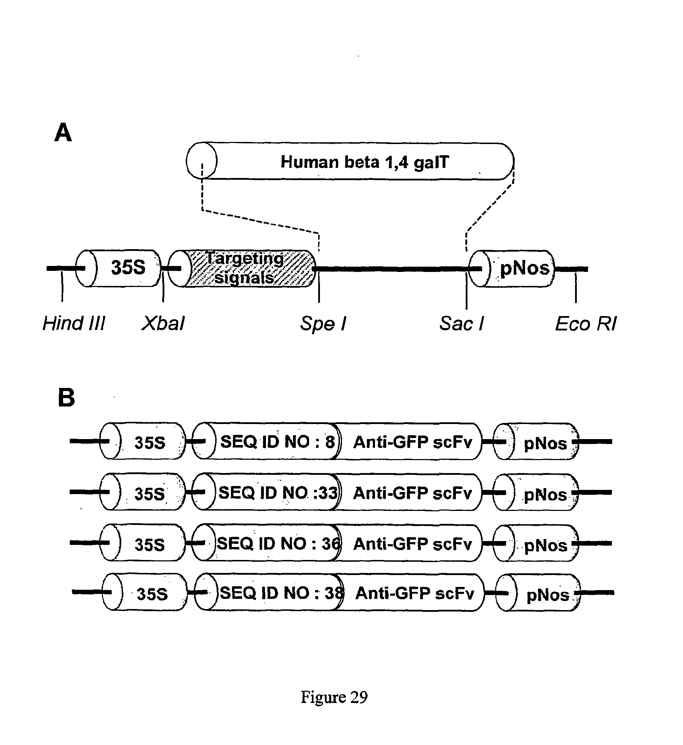

Figure 29 illustrates that an heterologous enzyme, here human beta 1,4 galactosyltransferase, can be targeted in the different compartments of the plant secretory pathway after fusion with one of the targeting signals described herein. As example panel B illustrates plasmids used for expression of fusions of human β1,4 galactosyltransferase with SEQ ID NO: 8, 33, 36, or 38 in plant cells.

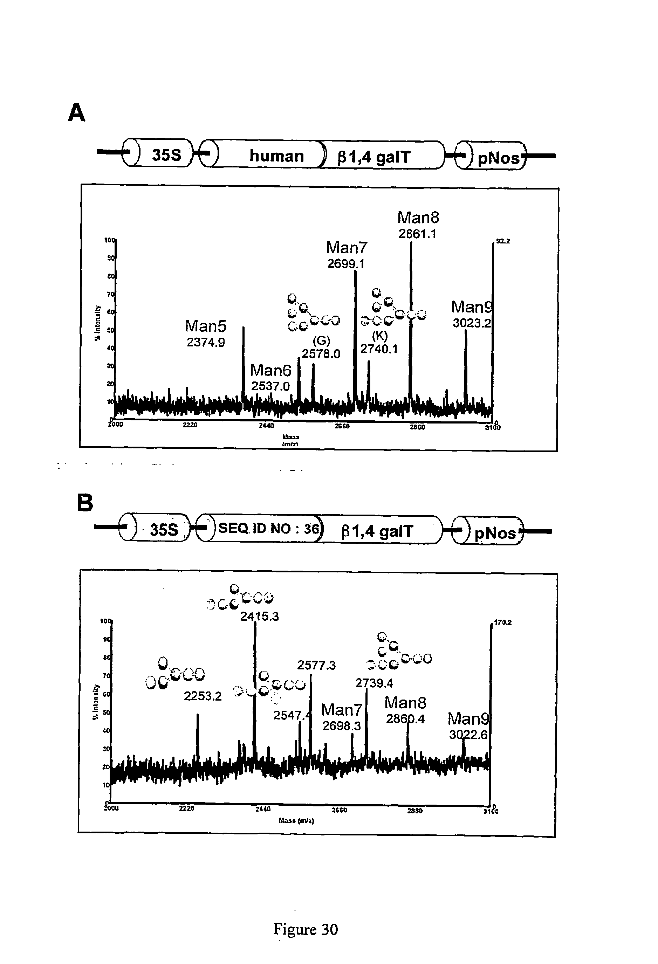

Figure 30 illustrates that targeted expression of human beta 1,4-galactosyltransferase after fusion of this glycosyltransferase catalytic domain with SEQ ID NO: 36 strongly improves the efficiency of this heterologous glycosyltransferase (panel B) when compared to glycosylation patterns obtained when the same glycosyltransferase is targeted in the plant secretory pathway by its own human targeting sequence (panel A).

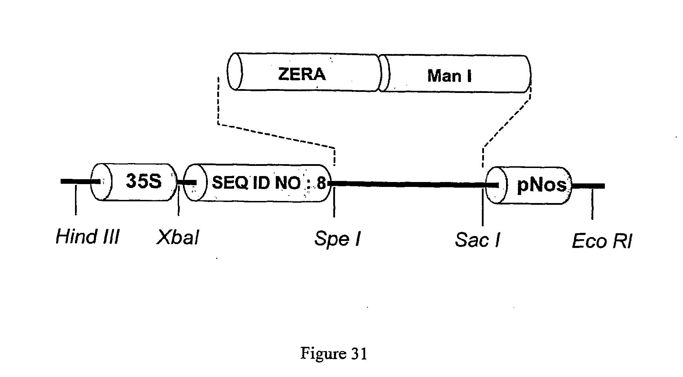

Figure 31 presents one of the plasmids prepared to accumulate a glycan maturation enzyme in protein bodies generated by the ZERA® peptide. The plasmid detailed here allows expression of mannosidase fused with Zera® in a plant cell.

### EXAMPLES

### EXAMPLE A: ADRESSING PROTEINS TO THE ER AND/OR GA (ANNEXED FIGURE 3A)

### Example 1: Identification of the targeting signal

### 1. Localisation of the Early Golgi type II membrane proteins

To better understand the mechanisms allowing the selective retention of N-glycan processing enzymes in the early Golgi compartments, the localization of a series of GFP fusions to four different members of the N-glycan processing machinery (a-glucosidase I, mannosidase I, N-acetylglucosaminyltransferase I and b-1,2-xylosyltansferase, annexed figure 4) was studied after stable expression in tobacco BY-2 cells.

The construct for expressing the GFP fusion protein was made as discloded in Saint-Jore-Dupas et al, 2006 (**ref**. 58). All Mannosidase I fusion constructs were derived from the full-length GFP fusion (here called ManI-GFP) originally described by Nebenführ *et al.* (1999) (**ref**. 43).

First, a linker containing an *AatII* restriction site was introduced between the ManI and the GFP coding regions. In combination with the native *AatII* site near the end of the predicted stem region this allowed for simple removal of the catalytic domain to yield Man99-GFP.

Second, to facilitate the removal of specific segments of the N-terminal region three new restriction sites were introduced by PCR mutagenesis: One *Nhel* site immediately behind the start codon, one *Spel* site at codons 21 and 22, and another *AatII* site at codons 50 and 51. The integrity of the modified construct was confirmed by sequencing. In this new construct, the cytoplasmic tail could be removed with *Nhel* and *Spel* to give Δ19CTManI-GFP, while an *Aatll* digest would remove the entire lumenal part of ManI to yield Man49-GFP. Combination of the two procedures resulted in Δ19CTMan49-GFP. Finally, forward (5'-GATCCTTGGGAATGCTTGCTCTGCTCTTCATCGTTTTCGTTTGTGTC TCTTTCGTTTTCTGGGACCGTCAAA-3' (SEQ ID N°40)) and reverse (5'-CTAGTTTGACGGTCCCAGAAAACGAAAGAGACACAAACGAAAACGAT GAAGAGCAGAGCAAGCATTCCCAAG-3' (SEQ ID N°41)) oligonucleotides encoding the 18 aa of the TMD domain with a start codon were synthesized, fused and subcloned into the pBLTI121 binary vector containing the GFP without any Start codon (Kiefer-Meyer et al., unpublished data) to give the DCTMan49-GFP. The same strategy has been used to generated the MAAMan49-GFP using forward (5'-GATCCTTGGGAATGGCTGCTGCTCTTGCTCTGCTCTTCATCGTTTTCG TTTGTGTCTCTTTCGTTTTCTGGGACCGTCAAA-3' (SEQ ID N°42) and reverse (5'-CTAGTTTGACGGTCCCAGAAAACGAAAGAGACACAAACGAAAACGAT GAAGAGCAGAGCAAGAGCAGCAGCCATTCCCAAG-3' SEQ ID N°43) primers.

Third, a longer TMD region was introduced in a two-step PCR mutagenesis of the modified Manl described above. In the first step, the *AatII* site following the TMD was replaced with a *BspEl* site. In the second step a long PCR primer was used to duplicate the last seven aa of the predicted TMD to yield ManTMD23-GFP. Finally, the catalytic domain of this construct was removed with *AatII* to give Man99TMD23-GFP.

All cloning steps described above were carried out in pBluescript. The finished expression cassettes (including a double 35S promoter and a Nos terminator) were then moved to pBIN20.

To obtain the plant binary vector encoding ST-mRFP, GFP is replaced with monomeric RFP (provided by Roger Tsien) in pVKH18En6 ST-GFP (Saint-Jore *et al.,* 2002 (**ref**. 56). ST-mRFP expression is under control of 6x tandemly-repeated CaMV 35S promoters.

The GNTI-GFP, the GNT38-GFP were amplified by PCR using the *N. tabacum* cDNA encoding N-acetylglucosaminyltransferase as template (Strasser et al, 1999 (**ref**. 60)). Reverse primers 5' GGTCACTAGTATCTTCATTTCCGAGTTG-3' (SEQ ID N°44) and 5'-GGTCACTAGTGCGATCTGCATATTCTGACTG-3' (SEQ ID N°45), were used for PCR with forward 5-AACGTCTAGAATGAGAGGGTACAAGTTTTGC-3' (SEQ ID N°46) primer to amplify the GNTI and the N-terminal 38aa end of the GNTI to obtain GNTI-GFP and GNT38-GFP, respectively. To express GCSI-GFP, the total cDNA was amplified by PCR using A. thaliana cDNA cloned in Boisson et al, 2001, fused at the N-terminal end of GFP and sub-cloned in pBLTI121 *(Pagny et al.,* 2003 (**ref**. 50)). Then, the first 90aa were amplified by PCR with forward (5'-CGGGGTACCCCATGACCGGAGCTAGCCGT-3' (SEQ ID N°48)) and reverse (5'-GACTAGAAAAGGAGTGATAACCCT-3' (SEQ ID N°49)) primers and subcloned in the Spe I restriction site located at the 5' end of GFP contained in the pBLTI121 binary vector to give the GCS90-GFP. In the same way, the 90 aa deleted of the first 13 aa were amplified by PCR with forward (5'-CGGGGTACCCCATGAAATCATCATCATTATCTCCC-3' (SEQ ID N°49)) and the same reverse primers as above to give D13GCS90-GFP.

Fluorescence of a full-length ManI-GFP fusion construct was detected by confocal laser scanning microscopy in small bodies (annexed figures 5A and 5B) that moved through the cytoplasm as it has been described previously for this construct in another independent cell line (Nebenführ *et al.,* 1999 (**ref**. 43)).

In addition, a substantial fluorescence signal was observed in a reticulate network throughout the cytoplasm that was indistinguishable from the ER network stained by a GFP-HDEL construct (annexed figures 5D and 5E). To check if ER labeling was due to overexpression of the recombinant proteins, BY-2 cells expressing ManI-GFP were incubated with the protein synthesis inhibitor cycloheximide. After 2h of treatment, the targeting pattern remained unchanged showing the steady state location of ManI-GFP is the Golgi and the ER (compare annexed figures 5C and 5B).

To confirm that fluorescent spots were Golgi stacks, the cells were treated for 2h with 50 mg.mL⁻¹ of b**ref.**eldin A (BFA). This BFA treatment caused the green spots to disappear and the cortical and transvascular ER became more fluorescent (compare annexed figure 5F to annexed figures 5B and 5E) as has been described previously for several Golgi-localized GFP fusion proteins expressed in tobacco leaf epidermis and BY-2 suspension cultured cells (Saint-Jore *et al.,* 2002 (**ref**. 56); Ritzenthaler *et al.,* 2002 (**ref**. 54)).

The comparison of the location of Manl to the one of other plant N-glycosylation enzymes in the secretory pathway was analyzed under the same conditions the sub-cellular localization of N-glycan maturation enzymes acting before, just after Manl or much later. The first enzyme studied was a-glucosidase I from Arabidopsis (GCSI). This type II membrane protein trims the first sugar residue from the precursor oligosaccharide in the ER immediately after its attachment to the nascent glycoprotein (see a schematic representation of plant N-glycan maturation in annexed figure 2B). The full length protein (Boisson *et al.,* 2001 (ref. 6)) was fused to GFP and, consistent with what was shown for human GCSI in COS cells (Hardt *et al.,* 2003 (ref. 28), the fusion protein was exclusively located in the ER in BY-2 cells (annexed figure 5G).

The second candidate investigated was (GNTI) from *Nicotiana tabacum* (Strasser *et al.,* 1999 (ref. 60)). This glycosyltransferase adds the first N-acetylglucosamine residue on N-glycans soon after Manl has removed an a-1,2-mannose (annexed figure 2B). The full length protein was fused to GFP and GNTI-GFP was expressed in tobacco BY-2 suspension-cultured cells. Interestingly, the steady state location of the fusion was the Golgi and the ER (annexed figure 5H) in a pattern very similar to ManI-GFP (compare annexed figures 5H and 5B). These data strongly suggest the N-glycan processing enzymes considered to act very early in the Golgi apparatus such as ManI and GNTI are targeted to the Golgi but also to the ER in tobacco BY-2 suspension cultured cells.

Finally, the third candidate, b-1,2-xylosyltansferase (XylT) from *Arabidopsis* was located in the Golgi only (annexed figure 5), confirming the results from Pagny *et al.* (2003) (**ref.** 50) who demonstrated that the N-terminal end of this enzyme targets GFP to a medial subset of cisternae of Golgi stacks.

To ascertain whether protein expression levels might alter localization of our fusion proteins, these results were confirmed in different stable independent cell lines expressing the fusion proteins. Imaging of cells was always performed on the third or fourth day after sub-culturing which corresponds to the optimal growth phase under our culture conditions. Nevertheless to further validate that ER labelling was not due to over-expression of the fusion, the labelling pattern for each fusion was controlled by verifying that it was unchanged after a 2h treatment with cycloheximide.

Western-blots revealed with anti-GFP antibodies and the ECL staining have shown a very low signal over background for the recombinant proteins, this indicating a low level of expression for all fusion proteins in this study (data not shown). Further evidence for a level of fusion protein expression compatible with a functional non saturated secretory pathway was obtained from co-expression experiments when in same cell ManI-GFP is located both in the ER and Golgi, while a Golgi marker (ST52-mRFP) is found exclusively in the Golgi (annexed figures 7A-C).

All together, the results obtained under these carefully controlled conditions clearly show that N-glycosylation enzymes are targeted specifically to the ER (GCSI) or to the Golgi (XylT) exclusively, but some enzymes have a dual steady state location in both organelles as it is the case for the Manl and the GNTI and other membrane proteins such as prolyl 4-hydroxylase (Yuasa *et al*., 2005 (**ref**. 65) and ERD2 (Boevink *et al.,* 1998 (**ref**. 5); Saint-Jore *et al.,* 2002 (**ref**. 56)).

In a next step, the signals which were responsible for the targeting of the population of glycosylation enzymes showing a dual steady state distribution Golgi / ER was investigate.

### 2. Experiments showing that the luminal domain is not necessary for Golgi and ER targeting of ManI and GNTI

In the regarding the specific Golgi retention of the three plant glycosylation enzymes GNTI, XylT and Arabidopsis Manl (annexed figure 2) indicate that their specific targeting is mediated by signals contained in their N-terminal part including the cytoplasmic tail, the TMD, and the stem for GNTI (Essl *et al.,* 1999 **(ref.** 16), Dirnberger et al., 2002 **(ref.** 15); Pagny *et al.,* 2003 (**ref**. 50); Strasser *et al.,* 2006 (**ref**. 61)). The present example investigates the role of the luminal domain in the targeting of Manl and GNTI.

In order to determine if the portion of ManI located in the Golgi lumen plays a role in the targeting of this glycosidase to the Golgi and the ER membranes, the first 99 aa (CT+TMD+S) or the first 49 aa (CT+TMD) of Manl were fused to GFP and the corresponding chimeric proteins were named Man99-GFP and Man49-GFP, respectively (annexed figure 4). Man99-GFP and Man49-GFP were either stably expressed in BY-2 suspension cultured cells or transiently expressed in tobacco leaf epidermal cells by leaf infiltration. Both Man99-GFP and Man49-GFP chimeric proteins were observed in the Golgi and in the ER in both expression systems (annexed figures 6A, 6B, 6E and 6D, 6F respectively) exactly as previously observed for the full length construct (annexed figures 5A and 5B).

It is important to note that when these truncated fusions were transiently expressed in tobacco leaves, the ER labeling was still observed 5 days after transformation when the overall expression levels are already strongly declining (annexed figure 6F) and whereas XylT35-GFP was located in the Golgi only (Pagny *et al.,* 2003 (**ref**. 50); annexed figure 6l). This further confirms that the partial location of the Manl-fusions in the ER is not due to over-expression of the chimeric proteins. In addition, when BY-2 cells expressing Man99-GFP were treated with the protein synthesis inhibitor cycloheximide for 2h, the ER labelling did not disappear (annexed figure 6C) as had been observed for the full-length fusion (compare to annexed figure 5C).

To get a better understanding of where the fusion proteins are localized within the Golgi stacks, ManI-GFP was co-expressed with the *trans* Golgi marker ST52-mRFP which is derived from ST52-GFP (Saint-Jore *et al.,* 2002 (**ref**. 56); Runions *et al.,* 2006 (**ref**. 55) by replacing GFP with the monomeric red fluorescent protein (mRFP, Campbell *et al.,* 2002 **(ref. 11**). When the two chimeric proteins were expressed simultaneously in BY2 cells, in contrast to ManI-GFP, ST52-mRFP was not detected in the ER and both fluorescence signals were observed in Golgi bodies but did not perfectly co-localize (annexed figures 7A-C). These results are consistent with previous studies that have demonstrated that

1) the ManI-GFP fusion is located in the cis-half of the Golgi in BY-2 cells (Nebenführ *et al.,* 1999 (**ref**. 43)) and

2) the 52 amino-terminal amino acids of rat a-2,6-sialyltransferase are sufficient to target a reporter protein predominantly to the *trans*-half of Golgi stacks (Boevink *et al.,* 1998 (**ref**. 5)).

Thus, confocal microscopy was sufficient to illustrate that ManI-GFP and ST52-mRFP accumulate in a different subset of cisternae in the Golgi apparatus. Finally, when, Man99-GFP or Man49-GFP were co-expressed with the trans-Golgi marker ST52-mRFP, the two fluophores only partially overlapped (annexed figure 7D-F and 7G-I, respectively) suggesting that the intra-Golgi localization of the truncated fusion proteins was the same as that of the full-length fusion ManI-GFP.

The first 77 N-terminal aa of the tobacco GNTI, including the CT, the TMD and the stem, were previously described to contain the information required to maintain Golgi retention of this glycosyltransferase (Essl *et al.* 1999 **(ref.** 16)). This polypeptide domain fused to GFP has been shown to be preferentially located in the Golgi but the chimeric protein was also detected in the ER as observed here for the full length construct (annexed figure 5H). In order to determine if the sequence remaining in the Golgi lumen is involved in the Golgi and ER targeting of GNTI, the lumenal part (39 aa) was removed and the remaining first 38 N-terminal aa (CT+TMD) of this glycosyltransferase were fused to GFP (annexed figure 4). The fusion protein was named GNT38-GFP and stably expressed in BY-2 suspension cultured cells or transiently in tobacco leaf epidermal cells.

In both expression systems, GNT38-GFP was located in the Golgi and in the ER (annexed figure 6G and 6H) as previously observed for the full length construct (GNTI-GFP, annexed figure 5H). In addition, when GNT38-GFP was stably co-expressed with ST52-mRFP in BY-2 cells, as observed before with Man99-GFP and Man49-GFP, the two fluorescents spots overlapped, but some red fluorescence was distinguishable from the yellow suggesting GNT38-GFP is not in the trans-Golgi (annexed figures 7J-L) as previously observed for ManI.

It is clear from these results that the cytosolic tail and TMD of both Manl and GNTI are sufficient to target these glycosylation enzymes to their steady state location: the ER and the early Golgi compartments. In contrast, the same domain (CT+TMD) targets XylT35-GFP to the Golgi only both in BY-2 cells (Pagny et al., 2003 (**ref**. 50)) and nicotiana leaf epidermal cells (annexed figure 6l).

### 3. Experiments showing that the cytoplasmic tail is not necessary for the retention of ManI in the early compartments of the secretory pathway.

The N-terminal cytosolic region of many membrane bound proteins residing in the mammalian and yeast ER and/or in the Golgi apparatus contains signals which facilitate either their retrieval from the Golgi back to the ER (Teasdale and Jackson, 1996 (**ref**. 62); Zerangue *et al.,* 1999 (**ref**. 66)) or their export from the ER to the Golgi (Giraudo and Maccioni, 2003 (**ref**. 20). In plants, the length of cytoplasmic tails can vary widely between the different glycosidases and glycosyltransferases (annexed figure 8). For instance, GCSI and Manl contain a long cytoplasmic tail (51/29 aa respectively). In comparison, the CTs of GNTI and XylT are only composed of 11 aa.

To define more precisely the targeting signal of Manl and to investigate the role of the relatively long cytoplasmic domain (29 aa) of this glycosidase in this targeting, two fusion proteins were generated D19Man-GFP and D19Man49-GFP. In the latter two proteins, 19 amino acids were removed so that the CT was shortened down to 10 amino acids, alike the CTs of XylT and GNTI. This truncation removed a potential dibasic motif (KxR) that might function in ER-to Golgi transport (Giraudo and Maccioni, 2003 **(ref. 20));** although another potential ER-export signal remained. A complete removal of the CT was attempted (DCTMan49-GFP, annexed figure 4) but it was not possible to get this fusion protein expressed in tobacco cells. In order to look for a targeting determinant in the remaining 10 aa of the CT, the CT sequence was substituted by an artificial sequence MAAA, (MAAAMan49-GFP, annexed figure 4). This artificial sequence does not contain any known targeting sequences and does not affect the length of the hydrophobic transmembrane domain.

The three constructs, MAAAMan49-GFP, D19Man-GFP and D19Man49-GFP have been expressed in tobacco cells. The two latter labelled the ER and the Golgi (annexed figures 9A-D) just like the constructs they originate from (annexed figure 5A, 5B and 6D, 6F respectively). In addition, the fusion protein containing an artificial MAAA CT was located in the same compartments (annexed figure 9E) indicating that the N-terminal cytosolic region is not necessary for Manl targeting and consequently all information required for its steady state localization to both the ER and the Golgi apparatus is contained within the 20 aa of the MAAAMan49-GFP construct *i.e.,* in the TMD and C-terminal flanking amino acids.

### 4. Experiments showing that the Trans Membrane Domain (TDM) length plays the key role in Golgi targeting and subcompartimentation of Manl

For mammalian cells, several models have been proposed to explain how type II membrane proteins are retained at different levels within the Golgi.

According to a first model, the "kin recognition" model (Nilsson *et al.,* 1993b (**ref**. 45)), aggregation of N-glycan maturation enzymes by homo/hetero-oligomerization would prevent the resulting large complexes from being delivered to secretory vesicles and ongoing forward transport downstream in the secretory pathway.

One of the first reported cases of this type of association involved a-mannosidase II, and GNTI, two glycosylation enzymes located in the medial-Golgi and acting sequentially in mammalian N-glycan maturation (Nilsson *et al.,* 1994 (**ref**. 46)). It should be noted that this model originally assumes the presence of stable Golgi cisternae and the anterograde flow of secretory cargo via vesicular shuttles (Nilsson *et al.,* 1993b (**ref**. 45)). To fit with the cisternal progression/maturation concept, the "kin recognition" model would have to be modified to allow for the oligomeric complexes to be preferentially packaged into retrograde vesicles (Füllekrug and Nilsson, 1998 **(ref.** 18)).

A second model, the lipid bilayer model (Bretscher and Munro, 1993 **(ref.** 10)) proposes that the fit between the length of TMD of glycan maturation enzymes and the thickness of the lipid bilayer of each organelle membrane determines the localization because each organelle has its specific membrane lipid composition and consequently its own thickness (Hartmann and Benveniste, 1987 (**ref**. 29); Lynch, 1993 (**ref**. 33); Moreau *et al.,* 1998 (**ref**. 36); Morré and Mollenhauer, 1974 (**ref**. 37)).

According to the membrane thickness model, the distribution of N-glycan maturation enzymes in the secretory pathway is based on the length of their TMDs (Bretscher and Munro, 1993 **(ref.** 10)). The membranes of the secretory pathway organelles increase in thickness from the ER to the plasma membrane. The ER and the cis-Golgi membranes are only 4-5 nm thick whereas the membranes of the *trans*-Golgi, the secretory vesicles and the plasma membrane are 8-9.5 nm thick (Grove *et al.,* 1968 (**ref**. 26); Morré and Mollenhauer, 1974 (**ref**. 37)). Moreover, targeting related to TMD length was previously illustrated by studying the location of reporter proteins after varying the length of their TMD, in animal systems (Munro 1991, 1995a,1995b (**ref**. 38)) and, for type I proteins, also in plant cells (Brandizzi et al., 2002a (**ref**. 9)). This implies, the membrane of a specific compartment can only accommodate hydrophobic TMDs of the matching length

Comparisons revealed that the length of Golgi protein TMD were on average 5 aa shorter than those of plasma membrane proteins (Masibay *et al.,* 1993 (**ref**. 41); Munro, 1995a (**ref**. 48)). Several examples are in favour of this model. An increase of the length of the TMD of rat a-2,6-sialyltransferase and bovine b-1,4-galactosyltransferase reduced the Golgi retention of these glycosyltransferase. In addition, a synthetic type I TMD made of 17 leucines resulted in Golgi retention of the lymphocyte surface antigen CD8 extracellular domain whereas a 23 leucine TMD was found in increased amounts at the cell surface (Masibay *et al.,* 1993 (**ref.** 41); Munro, 1991 (**ref**. 47), 1995b (**ref**. 49)). Furthermore, incremental increases in the length of the 18 amino acids a-2,6-sialyltransferase TMD by insertion of 1-9 hydrophobic amino acids also resulted in increased cell surface expression of similar a-2,6-sialyltransferase-lysosyme chimeras, while the decrease in the length of a plasma membrane protein TMD led to its increased retention in the Golgi (Munro, 1995b (**ref**. 49)).

In plant cells, preliminary data in favor of a subcompartmentation of membrane proteins along the endomembrane system related to the TMD length was obtained by varying the length of TMDs in two type I membrane proteins fused to GFP (Brandizzi *et al.,* 2002a (ref. 9)).

First, the human lysosomal membrane protein LAMP1 containing a 23 aa TMD was fused to GFP and was expressed in tobacco leaves. The fusion was located in the plasma membrane. In contrast, when the TMD was shortened to 20 and 17 aa, the GFP chimeras were localized to the Golgi and ER membranes, respectively. Secondly the 19 aa long TMD of the vacuolar sorting receptor BP80 targeted GFP to the Golgi whereas a lengthened TMD of 22 aa targeted GFP to the plasma membrane.

The TMD length of the type II membrane protein Manl was investigated in order to know how it could affect its sub-compartmentation in the Golgi. In particular, the TMD length was increased from 16 to 23 aa by duplicating the seven last aa of this domain. In contrast with their homologues containing a 16 aa TMD, which were located in the ER and the cis-half of the Golgi apparatus, chimeric proteins with a 23 aa TMD were localized exclusively to the Golgi and more precisely in the *trans*-half of the Golgi stacks

In the present example, the information required for Manl targeting is contained within a 20 amino acid (aa) sequence including the 16 aa TMD. To investigate whether the length of the TMD could play a key role in the targeting of this type II membrane protein in the early plant secretory pathway, two fusion proteins, ManTMD23-GFP and Man99TMD23-GFP were designed, where the TMD of Manl was lengthened from 16 to 23 aa by duplication of its last seven aa (annexed figure 4). ManTMD23-GFP and Man99TMD23-GFP were expressed in BY-2 suspension cultured cells and in tobacco leaf epidermal cells. In both plant expression systems, ManTMD23-GFP and Man99TMD23-GFP were exclusively located in bright spots (annexed figure 10A, 10B, 10D), sensitive to the fungal toxin brefeldin A (50 µg.mL⁻¹, 2h, annexed figure 11 C). The expression patterns of ManTMD23-GFP and Man99TMD23-GFP were similar to either the XylT-GFP fusion (annexed figure 10), or the ST52-mRFP fusion (annexed figure 10) both located exclusively in the Golgi in BY-2 suspension cultured cells and tobacco leaf epidermal cells as it has been confirmed previously by electron microscopy (Boevink *et al*., 1998 (**ref**. 7); Pagny *et al.,* 2003 (**ref**. 63)).

To further investigate the sub-compartmentation of ManTMD23-GFP and Man99TMD23-GFP, stable BY-2 suspension cultured cells co-expressing one or the other of these GFP fusions and ST52-mRFP were established. In the merged images, it was impossible to separate green spots from red spots, suggesting that the GFP-fusions containing a 23 aa TMD have moved forward within the Golgi toward the *trans*-face so that they co-localize with ST52-mRFP at the confocal level (compare annexed figure 10). Interestingly, the spot patterns were similar in cortical images (annexed figure 8D-F) compared to cross sections (annexed figures 10G-I) reinforcing the assumption the Man-GFP fusions with a longer TMD and the trans-Golgi marker ST52-mRFP perfectly co-localize. In contrast, the medial Golgi marker (XylT35-GFP) and the *trans* Golgi marker (ST52-mRFP) resulted in fluorescent spots that did not overlap perfectly in the merged image (annexed figure 10J-L).

Electron microscopy coupled to immunogold-labeling with polyclonal anti-GFP antibodies allowed us to to determine more precisely the intra-Golgi localization of these fusion proteins. As illustrated in annexed figure 11, the Man99-GFP fusion accumulated mainly to the *cis-*side of the Golgi (annexed figure 11 B) whereas the Man99TMD23-GFP fusions are principally localized to the *trans*-side of the Golgi (annexed figure 11 C).

Similar results were obtained with ManI-GFP and ManTMD23-GFP (data not shown). Control experiments using the pre-immune serum or wild-type tobacco BY-2 suspension- cultured cells showed no or very little non specific Golgi labeling (annexed figure 11A)..

This results demonstrating that the TMD is sufficient to confer an identical localization as the full length protein ManI, the data suggest that the length of the TMD is a crucial factor for precise positioning of this type II membrane protein within the Golgi stacks and the ER. Thus, protein-lipid interactions are expected to play a key role in ManI targeting within the secretory system.

Interestingly, these results also clearly point out differences in TMD length requirements in the targeting of type I and type II membrane protein in the plant secretory system. Indeed, the 23 aa TMD of XylT (Dirnberger *et al.,* 2002 **(ref.** 15); Pagny *et al.,* 2003 (**ref**. 50)) or the 22 aa TMD of ManII (Strasser *et* a/., 2006 (**ref**. 60)) targets GFP to the Golgi only. In addition, in the present experiment, ManI with a lengthened TMD (23 aa) was also detected exclusively in the Golgi. In contrast the 23 aa TMD of a type I membrane protein and the lengthened 22 aa TMD of BP80 target GFP to the plasma membrane in (Brandizzi *et al.,* 2002a (**ref**. 8)).

The TMD length requirements for a membrane protein to stay in a membrane with a given thickness might depend on the topology of the protein (type I or type II).

While these experiments clearly demonstrate the role of TMD length in Manl protein targeting, but others experiment show that other enzymes require other signals for proper localization. For example, contradicting the trend to longer TMDs in the later parts of the Golgi, the ST52-GFP fusion with an 18 aa TMD is found further downstream in the *trans* Golgi (Boevink *et al.,* 1998 (**ref**. 5); Wee *et al.,* 1999 (**ref**. 63)) than the XylT35-GFP fusion with a 23 aa TMD (Pagny *et al.,* 2003 (**ref**. 50)).

Similar results were obtained in other plant systems used for transient expression. Indeed, Man99-GFP was located in the Golgi and ER in soybean (annexed figure 12A) or in tomato (annexed figure 12C), whereas Man99TMD23-GFP was found almost exclusively in the Golgi in both expression systems (annexed figure 12B and 12D).

Even in a situation illustrated here with GCSI, whose TMD is one' of the shortest identified so far for a plant glycosylation enzyme and allows for a localization in the ER, the experiment shown that additional information contained in the CT are required for proper targeting. Thus, in *silico* analyses and mutagenesis studies performed on GCSI are not consistent with TMD length as the only signal for compartmentation of glycosylation enzymes in the plant secretory system.

In other words, while the TMD length has a key role for Manl targeting in the ER and the cis-Golgi, results obtained with GCSI illustrates that specific localization of some membrane proteins in the ER or Golgi membranes could also depend on both protein-lipid (via the TMD) and protein-protein (via special sorting motifs) interactions. The identification of cytosolic partners such as Golgi matrix proteins or cytoplasmic regulators permit to explain mechanisms involved in this second model for partitioning the N-glycan maturation enzymes along the plant secretory pathway.

The large collection of enzymes localizing to different levels in the Golgi has allowed testing the question whether all cisternae within the Golgi stack fuse with the ER in response to treatment with the fungal toxin brefeldin A (BFA). Indeed the Man-GFP fusions containing either a 16 or 23 aa TMD and ST52-mRFP all moved back to the ER or in Golgi clusters over a 2h time-course experiment with BFA. These conclusions are consistent with previously published results (annexed in figure 12) (Nebenführ et al., 2002 (**ref**. 43); Ritzenthaler et al., 2002 (**ref**. 54)).

In conclusion, together these results indicate that the TMD length plays a key role in the targeting of Manl to the ER and the cis-Golgi compartments and an increase in the length of the TMD from 16 to 23 aa relocates this type II membrane protein further downstream toward the trans-face of the Golgi (annexed figure 11 D).

### 5. Experiments showing that the late and early Golgi proteins redistribute in the ER in presence of brefeldin A (BFA).

Taking advantage of the large panel of Golgi marker generated during this study, the possibility that Golgi proteins located in different Golgi subcompartments may behave differently after BFA treatment was investigated.

Cells expressing ER soluble or membrane markers (GFP-HDEL or Glu90-GFP, annexed figures 13A-D), the ER /early Golgi marker (D19Man49-GFP, annexed figure 13E-F), the medial Golgi marker (XylT35-GFP, annexed figure 13G-H) or the late Golgi marker (Man99TMD23-GFP or ST52-mRFP, annexed figure 13I-L) were treated with BFA (50mg.mL⁻¹) during 2h. At the end of the BFA treatment, all the fusion proteins accumulated in bright aggregates and in the ER (annexed figure 13E-L) except for the ER markers that were never found in aggregates (annexed figure 13A-D). All markers were relocated in the ER in presence of BFA.

In cells co-expressing, ER/early Golgi or late Golgi proteins with ST52-mRFP, BFA induces the redistribution of both markers into the ER and into Golgi aggregates (annexed figure 14), except for GFP-HDEL (annexed figures 14 A-F) and Glu90-GFP (annexed figure 14 D) that were not found in the aggregates. In some cases, there are subtle differences in timing, but these are not trivial to detect and also not informative with respect to intra-Golgi localization. Furthermore, no significative difference was observed in the fluorescent patterns observed after BFA treatment of cell expressing either a soluble (GFP-HDEL) or a membrane protein (Glu90-GFP) marker (annexed figures 14A-D).

### 6. Experiments showing that the TMD length model does not apply to all type II membrane proteins

To determine whether if the TMD length could be the only Golgi sorting determinant allowing the subcompartmentation of all glycosidases and glycosyltransferases along the plant secretory system, the N-terminal sequences of characterized glycosylation enzymes were compared (annexed figure 14).

This analysis was hampered by the small number of sequences of different enzymes cloned and functionally characterized from a single species as well as a still smaller number of electron microscopy data to correlate TMD lengths and membrane thickness in a single plant system. *In silico* analysis of the N-terminal sequence (CT + TMD) of all plant glycosylation enzymes cloned so far clearly shows a trend for longer TMDs in proteins with the most downstream location in the Golgi stacks (annexed figure 14). For instance, it is interesting to note that none of the enzymes that are supposed to be located in the late Golgi such as a-1,3-fucosyltransferases and a-1,4-fucosyltransferases have a TMD shorter than 20 aa. These results were confirmed with the MENSAT_V1,8, PHOBIUS or PRED_TMR programs (http://bioinf.cs.ucl.ac.uk/psipred/psiform.html and http://biophysics.biol.uoa.gr/PRED-TMR/input.html) which were used for TMD length prediction. However exceptions to this general trend can be noticed when similar glycosylation enzymes from different species are compared, for example the ManI TMDs ranging from 16 aa (soybean) to 20 aa (Arabidopsis).

Based on its short 18 aa TMD that could perfectly fits with the lipid bilayer model to explain its localization in the ER membrane, GluI was selected to check for general applicability of this model. In order to define whether the TMD of Glul was sufficient for its targeting and retention in the ER, most of the luminal part of this glycosidase was deleted (containing the catalytic domain) and fused its first N-terminal 90 aa (CT + TMD + S) to GFP to get the fusion protein Glu90-GFP (annexed figure 4). When this fusion was expressed in tobacco cells, the ER was highlighted (annexed figures 15A and 15B) in a pattern very similar to the one obtained with the full length construct Glul-GFP and the GFP-HDEL construct (compare micrographs 14A and 14B to 4G and to 4D, 4E and 12C).

This result clearly shows that Glul targeting to the ER depends on signals located within the CT, the TMD and/or the 21 luminal aa remaining in this truncated protein.

In a further attempt at defining the minimal protein sequence required for localization of Glul in the ER, the first N-terminal 13 aa from the Glu90-GFP construct have been deleted to obtain D13Glu90-GFP (annexed figure4). When this fusion was expressed in tobacco suspension cultured or leaf epidermal cells, the chimeric protein was located exclusively in Golgi-like spots (annexed figures 15D and 15E) as observed for XylT-GFP (annexed figures 6I and 9E) and ST52-GFP (annexed figure 15F).

In conclusion, the 18 aa long TMD of Glul is not sufficient to target this glycosidase in the ER membrane and additional information contained in the first 13 aa of the CT is required for the normal localization of this glycosylation enzyme in the secretory system.

This result provides experimental proof that factors other than TMD length influence the positioning of glycosylation enzymes in the early secretory pathway.

### 7. Localisation of Arabidopsis thaliana GCSI type II membrane protein

**GCSI** accumulates strictly in the tobacco endoplasmic reticulum

Arabidopsis thaliana GCSI is a type II membrane protein, consisting of a 51 amino acid cytosolic tail, an about 18 residues transmembrane domain and a large catalytic domain directed toward the lumen (Boisson et al., 2001 (ref. 6)). To investigate the location of this glycosidase, the localization of a GFP fusion to a full length GCSI (annexed figure 15) was studied after stable expression in tobacco BY-2 cells (annexed figures 16A and 16 B; annexed figures 17 A and 17 B). Fluorescence of a full-length GCSI-GFP fusion construct was detected by confocal laser scanning microscopy exclusively in a reticulate network throughout the cytoplasm that was indistinguishable from the ER network stained by a GFP-HDEL construct (annexed figures 16 C and 16 D).

These results are consistent with the trimming of the first sugar residue from the precursor oligosaccharide in the ER immediately after its attachment to the nascent glycoprotein, and with what was shown for human Glul in COS cells (Hardt *et al.,* 2003 (**ref**. 28)). Furthermore, no significative difference was observed in the fluorescent patterns of cell expressing either a soluble (GFP-HDEL) or a membrane protein (GCSI-GFP) marker observed without (annexed figures 16 A to D).

### 8. Experiment showing that the first 90 amino acids of GCSI are sufficient to retain GFP in the ER.

To understand the mechanisms allowing the selective retention of GCSI in the ER, the role of the luminal domain in GCSI targeting was first investigated. In order to determine if the portion of GCSI located in the ER lumen plays a role in the targeting of this glycosidase to the ER, the first 150 amino acids (aa) (CT+TMD+stem81aa) or the first 90 aa (CT+TMD+stem21aa) of GCSI were fused to GFP and the corresponding chimeric proteins were named GCS150 and GCS90, respectively (annexed in figure 15). GCS150 and GCS90 were either stably expressed in BY-2 suspension cultured cells or transiently expressed in tobacco leaf epidermal cells by Agro-infiltration and were both observed in the ER in both expression systems (annexed in figure 17 A,C,E and 17B,D,F respectively) exactly as previously observed for the full length construct (annexed in Figure 16 A,B). It is important to note that when these truncated fusions were transiently expressed in tobacco leaf epidermal cells, the ER labeling was still observed five days after transformation when the overall expression level is already strongly declining and whereas Golgi fusions analyzed in the same condition are located exclusively in the Golgi (Saint-Jore-Dupas *et al.,* 2006; data not shown). This data show the catalytic luminal domain of glucosidase I is not necessary for the ER location of the enzyme and the first 90 amino acids of GCSI are sufficient to retain GFP in the ER

### 9. Experiments showing that the cytoplasmic tail contains an ER retention sequence

The N-terminal cytosolic region of many membrane proteins residing in the mammalian and yeast ER contains signals which facilitate either the strict retention (Nilsson *et al.,* 1994 (*ref*. 46); Opat *et al.,* 2000 (**ref**. 48), Hardt *et al.,* 2003 (**ref**. 28); Ciczora *et al.,* 2005(**ref.** 12)), or their retrieval from the Golgi back to the ER (Teasdale and Jackson, 1996 (**ref**.62); Zerangue *et al*., 1999 (**ref**.66)) whereas some others promote the export from the ER to the Golgi (Giraudo and Maccioni, 2003 (**ref**.20)). In plants, only few studies demonstrate the presence of ER export sequence in the CT of membrane proteins (Contreras *et al.,* 2004 (**ref**. 4613); Yuasa *et al.,* 2005 (**ref**. 65); Hanton *et al.,* 2005b (**ref**. 27)) and **ref.**er to the characterization of cytosolic motifs responsible of membrane protein ER retention (Benghezal *et al.,* 2000(**ref**.4); McCartney *et al.,* 2004(**ref**.35)).

To define more precisely the sequence containing the ER targeting information in GCSI N-terminus, the first 13 amino acids located at the N-terminal end of GCS90 was first deleted and the resulting chimeric protein was named D13GCS90 (annexed in figure 15). This truncation removed two potential dibasic motifs RR or RXR that might function in ER retention although other potential ER-retention signals (RR or KXK) remained in the CT of this fusion protein. In parallel, the first 13 N-terminal amino acids peptides of GCSI were fused to a Golgi reporter protein (XYLT35, annexed Figure 15) previously located in the medial Golgi (Pagny *et al.,* 2003(**ref**. 50)). In the experimental conditions, used in the present study, XYLT35 was confirmed to be exclusively accumulated in the Golgi apparatus of BY-2 tobacco cells as it is illustrated in Figure 18 G to H.

The two constructs (D13GCS90 and 13GCS-XYT35) were also stably expressed in BY-2 cells. The deletion of the first 13 amino acid of the cytosolic tail relocated the GCS90 protein into bright spots (Figure 18C-D, Figure 19A) similar to the one observed with the Golgi marker XYLT35 (Figure 18E-F). In addition, the D13GCS90 did not colocalize with the ER marker mRFP-HDEL (Figure 19B) but perfectly colocalized with the ST-mRFP Golgi marker (Figure 19C) after transient expression in tobacco leaf epidermal cell. Interestingly, the fusion of the 13 N-terminal amino acids of GCSI to the XYLT35 Golgi marker blocked the reporter protein in the ER (Figure 18G-H; Figure 19G). The GCS13-XYLT35 fusion protein labeled the ER like the GCS90 construct (Figure 18A-D) and colocalized with the mRFP-HDEL ER marker (Figure 19H). In addition to a strong ER labeling, a few bright spots were also observed when GCS13-XYLT35 was expressed in tobacco leaf epidermal cells. These spots move and colocalize with the ST-mRFP Golgi marker (Figure 19I).

In conclusion, the first 13 amino acid of the GCSI are necessary to retain the GCS90 fusion protein in the ER and are sufficient to relocate a Golgi marker in the ER.

### 10. Experiments showing that a cytosolic arginine-rich sequence is an ER retention signal in plants

In order to further investigate whether arginine residues are essential for ER retention in the 13 N-terminal amino acids of *At*GCSI, the peptide for either and arginine rich domain located in the N-terminal end of the human homologue of *At*GCSI or an arginine rich domain located in the cytosolic C-terminal end of a type I plant ER resident membrane protein, A. *thaliana* calnexin was changed.

To investigate the capability of the arginine-rich peptide located at the N-terminal end of human GCSI to be recognized in a plant cell, the first N-terminal 13 amino acids of GCS90 was substituted by the first N-terminal 10 amino acids of human GCSI (Hs10-GCS90, Figure 15). When transiently expressed in tobacco leaf epidermal cells, Hs10-GCS90 was located in the ER indicating that the N-terminal cytosolic region is perfectly recognized by the plant secretory system (Figure 17J to L).

In a second time, to investigate if a similar arginine-rich motif carried out by a type I membrane protein, could mediate the targeting of a type II protein in the ER, the last 11 amino acids located at the C-terminal end of A. *thaliana* calnexin was fused to the N-terminal end of the Golgi marker XYLT35 (CNX11-XYLT35, Figure 15, Table 1). CNX11-XYLT35 was transiently expressed in tobacco leaf epidermal cells. As illustrated in Figure 19M to O, the arginine rich C-terminal peptide of a type I membrane protein is sufficient to retain a type II XYLT35 Golgi marker in the ER (Figure 19 M to N). Note same Golgi labeling is also detected (Figure 19 O) as for the GCS13-XYLT35 fusion (Figure 19 I).

### 11 Experiments showing that the arginine residues in the cytosolic tail of GCSI contain ER localization information.

In order to define more precisely the role of the four arginine residues within the 13 first amino acids of GCSI, these residues were replaced by either leucine or alanine residues using PCR site-directed mutagenesis (see Table 1 for the construct details) and the resulting fusion proteins were expressed in tobacco cells.

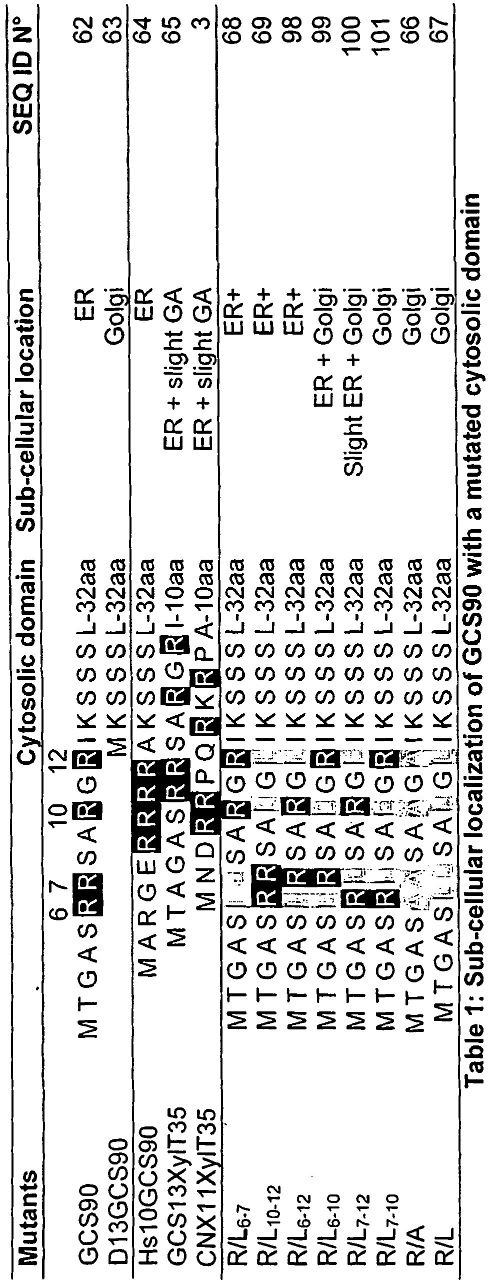

While GCS90 was exclusively located in the ER and perfectly co-localized with the ER marker mRFP-HDEL (Figure 20 A,B), but not with the Golgi marker ST-52-mRFP (Figure 20 C), when Arg-6, Arg-7, Arg-10 and Arg-12 were replaced with alanine residues, R/LGCS90 highlighted exclusively bright spots (Figure 20 D). These spots correspond to the Golgi apparatus as illustrated here from colocalization with the Golgi marker ST-mRFP (Figure 20 F). No ER labeling was detected (Figure 20 E). The same effect on sub-cellular localization was observed after substitution of these four arginine residues by four leucine residues, (Figure 20G-I). These observations indicate that the four arginines located at position 6-7-10 and 12 to the N-terminus are likely to encode for structural information responsible for ER residency of GCS90.

In a second step, to define whether (RR) and (RXR) act as two independent signals, Arg-6 and Arg-7 or Arg-10 and Arg-12 have been substituted with leucine residues. The presence of either an RR motif (Arg-6 and Arg-7 in construct R/L₁₀₋₁₂GCS90) or an RXR motif (Arg-10 and Arg-12, in construct R/L₆₋₇GCS90) but also the RXXR motif (Arg-7 and Arg-10 in construct R/L₆₋₁₂GSC90) was sufficient for ER retention of the fusion protein (Figure 20J to L and M to O). However, in these three cases in addition to ER labeling, fluorescent spots that were distinct to Golgi stacks have been observed (Figure 20 L and 20 O).

### 12. Experiments showing that fusion proteins harboring RR, RXR or RXXR motifs accumulate in the ER and in fluorescent spots associated with the Golgi

The next step was to identify the structure labeled as fluorescent spots by the R/L₆₋₇GCS90, R/L₁₀₋₁₂GCS90 and R/L₆₋₁₂GSC90 chimeric fusion protein. As illustrated with the R/L_{6/7}GCS90 proteins, GFP fluorescence accumulated in the ER and in fluorescent structures which are smaller that Golgi stacks (Figure 20 J to L and Figure 21 A to B). To define more precisely the localization of these structures in the cell, the mRFP-HDEL ER marker, the ST52-mRFP Golgi marker and either R/L₆₋₇GCS90, R/L₁₀₋₁₂GCS90 or R/L₆₋₁₂GSC90 fusion proteins were expressed simultaneously.

The results are illustrated in Figure 21A-B and show the small fluorescent spots are closely associated to but distinct to Golgi stacks. Units formed by association of one dictyosome and one spot move together along the ER and they never dissociate.

Considering these results, the R/L₆₋₇GCS90, R/L₁₀₋₁₂GCS90 and R/L₆₋₁₂GSC90 fusion proteins were accumulated in the ER in small intermediate domains located between the ER and the Golgi, from which ER resident soluble proteins are excluded (Figure 20K and 20N). These domains are strongly associated with the Golgi and move with the Golgi stacks along the ER cortical network, so these domains could be ER-exit-sites (ERES) as described in Yang *et al.,* 2005. To verify this R/L₆₋₇GCS90, R/L₁₀₋₁₂GCS90 and R/L₆₋₁₂GSC90 fusion proteins were co-expressed with Sar1p-mRFP in tobacco leaf epidermal cells.

Unfortunately, no recruitment of Sar1p-mRFP at the fluorescent spots was shown (Figure 22 A to C). Nevertheless, Sar1p has been shown to be involved in the transport of mutated form of GCS90. Indeed, as illustrated figure 22 J to L, when R/LGCS90 was co-expressed with Sar1p/GTP-mRFP, Sar1p was accumulated at the ER and at fluorescent spots (Figure 22K).

In contrast, GCS90 did not recruit Sar1p-mRFP, as Sar1p-mRFP expressed alone is in the ER (data not shown) and the co-expression of GCS90 with Sar1p-RFP did not modify GCS90 labeling (Figure 22D to I). Moreover, the expression of the mutated form Sar1p/GTP-mRFP led to the retention of Golgi R/LGCS90 in the ER.

In conclusion, the transport of R/LGCS90 is regulated by the cytosolic protein Sar1p. However, the small domains labeled with fusion proteins harboring one RR, RXR or RXXR motif are not associated with the Sar1p-mRFP although they are located between the ER and the Golgi compartments.

### 13. Experiments showing that ER retention of AtGCSI does not depend on the N-terminal arginine motifs only

In the present exemple the GCSI arginine-motifs fused to a Golgi reporter protein localized it to the ER. However, there is no evidence that these signals are the main retention signals involved in the ER localization of the full length GCSI.

Based on the observation that replacement of Arg-6, Arg-7, Arg-10 and Arg-12 by leucine or alanine resulted in disruption of ER-directing information for the GCS90 construct, the N-terminal 13 aa were deleted from the full-length sequence of the GCSI to evaluate the significance of the motifs (RXR and RR) in ER targeting of wild type enzyme (D13GCSI, Figure 15). In addition, the GCS150 truncated form was synthesized lacking the amino acid residues 1-13 at the N-terminal end, (D13GCS150, Figure 15). Fusion proteins were then expressed transiently in leaf epidermal cells. In contrast to the D13GCS90 which was accumulated in the Golgi apparatus, both D13GCSI and D13GCS150 were exclusively located in the ER (Figure 23D-E). Moreover, when the chimeric protein was expressed simultaneously in tobacco cells with an ER-marker (mRFP-HDEL), in contrast to GCS90, both D13GCSI and D13GCS150 were not detected in the Golgi and both fluorescence signals were observed in the ER and perfectly co-localized with mRFP-HDEL (Figure 23G-L). These results are consistent with previous studies of Hardt *et al.,* (2003) (ref. 28) that demonstrated the human GCSI contains a functional arginine-based signal that is not required to localize the full length enzyme. The differences in labeling observed between GCS90 (Figure 23F, 23I and 23L), GCSI (Figure 23D, 23G and 23J) and GCS150 (Figure 23E, 23H and 23K) suggested that the key information determining ER localization must be contained in the luminal polypeptide chain of GCSI, and more probably in the stem domain, between the amino acid residues 70-150.

In order to validate this hypothesis, the 81 amino acid residues 70-150 or the 61 aa 90-150 were fused to the luminal domain of the medial Golgi marker XYLT35 (Figure 24A) and transiently expressed these new proteins in tobacco leaf epidermal cells (XYLT35-GCS81 and XYLT35-GCS60, Figure 24B,C). Most of the GFP labeling was found in the ER, the "remnant" being in the Golgi.

It has been shown in mammalian cells that ER residency can be accomplished by direct retention involving association of protein subunits to give large oligomeric complexes via their transmembrane and/or luminal domains, as previously described in the kin-recognition model for Golgi-located membrane proteins. (Nilsson *et al.,* 1994 (ref. 46); Opat *et al.,* 2000 (ref. 48)). These large protein oligomers were assumed to escape packaging into transport vesicles, thus preventing their export from the organelle. This type of mechanism may be functional in the ER retention of subunit components of the hetero-oligomeric oligosaccharyltransferase complex but were not described in plants yet.

In conclusion, both the cytosolic tail and the luminal domain of AtGCSI contain ER targeting determinants Consistent with its specificity and in complete agreement with observations made in other eukaryotic systems, the results demonstrante that AtGCSI is localized in the plant ER and exclusively in this compartment. Other glycosylation enzymes acting early in the plant N-glycan maturation such as N-acetylglucosaminyltransferasel (GNT1) and a1,2 mannosidase (ManI) have been shown to be located both in the ER and in the *cis*-Golgi . The GNTI and ManI, ER and *cis*-Golgi have been shown to contain targeting information resides in the cytosolic tail whereas the first 13 N-terminal amino acids of GCSI cytosolic tail contain ER targeting information. The present exemple shows which were the key amino-acids in the cytosolic tail involved in ER targeting but also demonstrates that an arginine-rich cytosolic tail is not the only ER targeting determinant in the whole protein sequence.

Indeed, the deletion of the arginine-rich sequence from GCS150, does not permit the exit of the ER to a later compartment such as the Golgi and D13GCS90 is still exclusively located in the ER.

Together these results indicate that at least two domains, sufficient to confer ER retention to a Golgi type II membrane protein, coexist in the GCSI sequence. Deletions in the luminal domain of GCSI have indicated that information for ER retention is contained in a 60 amino acid peptide located between the amino acids 90 and 150 in the GCSI sequence.

Comparable results were obtained for human glucosidase I concerning the presence of a luminal ER targeting signal but to date the luminal sequence involved is not yet characterized (Hardt *et al.,* 2003 (**ref**. 28)). As mentioned above, when associated to the Golgi reporter protein XYLT35 the plant luminal peptide is sufficient to relocate XYLT35 to the ER. The luminal domain of GCSI could facilitate the formation of complexes between *At*GCSI and soluble and/or membrane bound ER resident proteins. However, in contradiction with the kin recognition model, it has been shown that some large protein complexes are located in the plasma membrane and have to be completely assembled to be transported out of the ER.

In fact, it does not seem necessary to consider the size of these complexes to explain retention. Indeed, due to the very high concentration of proteins in the ER lumen, it is probably more difficult to leave the ER than to stay inside for a protein having the capacity to form protein-protein complexes. As previously described for another ER chaperone system involving BiP (we are currently investigating whether the nascent glycoprotein folding machinery could form a large multi protein complex made of GCSI, glucosidases II, calnexin, calreticulin and ERp57 in the plant ER

### 14. The cytosolic tail of GCSI contains three di-arginine signals independently sufficient for ER retention

It has been shown that the arginine-rich sequence (MTAGASRRSARGRI (SEQ ID N°1) is sufficient to target the Golgi marker XYL35 in the ER. Based on previous studies on membrane protein targeting in the secretory pathway of mammalian cells, the present example was made to identify if the four arginines residues were containing ER retention information. Mutation of the four arginines into alanines or leucines residues completely abolished ER retention capacity of this sequence as L/GGCS90 was found in the Golgi, thus validating the key role of arginine residues in ER retention.

Further analysis based on directed mutagenesis in this arginine-rich sequence has shown that in fact two arginine residues (RR, RXR or RXXR SEQ ID N°70) are sufficient to confer ER retention of GCS90 and that consequently the 13 aa peptide contains three distinct di-arginine motifs sufficient for ER retention that co-exist in the cytosolic tail of GCSI. Interestingly, when only one out these three di-arginine motifs is present in the cytosolic tail of a GCS90, fluorescence is detected not only in the ER but also in small spots associated to- and moving with the Golgi stacks along ER tracks.

In addition, a soluble ER marker protein GFP- HDEL is excluded from these small spots. The hypothesis was that these small spots where R/L₆₋₇GCS90, R/L₆₋₁₂GCS90 and R/L₁₀₋₁₂GCS90 are detected, correspond to secretion units (ERES) located at the ER surface and mediating material exchange between the ER and the Golgi apparatus (Runion *et al.,* 2006 (ref. 55). Further investigations validating this hypothesis would be in favor of an ER/Golgi transport model based on a single secretion unit connected to and moving with a dictyosome at the ER surface. However, mini-spots do not colocalize with Sar1p. Di-arginine motifs have been extensively studied in mammalian membrane proteins but they were never characterized before the present study in their plant homologues. Interestingly a di-arginine motif previously identified in human GCSI has been shown to mediate ER retention in plant cell (Hardt *et al.,* 2003 (ref. 28)).

However di-arginine motifs identified in AtGCSI look more flexible than their human homologue. Indeed the di-arginine motif of human a glucosidase I is made of two arginine residues in position +7 and +8 and of a basic amino acid in position +9. In AtGCSI the distance between two arginine residues looks more flexible but cannot exceed two amino acids for a good efficiency. Furthermore this motif should be in a close proximity of the N-terminal end of the protein. Indeed, in GCSI a di-arginine motif RR in position +23 and +24 is still present in the fusion protein D13GCSS90-GFP but is not sufficient to confer ER retention.

Finally a comparison of GCSI sequences available has shown that di-arginine motifs at the terminal end of these ER resident proteins are highly conserved (Table 2, Boisson *et al.,* 2001 **(ref.** 6) ; Hong *et al.,* 2004 (ref.30)).

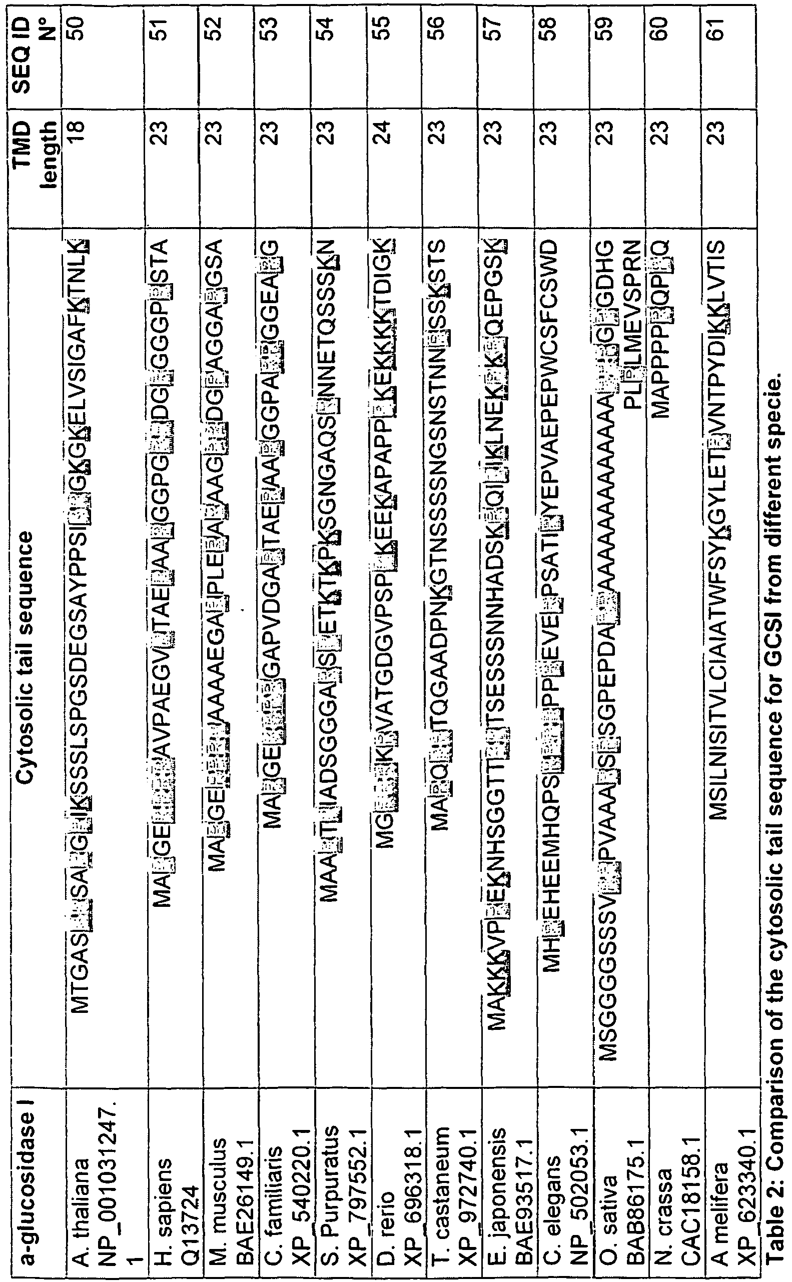

### Example 2: Description of identify sequences

As shown in the previous exemples, each signal listed in Table 3, is sufficient to target a reporter protein such as the green fluorescent protein to the ER and/or the GA (see annexed figures 25A and 25B).

**Table 3: LOC = Localisation of reporter protein when fused to the signal (cf annexed figure 1)**

| **Signal** | **Sequence** | **LOC.** | **SEQ IDn°** |
|---|---|---|---|
| **Signal 1** | MTGASRRSARGRI | ER | 1 |
| First 13 amino acids of Arabidopsis *thaliana* glucosidase I | | | |
| **Signal 2** | MARGERRRRA | ER | 2 |
| First 10 amino acids of *Homo sapiens* glucosidase I | | | |
| **Signal 3** | MNDRRPQRKRPA | ER | 3 |
| Last 11 amino acids at the C-terminal end of At calnexin | | | |
| **Signal 4** | | ER | 8 |
| First 150 amino acids of *Ath* glucosidase I | | | |
| **Signal 5** | MARGSRSVGS SSSKWRYCNP SYYLKRPKRL ALLFIVFVCV SFVFWDRQT | ER+GA | 32 |
| First 49 amino acids of *Glycine max* mannosidase I | | | |
| **Signal 6** | | ER+GA | 33 |
| First 99 amino acids of *Gm* mannosidase I | | | |
| **Signal 7** | MAAALALLFIVFVCVSFVFWDR | ER+GA | 34 |
| Transmembrane domain of *Gm* mannosidase I | | | |
| | | | |
| **Signal 8** | | ER+GA | 35 |
| First 68 amino acids of Ath alpha 1,3 fucosyltransferase 2 | | | |
| **Signal 9** | MRGYKFCCDF RYLLILAAVA FIYIQMRLFA TQSEYADR | ER+GA | 36 |
| First 38 amino acids of *Nicotiana tabacum* N-acetylglucosaminyltransferase I | | | |
| **Signal 11** | MGVFSNLRGP KIGLTHEELP WANGSTSSS SSPSSFKRKV STFLPICVAL VVIIEIGFLC RLDNASTS | Medial Golgi | 37 |
| First 1 68 amino acid of *Ath* alpha 1,3 fucosyltransferase | | | |
| **Signal 10** | MLVMPQPPKP FNTITITIMI AFTFFLLFLT GFLQFPSISP S | *Trans Golgi* | 38 |
| First 41 amino acid of *Medicago sativa* alpha 1,4 fucosyltransferase 1 | | | |
| **Signal 12** | | *Trans* Golgi | 39 |
| First 99 amino acids of G*m* mannosidase I with a modified transmembrane domain | | | |

As disclose in the previouly, SEQ ID N°1 to 3 represents a set of anchoring sequences for membrane protein targeting to the ER. These sequences are located in the cytosolic tail of the membrane protein located at the C- or N-terminal end.

SEQ ID n°1: MTAGASRRSARGRI

SEQ ID n°2: MARGERRRRA

SEQ ID n°3: MNDRRPQRKRPA

It has been shown that the following di-Arg motif(s) motifs sequences present in these cytosolic tails (SED ID N°1 to 3) have been sufficient to retain a reporter membrane protein in the ER:

Motif sequences:

| | **SEQ ID** | | **SEQ ID** | | **SEQ ID** |
|---|---|---|---|---|---|
| RR | | RRXXRXR | 76 | RRRK | 88 |
| RXR | | RKXXRXR | 77 | RRKK | 89 |
| RXXR | 70 | RRXXRXK | 78 | RKKR | 90 |
| RXXXR | 71 | RRXXKXR | 79 | KKRR | 91 |
| RK | | KRXXRXR | 80 | | |
| RXK | | KKXXRXR | 81 | | |
| RXXK | 72 | RRXXKXK | 82 | | |
| RXXXK | 73 | RKXXKXR | 83 | | |
| KR | | RKXXRXK | 84 | | |
| KXR | | RRRR | 85 | | |
| KXXR | 74 | RKRR | 86 | | |
| KXXXR | 75 | RRKR | 87 | | |

As illustrated in Figure 25, these di-Arg motif can be located at the N- or C-terminal part of respectively, the type II or type I membrane protein

The other following sequences have been tested accordingly :

SED ID N° 4 to 7 are responsible of a strict retention of the recombinant membrane polypeptide in the ER. These sequences are located in the ER lumen.

SEQ ID n°4:

FQGEHKESLYWGTYRPHVYFGVRARTPLSLVAGLMWLGVKD EMYVMRHFC

SEQ ID n°5:

FQGDHKESLYWGTYRPNVYLGIRARTPLSLIAGIMWIGAKNGQ YFLRHVC

SEQ ID n°6:

ESDASLLWGTYRPQIYFGLRPRLPGSLLTGLAWFGLQDYSDF QHIRHQC

SEQ ID n°7:

ERSNRLFWGTYRPGIYFGMKHRSPISLLFGVMWTVQDAENFA FRHSC

It is estimated that every sequence having at least 70 % of homology with the SEQ IDn°4 of the present invention have the same effect that the targeting signal of the present invention.

In figure 26, SEQ IDn° 4 to 7 located in the stem (S3) is responsible for a strict retention of membrane proteins in the ER. S3 is a sequence located near the transmembrane domain.

A strict retention of the recombinant polypeptide in the ER (see figure 26) is done by the conjunction of one of the SEQ ID N° 1 to 3, containing the Di-Arg motif, and one of the SEQ ID N°4 to 7, as illustrated in the SEQ ID N°8, and is responsible of membrane protein strict retention in the ER and stabilization of recombinant proteins.

A transmembrane domain (GS2) of from 16 to 23 amino acids has been shown to be sufficient to address a protein to the Golgi. The use of this domain is sufficient to anchor a recombinant protein or an enzyme in the Golgi membranes. Examples of tested transmembrane domains (GS2) that are included in the peptidic signal are as following:

| **signal** | **SEQUENCE** | | **SEQ IDn°** |
|---|---|---|---|
| 16 | XXXLALLFIVFVCVSFVFWDR | cis | 9 |
| 20 | XXRYLLILAAVAFIYIQMRLFATQS | cis | 10 |
| 18 | XXXLGILFAVTLSIVLMLVSVXXX | | 11 |
| 19 | XXKIFLYMLLLNSLFLIIYFVFH | median | 12 |
| 21 | XXXRKLSNLLPLCVALVVAEIGFLG | cis | 13 |
| 21 | XXXRKVSTFLPICVALVVIIEIGFLC | median | 14 |
| 23 | XXFNTITITIMIAFTFFLLFLTGFLQFXX | trans | 15 |
| 23 | XXKRLALLFIVFVCVSFVFWCVSFVFWDR | trans | 16 |

Arrangement between the cytosolic tail (GS1), the transmembrane domain (GS2) and the stem (GS3) from Golgi enzymes is responsible of membrane protein retention in the cis, medial or trans Golgi (see annexed figure 26, bottom schematic. This arrangement allows subcompartimentation of enzymes or recombinant protein in the Golgi apparatus. Example of such peptide signal sequences are as following:

**GS1**

SEQ ID n°17: MARGSRSVGSSSSKWRYCNPSYYLKRPKR

SEQ ID n°18: MGVFSNLRGPRAGATHDEFPATNGSPSSSS SPSSSIKRK

SEQ ID n°19: MRGYKFCCDFR

SEQ ID n°20:

MGVFSNLRGPKIGLTHEELPVVANGSTSSSSSPSSFKRK

SEQ ID n°21: MLVMPQPPKPFN

SEQ ID n°22: MARGSRSVGSSSSKWRYCNPSYYLKRPKR

SEQ ID n°23: MANLWKKQRLRDTGLCR

Exemples of peptide signal issued from the GS3 domain are the followings:

| **SEQ ID n°** | **GS3** |
|---|---|
| 24 | |
| 25 | |
| 26 | |
| 27 | |
| 28 | |
| 29 | |
| 30 | |
| 31 | |

These signals have been used to target the expression of several membrane proteins in tobacco, soybean, tomato or radish cells after transient or stable transformation. These signals can be added either to the N-terminal (type II membrane protein) or to the C-terminal end (type I membrane protein) of membrane proteins with the same targeting efficiency and specificity (it seems that the signal could be also added to a type III or IV membrane protein).

**Exemple of arrangement between GS1/GS2/GS3**

| | | |
|---|---|---|
| SEQ IDn°32 | | ER+ CIS GA |
| SEQ IDn°33 | | ER+ CIS GA |
| SEQ IDn°34 | MAAALALLFIVFVCVSFVFWDR | ER+ CIS GA |
| SEQ IDn°35 | | ER+ CIS GA |
| SEQ IDn°36 | MRGYKFCCDF RYLLILAAVA FIYIQMRLFA TQSEYADR | ER+ CIS GA |
| SEQ IDn°37 | | MEDIAL GA |
| SEQ IDn°38 | MLVMPQPPKP FNTITITIMI AFTFFLLFLT GFLQFPSISP S | Trans GA |
| SEQ IDn°39 | | Trans GA |

### Example 3: Prevention of the addition of immunogenic residue on N-glycans by storage of recombinant protein within the early secretory pathway compartment by using targeting sequence.

The structural analysis of plant ER-resident proteins has shown that they bear exclusively high-mannose-type N-glycans (Navazio *et al.,* 1997 (**ref**. 41), 1998 (**ref**. 42); Pagny *et al*., 2000 (**ref**. 49)). These oligosaccharide structures are common to plants and mammals, and therefore are not immunogenic. This observation has suggested a strategy to prevent the association of immunogenic residues such beta1,2 xylose or alpha1,3 fucose to plant-made pharmaceuticals (PMPs) N-glycans. This strategy consists in the storage of recombinant proteins within the ER, i.e., upstream of Golgi cisternae, where immunogenic glyco-epitopes are added to maturing plant N-glycans. It was first shown that the addition of H/KDEL amino acid sequences at the C-terminal end of a recombinant soluble protein is sufficient for its retention in the plant ER (Gomord *et al.,* 1997 (**ref**. 24), 1999 (**ref**. 22)).

In the present example, using the same strategy, KDEL-ER signal sequence was fused to both heavy and light chains of the antibody of two different antibodies.

The sequence SEQ ID N° 1 to 8 have been used to target heavy and light chain of the antibodies to the ER.

The sequence SEQ ID N° 32 to 36 have been used to target heavy and light chain of antibodies to the ER and GA.

These antibodies present exclusively non immunogenic high-mannose-type N-glycans (Sriraman et al., 2004 (ref. 59); Petrucelli et al., 2006 (ref. 51)), indicating a very efficient recycling based on glycan maturation limited to enzymes located in the ER and *cis*-Golgi, such as a-mannosidase I (Nebenfuhr et al., 1999 (ref. 43)). Therefore, preventing the association of immunogenic N-glycans to PMPs through the fusion to ER retention signals is possible.

### EXAMPLE B: EXPRESSION OF AN ANTIBODY OR AN ANTIBODY FRAGMENT IN THE ER AND/OR THE GA (ANNEXED FIGURE 15, LANE B)

The expression of an antibody or an antibody fragment in the ER and/or in the GA offer different strategies to improve recombinant protein production. For example it can lead to target non modified (mutated) therapeutical proteins to the ER and/or to the GA.

Two major research directions have been established since the original demonstration of a functional expression antibody in a plant. The first was the use of plants as bio-reactors for large-scale production of therapeutic antibodies or antibody fragments. In the second, antibody or an antibody fragments are expressed in a host cell to affect a physiological process by a mechanism termed immunomodulation. The potential immunomodulation was recently illustrated when the expression of an antibody specific for a herbicide was shown to confer resistance *in planta* (Almquist KC *et a*/*.* 2004, **(ref. 1)** and see Annexed figure 27).

### EXAMPLE C: EXPRESSION OF A HOMOLOGOUS OR HETEROLOGOUS ENZYME IN THE ER AND/OR GA OF PLANT CELLS AND EXPRESSION OF RECOMBINANT PROTEIN IN SAID CELLS:

### Example 1 Humanization of N-glycosylation in plant cells (annexed figure 15, lane C) by addressing mammalian glycosyltransferases in the ER/GA of plant cells.

In plants; as in other eukaryotic cells, N-glycosylation starts in the ER, with a cotranslational addition of an oligosaccharide precursor (Glc3Man9GlcNAc2) to specific asparagine residue on the nascent polypeptide. Once transferred on to the protein, and while the secreted glycoprotein is transported along the secretory pathway, the oligosaccharide undergoes several maturations resulting in complex N-glycan. Many pharmaceuticals, including antibodies used for their effector functions, such as the triggering of the immune response (Wright and Morrison, 1994 (**ref**. 64)), require glycosylation for their in *vivo* activity and stability. This is why use the potential plants can offer for the production of recombinant antibodies, it becomes necessary to inhibit these plant-specific maturations in order to obtain 'humanized' non-immunogenic N-glycans.

An attractive strategy to humanize plant N-glycans is to express mammalian glycosyltransferases in the plant, which would complete (or compete with) the endogenous machinery of N-glycan maturation in the plant Golgi apparatus. Based on these complementation strategies, the expression of human beta(1,4)- galactosyltransferase, in the Golgi of plant cells, lead to a partial humanization of plant N-glycans and, possibly, compete with the addition of beta(1,2)-xylose and alpha(1,3)-fucose.

The expression of human beta(1,4)-galactosyltransferase in alfalfa or tobacco plants, transfers galactose residues on to the terminal N-acetylglucosamine residues of plant N-glycans. However only, 30 to 40 % of N-glycans carried by glycoprotein produced in tobacco or alfalfa plants expressing this human galactosyltransferase, bear terminal N-acetyllactosamine sequences of the mammalian type (Bakker *et al.,* 2001 (ref. 2);

The human beta(1,4)-galactosyltransferase was fused with the golgi targeting signals.

Plasmids for hGalT and GNTIhGalT expression were assembled from pBLTl121 (Pagny et al., 2003 ref 50). The CaMV 35S promoter was replaced- by the alfalfa plastocyanin promoter at Hindlll-Xbal sites. The human β(1,4)-galactosyltransferase (hGalT) gene (UDP galactose: β-N-acetylglucosaminide: β(1,4)-galactosyltransferase; EC 2.4.1.22) was isolated from pUC19-hGalT with EcoRl digestion. After klenow treatment, the 1.2 kb hGalT fragment was cloned into pBLTl221 at Smal sites. A flag tag was then fused to the C-terminal end of the coding region by PCR using the FGalT forward (5'-GACTCTAGAGCGGGAAGATGAGGCTTCGGGAGCCGCTC-3' SEQ ID N° 92) and the reverse RGalTFlagStu (5'-AAGGCCTACGCTACTTGTCATCGTCATCTTTGTAGTCGCACGGTGTCC CGAAGTCCAC-3' SEQ ID N° 93) primers. R622 was then produced by cloning this Xbal-Stul fragment into the binary vector pBLTl121 under control of Plasto promotor and Nos terminator.

The first N-terminal 38 a.a. from *N. tabacum N-*acetylglucosaminyltransferase I (GNTI) corresponding to the transmembrane domain were amplified by PCR using the forward FGNT (5'-ATCGAAATCGCACGATGAGAGGGTACAAGTTTTGC-3' SEQ ID N° 94) and reverse RGNTspe (5'-CCCATGATGCGATCTGCATATTCTGACTGTGTCGC-3' SEQ ID N°95) primers and successively cloned into pGEM-T vector, and into pBLTl221 by Apal/BamHl. For the fusion between GNTI and hGalT, PCR amplification was done from pUC19-hGalT to eliminate its own TMD and create Spel and Stul sites. The forward FGalTspe (5'-GGACTAGTGCACTGTCGCTGCCCGCCTGC-3' SEQ ID N°96) and reverse RgalTFlagStu (5'-AAGGCCTACGCTACTTGTCATCGTCATCTTTGTAGTCGCACGGTGTCC CGAAGTCCAC-3' SEQ ID N°97) were used to amplify the Spel/Stul hGalT fragment.

This fragment was then cloned into pBLTI221-GNTI. Finally, digestion by the surrounding sites Xbal/Stul allowed to isolate a 1030 bp fragment and R 622 was then produced by cloning this 1030 stretch into the binary vector pBLTI121-Plasto.

Transformation of alfalfa plants was done as follow.

Alfalfa *(Medicago sativa* L.), ecotype R2336, was transformed using an *Agrobacterium tumefaciens* AGL1 and modified as follow: petiole, stem and leaf explants were co-cultured with *Agrobacterium* for 5 to 7 days. The co-culture step was performed with an undiluted culture of *Agrobacterium* at 0.8 to 1 OD and 3% sucrose (instead of 1.5% sucrose) in the SH2K medium Well-established plants resulting of embryo development of responsive explants were transferred into soil in the greenhouse and leaves were analyzed.

The analysis of N-linked glycans isolated from wild-type and GAlT or GNTI/GalT-transformed alfalfa plants was done as follow.

Proteins were extracted for 30 minutes at 4°C from 500 mg of fresh alfalfa leaves in 5 mL of extraction buffer (0.7 M Saccharose, 0.5 M Tris, 30 mM HCl, 0.1 M KCI, 2% beta-mercaptoethanol). Insoluble materiel was eliminated by centrifugation during 10 minutes, 5,000 g, at 4°C. The resulting supernatant is treated by adding 1 volume of water saturated phenol, during 30 minutes at 4°C. Then, proteins and glycoproteins contained in the phenolic fraction were precipitated, overnight, at -20°C, by 5 volumes of PB (0.1 M Ammonium acetate dissolved in Methanol). After washing the pellet with 5 mL of PB, the proteins and glycoproteins were digested by successive treatments with pepsin and PNGase A as previously described in Bakker *et al*., 2001. Then, the N-glycans were fluorescent labelled by 2-Amino Benzamide (2-AB).

MALDI-TOF mass spectra of these derivatized N-glycans were acquired on a Voyager DE-Pro MALDI-TOF instrument (Applied Biosystems, USA) equipped with a 337-nm nitrogen laser. Mass spectra were performed in the reflector, delayed extraction mode using 2, 5-dihydroxybenzoic acid (Sigma-Aldrich) as matrix.

The matrix, freshly dissolved at 5 mg.mL⁻¹ in a 70:30 % acetonitrile / 0.1% TFA, was mixed with the solubilized oligosaccharides in a ratio 1:1 (V/V). These spectra were recorded in a positive mode, using an acceleration voltage of 20,000 V with a delay time of 100 ns. They were smoothed once and externally calibrated using commercially available mixtures of peptides and proteins (Applied Biosystems). In this study, the spectra have been calibrated using des-Arg¹-Bradykinin (904.4681 Da), Angiotensin I (1296.6853), Glu¹-Fibrinopeptide B (1570.6774 Da), ACTH clip 18-39 (2465.1989) and bovine insulin (5730.6087). Laser shots were accumulated for each spectrum in order to obtain an acceptable signal to noise ratio.

As disclosed in the previously, a large panel of signals, which are used to target only glycosyltransferase activity in subcellular compartment and subdomains is disclosed. This offer a large panel to improve the efficiency of N- and O-glycosylation in host expression system and to optimize post transcriptional modification of heterologous proteins by co-expression with enzymes presented table 5

**Table 5: enzymes fused with the sequences of the present invention.**

| | |
|---|---|
| N-glycosylation | Human Beta (1,4) galactosylation |
| | Yeast Mannosyltransferase |
| | OCH1p |
| | Human GNT III |
| O-glycosylation | N-acetylglucosaminyl transferase |
| | Galactosyltransferase |
| Proteolytic cleavage | Serine proteases |
| | Cyteine proteases |
| Amidation | Oxygenese |
| | Lyase |
| Phosphorylation | Phosphorylase |
| Gamma-carboxylation | Gamma carboxylase |
| Proteoglycan modif | Glycotransferase |
| | Glycosidase |
| Sulfation | Sulfatase |
| Hydroxylation | Hydroxylase |
| Acetylation | Acetylase |
| Cell wall polysaccharides modif. | Glycotransferase |
| | Glycosidase |

As disclose in the previous examples, the present invention provide a method for producing protein, for modifying expressing protein in subcompartment of plant cells, for expressing heterologous proteins in the RE and/or GA of plant cells. The invention provides also post transcriptional modified proteins.

### EXAMPLE D: EXPRESSION OF ZERA® PROTEIN FUSED WITH SIGNAL SEQUENCE.

In the present example, a fused protein comprising ZERA® and sequence signal (SEQ ID N° 8) and mannosidase I was made in order to accumulate the glycosidase as a membrane protein in the protein bodies. The targeting signal (SEQ IDn°8) was used to accumulate the enzyme in the membrane ER and allow the production of protein bodies by forming aggregates via the ZERA peptide.

The aim was to accumulate, in protein bodies, glycoprotein harbouring N-glycan ( Man5GlcNAc2)

Plasmide Construct: The ADNc encoding the ZERA fused to the human mannosidase I (Acess N°Q9UKM7) was amplified by PCR and sub-cloned in the pBLTl121 containing the targeting signal (SED IDn°8) at the SPel and SacI endonucleases sites.

*Agrobacterium*-mediated tobacco BY-2 cell transformation

pVKH18En6-mRFP, PBLTI121-GFP and pBIN20-GFP-fusions were transferred into *Agrobacterium tumefaciens* (strain GV3101 pMP90, Koncz and Schell, 1986) by heat shock. Transgenic *Agrobacterium* were selected onto YEB medium (per liter, beef extract 5 g, yeast extract 1 g, sucrose 5 g, MgSO₄-7H₂O 0.5 g) containing kanamycin (100 mg.mL⁻¹) and gentamycin (10 mg.mL⁻¹) and were used to transform *Nicotiana tabacum* (c.v. Bright Yellow-2) BY-2 cells, as described in Gomord *et al.,* 1998. Transformed tobacco cells were selected in the presence of kanamycin (100 mg.mL⁻¹) for PBLTI121-GFP and pBlN20-GFP-fusions or hygromycin (40 mg.mL⁻¹) for pVKH18En6-mRFP and cefotaxime (250 mg.mL⁻¹). For the double transformants coexpressing GFP and mRFP fusions, microcalli were first selected onto kanamycin plates, and were then transferred onto hygromycin plates. After screening, calli expressing the GFP and or mRFP-fusions were used to initiate suspension cultures of transgenic cells. 3-4 days old BY-2 suspension-cultured cells were used for experiments.

The fused proteins made in the present exemples was express in the transformed cells and accumulate the enzyme in the membrane ER and allow the production of protein bodies by forming aggregates via the ZERA peptide.

As diclose in the previous example, the present inventio permit to target proteins to specific domain of cells, to increase the yield of production of recombinant polypeptides, to prevent immunogenicity of recombinant polypeptides and to obtain therapeutically active recombinant polypeptides that are the exact copy of their natural counterpart. It also permits to produce post transcriptional modified proteins

### References

ref 1 Almquist, Kurt C. Yongqing Niu, Michael D. McLean, Frank L. Mena, Kerrm Y. F. Yau, Kirk Brown, Jim E. Brandle, J. Christopher Hall (2004) Immunomodulation confers herbicide resistance in plants Plant Biotechnology Journal 2 (3), 189-197.
ref 2 Bakker Hans, Muriel Bardor, Jos W. Molthoff, Véronique Gomord, Ingrid Elbers, Lucas H. Stevens, Wilco Jordi, Arjen Lommen, Loïc Faye, Patrice Lerouge, and Dirk Bosch (2001) Galactose-extended glycans of antibodies produced by transgenic plants PNAS February 27, 2001 vol. 98 no. 5 2899-2904
ref 3 Barrieu François and Maarten J. Chrispeels (1999) Delivery of a Secreted Soluble Protein to the Vacuole via a Membrane Anchor. Plant Physiol. (1999) 120: 961-968
ref 4 Benghezal, M., Wasteneys, G. O., and Jones, D. A. 2000. The Cterminal dilysine motif confers endoplasmic reticulum localisation to type I membrane proteins in plants. Plant Cell 12:1179-1201.
ref 5 Boevink, P., Oparka, K., Santa Cruz, S., Martin, B., Betteridge, A. and Hawes, C. (1998). Stacks on tracks: the plant Golgi apparatus traffics on an actin/ER network. Plant J. 15, 441-447.
ref 6 Boisson, M., Gomord, V., Audran, C., Berger, N., Dubreucq, B., Granier, F., Lerouge, P., Faye, L., Caboche, M. and Lepiniec, L. (2001). Arabidopsis glucosidase I mutants reveal a critical role of N-glycan trimming in seed development. EMBO J. 20, 1010-1019.
ref 7 Bracha K, Lavy M, Yalovsky S. The Arabidopsis AtSTE24 is a CAAX protease with broad substrate specificity.J Biol Chem. 2002 Aug 16;277(33):29856-64.
ref 8 Brandizzi, F., Frangne, N., Marc-Martin, S., Hawes, C., Neuhaus, J-M. and Paris, N. (2002a). The destination for single-pass membrane proteins is influenced markedly by the length of the hydrophobic domain. Plant Cell, 14, 1077-1092.
ref 9 Brandizzi, F., Snapp, E., Roberts, A., Lippincott-Schwartz, J. and Hawes, C. (2002b). Membrane protein transport between the ER and Golgi in tobacco leaves is energy dependent but cytoskeleton independent: evidence from selective photobleaching. Plant Cell 14, 1293-1309.
ref 10 Bretscher, M.S. and Munro, S. (1993). Cholesterol and the Golgi apparatus. Science 261, 1280-1281.
ref 11 Campbell, R. E., Tour, O., Palmer, A.E., Steinbach, P.A., Baird, G.S., Zacharias, D.A. and Tsien, R. Y. (2002). A monomeric red fluorescent protein. Proc. Natl. Acad. Sci. USA 99, 7877-7882.
ref 12 Ciczora Yann 1, Nathalie Callens1, Claire Montpellier1, Birke Bartosch2, François-Loïc Cosset2, Anne Op De Beeck1.† and Jean Dubuisson1 Contribution of the charged residues of hepatitis C virus glycoprotein E2 transmembrane domain to the functions of the E1 E2 heterodimer. J Gen Virol 86 (2005), 2793-2798;
ref 13 Contreras, Elena Ortiz-Zapater, Fernando Aniento (2004) Sorting signals in the cytosolic tail of membrane proteins involved in the interaction with plant ARF1 and coatomer The Plant Journal 38 (4), 685-698
ref 14 De Praeter, C. M., Gerwig, G. J., Bause, E., Nuytinck, L. K., Vliegenthart, J. F., Breuer, W., Kamerling, J. P., Espeel, M. F., Martin, J. J., De Paepe, A. M., et al. (2000). A novel disorder caused by defective biosynthesis of N-linked oligosaccharides due to glucosidase I deficiency. Am J Hum Genet 66, 1744-1756.
ref 15 Dirnberger, D, Bencur, P, Mach, L. and Steinkellner, H. (2002). The Golgi localization of Arabidopsis thaliana beta1,2-xylosyltransferase in plant cells is dependent on its cytoplasmic and transmembrane sequences. Plant Mol. Biol. 50, 273-281.
ref 16 Essl, D., Dirnberger, D., Gomord, V., Strasser, R., Faye, L., Glössl, J. and Steinkellner, H. (1999). The N-terminal 77 amino acids from tobacco N-acetylglucosaminyltransferase I are sufficient to retain a reporter protein in the Golgi apparatus of Nicotiana benthamiana cells. FEBS Lett. 453, 169-173.
ref 17 Faye Loïc, Aurelia Boulaflous, Meriem Benchabane, Véronique Gomorda and Dominique Michaud Protein modifications in the plant secretory pathway: current status and practical implications in molecular pharming Volume 23, Issue 15, 7 March 2005, Pages 1770-1778
ref 18 Füllekrug, J. and Nilsson, T. (1998). Protein sorting in the Golgi complex. Biochim. Biophys. Acta 1404, 77-84.
ref 19 Gillmor, C. S., Poindexter, P., Lorieau, J., Palcic, M. M., and Somerville, C. (2002). Alpha-glucosidase I is required for cellulose biosynthesis and morphogenesis in Arabidopsis. J Cell Biol 156, 1003-1013.
ref 20 Giraudo, C.D. and Maccioni, H.J.F. (2003). Endoplasmic reticulum export of glycosyltransferases depends on interaction of a cytoplasmic dibasic motif with Sar1. Mol. Biol. Cell 14, 3753-3766.
ref 21 Gomord, V., and Faye, L. (2004). Posttranslational modification of therapeutic proteins in plants. Curr Opin Plant Biol 7, 171-181.
ref 22 Gomord V, Wee E. and Faye L. (1999). Protein retention and localization in the endoplasmic reticulum and the Golgi apparatus. Biochimie, 81, 607-618
ref 23 Gomord V, Sourrouille C., Fichette A.C., Barbor M. Pagny S., Lerouge P., and Faye L. (2004) Production and glycosylation of plant made pharmaceuticals: the antibodies as a challenge. Plant Biotechnol., 2, 83-100.
ref 24 Gomord V., Denmat L.A., Fitchette-Laine A.C., Satiat-jeunemaitre, Hawes C. and Faye L. (1997). The C-terminal HDEL sequence is sufficient for retention of secretory proteins but promotes vacuolar targeting of proteins that escape the endoplasmic reticulum. Plant J., 11,313-326
ref 25 Gomord, V., Fitchette, A.C., Denmat, L.A., Michaud, D. and Faye, L. (1998). Production of foreign proteins in tobacco cell suspension culture. In Methods in Molecular Biotechnology, Vol 3 (Cunningham C. and Porter A.J.R. Eds.) Totowa: Humana Press Inc., pp. 155-164.
ref 26 Grove, S.N., Brecker, C.E. and Morré, D.J. (1968). Cytomembrane differentiation in the endoplasmic reticulum-Golgi apparatus-vesicle complex. Science 161, 171-173.
ref 27 Hanton SL, Renna L, Bortolotti LE, Chatre L, Stefano G, Brandizzi F. (2005). Diacidic motifs influence the export of transmembrane proteins from the endoplasmic reticulum in plant cells. Plant Cell. 2005 17, 3081-3093.
ref 28 Hardt, B., Kalz-Füller, B., Aparicio, R., Völker, C. and Bause, E. (2003). (Arg)3 within the N-terminal domain of glucosidase I contains ER targeting information but is not required absolutely for ER localisation. Glycobiology, 13, 159-168.
ref 29 Hartmann, M.A. and Benveniste, P. (1987). Plant membrane sterols: isolation, identification and biosynthesis. Methods Enzymol. 148: 632-650.
ref 30 Hong, Y., Sundaram, S., Shin, D.-J., and Stanley, P. (2004). The Lec23 Chinese Hamster Ovary Mutant Is a Sensitive Host for Detecting Mutations in {alpha}-Glucosidase I That Give Rise to Congenital Disorder of Glycosylation IIb (CDG IIb)10.1074/jbc.M410121200. J Biol Chem 279, 49894-49901.
ref 31 Janzik I, Macheroux P, Amrhein N, Schaller A LeSBT1, a subtilase from tomato plants. Overexpression in insect cells, purification, and characterization.J Biol Chem. 2000 Feb 18;275(7):5193-9
ref 32 Lienard D., Tran Dinh O., E. van Oort, L. Van Overtvelt, C. Bonneau, E. Wambre, M. Bardor, P. Cosette, A. Didier-Laurent, F. Dorlhac de Borne, R. Delon, R. van Ree, P. Moingeon, L. Faye and V. Gomord (2007) Suspension-cultured BY-2 tobacco cells produce and mature immunologically active house dust mite allergens... * DL and OTD have equal contributions to this work, Plant biotechnol. J. 5, 93-108
ref 33 Lynch, D.V. (1993). Sphingolipids. In Lipid Metabolism in Plants (Moore T.S. Ed.) Boca Raton, FL: CRC Press, pp. 285-308.
ref 34 Masibay, A.S., Balaji, P.V., Boeggeman, E.E. and Qasba, P.K. (1993). Mutational analysis of the Golgi retention signal of bovine b-1,4-galactosyltransferase. J. Biol. Chem. 268, 9908-9916.
ref 35 McCartney, A. W., Dyer, J. M., Dhanoa, P. K., Kim, P. K., Andrews, D. W., McNew, J. A., and Mullen, R. T. (2004). Membrane-bound fatty acid desaturases are inserted co-translationally into the ER and contain different ER retrieval motifs at their carboxy termini. Plant J 37, 156-173.
ref 36 Moreau, P., Hartmann, M.A., Perret, A.M., Sturbois-Balcerzak, B. and Cassagne, C. (1998). Transport of sterols to the plasma membrane of leek seedlings. Plant Physiol. 117, 931-937.
ref 37 Morré, D.J. and Mollenhauer, H.H. (1974). The endomembrane concept: a functional integration of endoplasmic reticulum and Golgi apparatus. In Dynamic Aspects of Plant Ultrastructure. (Robards A.W. Ed.) London: McGraw-Hill Book Cy, pp. 84-137.
ref 38 Munro, S. (1991). Sequences within and adjacent to the transmembrane segment of b-2,6-sialyltransferase specify Golgi retention. EMBO J. 10, 3577-3588. Munro, S. (1995a). A comparison of the transmembrane domains of Golgi and plasma membrane proteins. Biochem. Soc. Trans. 23, 527-530.
ref 39 Munro, S. (1995b). An investigation of the role of the transmembrane domain in Golgi protein retention. EMBO J. 14, 4695-4704.
ref 40 Munro, S. (1998). Localization of proteins to the Golgi apparatus. Trends Cell Biol. 8, 11-15.
ref 41 Navazio L., Sponga L., Damese P., Fitchette-Laine A-C., Faye L., Baldan B. and mariani P. (1997). The calcium binding protein calreticulin in pollen of Liriodendron tulipifera L. Plant Sci., 131, 35-42
ref 42 Navazio L., Nardi C., Pancaldu C., Danese P., Baldan B., Fitchette-Laine A.C., Faye L., Meggip F., Martin W. and Mariani P. (1998). Functional conservation of calreticulin in euglena gracilis. J. Euk. Microbiol., 45, 307-313.
ref 43 Nebenführ, A., Gallagher, L.A., Dunahay, T.G., Frohlick, J.A., Mazurkiewicz, A.M., Meehl, J.B. and Staehelin, L.A. (1999). Stop-and-go movements of plant Golgi stacks are mediated by the actomyosin system. Plant Physiol. 121, 1127-1142.
ref 44 Nilsson, T., Pypaert, M., Hoe, M.H., Slusarewicz, P., Berger, E.G. and Warren, G. (1993a). Overlapping distribution of two glycosyltransferases in the Golgi apparatus of HeLa cells. J. Cell Biol. 120, 5-13.
ref 45 Nilsson, T., Slusarewicz, P., Hoe, M.H. and Warren, G. (1993b). Kin recognition: A model for the retention of Golgi enzymes. FEBS Lett. 330, 1-4.
ref 46 Nilsson, T., Hoe, M.H., Slusarewicz, P., Rabouille, C., Watson, R., Hunte, F., Watzele, G., Berger, E.G. and Warren, G. (1994). Kin recognition between medial Golgi enzymes in HeLa cells. EMBO J. 13, 562-574.
ref 47 Nilsson, T., Rabouille, C., Hui, N., Watson, R. and Warren, G. (1996). The role of the membrane spanning domain and stalk region of N-acetylglucosaminyltransferase I in retention, kin recognition and structural maintenance of the Golgi apparatus in HeLa cells. J. Cell Sci. 109, 1975-1989.
ref 48 Opat, A.S., Houghton, F. and Gleeson, P.A. (2000). Medial Golgi but not late Golgi glycosyltransferases exist as high molecular weight complexes. J. Biol. Chem. 275, 11836-11845.
ref 49 Pagny S., Cabanes-Macheteau M., Gillikin J.W.; Leborgne-Castel N., Lerouge P., Boston R. Faye L. and Gomord V. (2000). Protein recycling from the Golgi apparatus to the endoplasmic reticulum in plants and its minor contribution to calreticulin retention. Plant Cell, 12, 739-755
ref 50 Pagny, S., Bouissonié, F., Sarkar, M., Follet-Gueye, M.L., Driouich, A., Schachter, H., Faye, L. and Gomord, V. (2003). Structural requirements for Arabidopsis b-1,2-xylosyltransferase activity and targeting to the Golgi. Plant J. 33, 189-203.
ref 51 Petruccelli S., Otegui MS., Lareu F., Trandinhthanhlien O., Fitchette AC., Circosta A., Rumbo M., Bardor M., Carcamo R., Gomord V. and Beachy RN. A KDEL tagged monoclonal antibody is efficiently retained in the reticulum endoplasmic in leaves but is both partially secreted and sorted to protein storage vacuoles in seeds. Plant Biotechnol. J., 4, 511-527)
ref 52 Rautengarten C, Steinhauser D, Bussis D, Stintzi A, Schaller A, Kopka J, Altmann T Inferring hypotheses on functional relationships of genes: Analysis of the Arabidopsis thaliana subtilase gene family. PLoS Comput Biol. 2005 Sep;1(4):e40. Epub 2005 Sep 2
ref 53 Reyes, F., Marchant, L., Norambuena, L., Nilo, R., Silva, H., and Orellana, A. (2006). AtUTr1, a UDP-glucose/UDP-galactose transporter from Arabidopsis thaliana, is located in the endoplasmic reticulum and up-regulated by the unfolded protein response. J Biol Chem 281, 9145-9151.
ref 54 Ritzenthaler, C., Nebenfuhr, A., Movafeghi, A., Stussi-Garaud, C., Behnia, L., Pimpl, P., Staehelin, L.A. and Robinson, D.G. (2002). Reevaluation of the effects of brefeldin A on plant cells using tobacco Bright Yellow 2 cells expressing Golgi-targeted green fluorescent protein and COPI antisera. Plant Cell. 14, 237-261.
ref 55 Runions J., Brach T., Külner S and Hawes C. (2006). Photoactivation of GFP reveals protein dynamics within the endoplasmic reticulum membrane. J. Exp. Bot. 57, 43-50.
ref 56 Saint-Jore, C.M., Evins, J., Batoko, H., Brandizzi, F., Moore, I. and Hawes, C. (2002). Redistribution of membrane proteins between the Golgi apparatus and endoplasmic reticulum in plants is reversible and not dependent on cytoskeletal networks. Plant J., 29, 661-678.
ref 57 Saint-Jore-Dupas C., Gomord V. and Paris N. (2004). Protein localization in the Golgi apparatus and the trans-Golgi network. Cell Mol. Life Sci., 61, 159-171.
ref 58 Saint-Jore-Dupas C., Nebenführ A., Follet-Gueye ML., Boulaflous A., Hawes C., Driouich A., Faye L. and Gomord V., (2006) Partitioning of N-glycan maturation enzymes along the secretory pathway and a key role of transmembrane domain length in early-Golgi targeting. Plant Cell 18, 3182-3200
ref 59 Sriraman R., Bardor M., Sack M., Vaquero C., Faye L., Fischer R., Finnern R, Lerouge P. (2004) Recombinant anti-hCG antibodies retained in the endoplasmic reticulum of transformed plants lack core-xylose and core-alpha(1,3)-fucose residues. Plant Biotechnol;, 2, 279-287
ref 60 Strasser, R., Mucha, J., Schwihla, H., Altmann, F., Glössl, J. and Steinkellner, H. (1999). Molecular cloning and characterisation of cDNA coding for b-1,2-N-acetylglucosaminyltransferase I (GlcNAc-TI) from Nicotiana tabacum. Glycobiology 9, 779-785.
ref 61 Strasser, R., Schoberer J., Jin C., Mucha, J., Glössl, J., Mach L. and Steinkellner, H. (2006). Molecular cloning and characterisation of Arabidopsis thaliana Golgi a-mannosidase II, a key enzyme in the formation of complexN-glycans in plants. Plant J. 45, 789-803
ref 62 Teasdale, R.D. and Jackson, M.R. (1996). Signal-mediated sorting of membrane proteins between the endoplasmic reticulum and the Golgi apparatus. Ann. Rev. Cell Develop. Biol. 12, 27-54.
ref 63 Wee, E.G.T., Sherrier, J., Prime, T. and Dupree, P. (1998). Targeting of active sialyltransferase to the plant Golgi apparatus. Plant Cell 10, 1759-1768.
ref 64 Wright A, Morrison SL. Effect of altered CH2-associated carbohydrate structure on the functional properties and in vivo fate of chimeric mouse-human immunoglobulin G1.J Exp Med. 1994 Sep 1;180(3):1087-96
ref 65 Yuasa, K., Toyooka, K., Fukuda, H. and Matsuoka, K.(2005). Membrane-anchored prolyl hydroxylase with an export signal from the endoplasmic reticulum. Plant J. 41, 81-94.
ref 66 Zerangue, N., Schwappach, B., Jan, Y.N. and Jan, L.Y. (1999). A new ER trafficking signal regulates the subunit stoichiometry of plasma membrane K (ATP) channels. Neuron 22, 537-548.

## Claims

1. An amino-acid sequence selected from the group consisting of SEQ ID N°1, SEQ ID N°4 and SEQ ID N°8.

2. A recombinant protein comprising an amino-acid sequence according to claim 1 and a protein, said amino-acid sequence being fused to the C-terminal or N-terminal extremity of the protein.

3. The recombinant protein according to claim 2, wherein the protein selected from the group comprising an enzyme, an antibody or part thereof, a reporter protein, a recombinant protein, a therapeutically active protein.

4. The recombinant protein according to claim 2, wherein protein is an enzyme, said enzyme being selected from the group comprising glycosidase, glycosyl transferases, protease, kinase, decarboxylase, epimerase, nucleotide-sugar transporter.

5. A nucleic acid sequence encoding an amino-acid sequence according to claim 1 or a recombinant protein according to claim 3 or 4.

6. A nucleic acid vector comprising the nucleic acid sequence according to claim 5.

7. A plant cell comprising at least: one amino-acid sequence according to claim 1 and/or one recombinant protein according to claim 3 or 4 and/or one nucleic acid vector according to claim 6.

8. A plant cell according to claim 7, wherein said plant cell comprises at least one protein according to claim 3 or 4, said protein being a heterologous protein.

9. A plant comprising at least: one amino-acid sequence according to claim 1 and/or one recombinant protein according to claim 3 or 4 and/or one nucleic acid vector according to claim 6.

10. A plant according to claim 9, wherein said plant comprises at least one protein according to claim 3 or 4, said protein being a heterologous protein.

11. A plant according to claim 9 or 10, said plant being selected from the group comprising Alfalfa, Arabidospsis thaliana, Nicotiana tabacum, Glycine max, Lycopersicon esculentum, Solanum lycopersicum.

12. A method for producing a post-translationally modified polypeptide comprising the steps of:
- transfecting or transforming a plant cell with at least one nucleic acid vector according to claim 6, said vector encoding a recombinant protein which is the polypeptide before being post-translationally modified or a recombinant protein different to said polypeptide;
- growing the transfected plant cell; and
- harvesting the post-translationally modified polypeptide;
wherein, when said recombinant protein is different to said polypeptide, the method also comprises a step of transfecting said plant cell with at least one nucleic acid vector encoding said polypeptide.

13. The method of claim 12, wherein the recombinant protein is the polypeptide before being post-translationally modified.

14. The method of claim 12, wherein the recombinant protein is different to said polypeptide, and wherein said recombinant protein is an antibody or part thereof recognizing and binding specifically said polypeptide.

15. The method of claim 12, wherein the recombinant protein is different to said polypeptide, and wherein said recombinant protein is an endogenous or heterologous enzyme involved in the post-translational modification of said polypeptide.

16. The method of claim 12, wherein the recombinant protein is different to said polypeptide, and wherein said recombinant protein is an antibody or part thereof modulating an enzyme involved in the post-translational modification of said polypeptide.

17. The method of anyone of claim 12 to 16, wherein polypeptide is co-expressed with a storage protein.

18. The method according to anyone of claims 12 to 17, wherein the post-translational modification of the peptide is carried out in the Endoplasmic Reticulum (ER) and/or Golgi Apparatus (GA) compartment membranes.

19. The method according to anyone of claims 12 to 18, wherein said post-translationally modified polypeptide is a therapeutically active protein.

20. The method according to anyone of claims 12 to 19, wherein the plant cells are cells pertaining to a plant selected from the group comprising Medicago sativa, Arabidospsis thaliana, Nicotiana tabacum, Glycine max, Lycopersicon esculentum, Solanum lycopersicum.

21. Use of an amino acid sequence according to claim 1 for allowing the retention of a recombinant polypeptide fused with said amino acid sequence under a membrane bound form in the endoplasmic reticulum.

22. Use according to claim 21, wherein the recombinant polypeptide is a membrane protein and the amino acid sequence allows anchoring of the membrane protein in the endoplasmic reticulum.

## Patentansprüche

1. Ein Aminosäuresequenz aus einer Gruppe bestehend aus SEQ ID NO 1, SEQ ID NO 4 und SEQ ID NO 8.

2. Ein rekombinantes Protein, das eine Aminosäuesequenz nach Anspruch 1 und ein Protein umfasst, wobei die Aminosäuresequenz mit dem C-Terminus oder dem N-Terminus des Proteins verschmolzen ist.

3. Rekombinantes Protein nach Anspruch 2, wobei das Protein aus einer Gruppe ausgewählt wird, die ein Enzym, einen Antikörper oder einen Teil hiervon, ein Reporter-Protein, ein rekombinantes Protein, ein therapeutisch aktives Protein umfasst.

4. Rekombinantes Protein nach Anspruch 2, wobei das Protein ein Enzym ist, das aus der Gruppe ausgewählt wird, die Glycosidase, Glycosyltransferasen, Protease, Kinase, Decarboxylase, Epimerase, Nukleotid-Zucker-Transporter umfasst.

5. Eine Nukleinsäuresequenz, die eine Aminosäuresequenz nach Anspruch 1 oder ein rekombinantes Protein nach Anspruch 3 oder 4 codiert.

6. Ein Nukleinsäurevektor, der die Nukleinsäuresequenz nach Anspruch 5 umfasst.

7. Eine Pflanzenzelle, die mindestens eine Aminosäuresequenz nach Anspruch 1 und/oder ein rekombinantes Protein nach Anspruch 3 oder 4 und/oder einen Nukleinsäurevektor nach Anspruch 6 umfasst.

8. Eine Pflanzenzelle nach Anspruch 7, wobei die Pflanzenzelle mindestens ein Protein nach Anspruch 3 oder 4 umfasst, wobei das Protein ein heterologes Protein ist.

9. Eine Pflanze, die mindestens Folgendes umfasst: eine Aminosäuresequenz nach Anspruch 1 und/oder ein rekombinantes Protein nach Anspruch 3 oder 4 und/oder einen Nukleinsäurevektor nach Anspruch 6.

10. Eine Pflanze nach Anspruch 9, wobei die Pflanze mindestens ein Protein nach Anspruch 3 oder 4 umfasst, wobei das Protein ein heterologes Protein ist.

11. Eine Pflanze nach Anspruch 9 oder 10, wobei die Pflanze aus der Gruppe ausgewählt wird, die Alfalfa, Arabidospsis thaliana, Nicotiana tabacum, Glycine max, Lycopersicon esculentum, Solanum lycopersicum umfasst.

12. Ein Verfahren zur Herstellung eines posttranslational modifizierten Polypetptids mit folgenden Schritten:
- Transfektion oder Transformation einer Pflanzenzelle mit mindestens einem Nukleinsäurevektor nach Anspruch 6, wobei der Vektor ein rekombinantes Protein codiert, das ein Polypeptid vor der posttranslationalen Modifizierung oder ein im Bezug auf das Polypeptid anderes rekombinantes Protein ist;
- Züchten der transfektierten Pflanzenzelle; und
- Ernten des posttranslational modifizierten Polypeptids;
wobei, wenn das rekombinante Protein sich von dem Polypeptid unterscheidet, das Verfahren ferner einen Schritt der Transkfektion der Pflanzenzelle mit mindestens einem das Polypeptid codierenden Nukleinsäurevektor umfasst.

13. Verfahren nach Anspruch 12, In welchem das rekombinante Protein ein Polypeptid vor der posttranslationalen Modifikation ist.

14. Verfahren nach Anspruch 12, in welchem das rekombinante Protein sich von dem Polypeptid unterscheidet und in welchem das rekombinante Polypeptid ein Antikörper oder ein Teil hiervon ist, der das Polypeptid spezifisch bindet oder erkennt.

15. Verfahren nach Anspruch 12, in welchem das rekombinante Protein sich von dem Polypeptid unterscheidet und in welchem das rekombinante Protein ein endogenes oder heterologes Enzym ist, das an der posttranslationalen Modifizierung des Polypeptids beteiligt ist.

16. Verfahren nach Anspruch 12, in welchem das rekombinante Protein sich von dem Polypeptid unterscheidet und in welchem das rekombinante Protein ein Antikörper oder ein Teil hiervon ist, das ein an der posttranslationalen Modifizierung des Polypeptids beteiligtes Enzym moduliert.

17. Verfahren nach einem beliebigen der Ansprüche 12 bis 16, in welchem das Polypeptid mit einem Speicherprotein co-exprimiert wird.

18. Verfahren nach einem beliebigen der Ansprüche 12 bis 17, in welchem die posttranslationale Modifizierung des Peptids in dem Membrankompartiment des endoplasmatischen Retikulums (ER) oder des Golgi-Apparats (GA) stattfindet.

19. Verfahren nach einem beliebigen der Ansprüche 12 bis 18, in welchem das posttranslational modifizierte Polypeptid ein therapeutisch aktives Protein ist.

20. Verfahren nach einem beliebigen der Ansprüche 12 bis 19, in welchem die Pflanzenzellen Zellen von Pflanzen sind, die aus der Gruppe gewählt werden, die Medicago sativa, Arabidospsis thaliana, Nicotiana tabacum, Glycine max, Lycopersicon esculentum, Solanum lycopersicum umfasst.

21. Verwendung einer Aminosäuresequenz nach Anspruch 1, um die Retention eines mit der Aminosäuresequenz verschmolzenen rekombinanten Proteins in einer im endoplasmatischen Retikulum membrangebundenen Form zu ermöglichen.

22. Verwendung nach Anspruch 21, wobei das rekombinante Polypeptid ein Membranprotein ist und die Aminosäuresequenz die Verankerung des Membranproteins in dem endoplasmatischen Retikulum ermöglicht.

## Revendications

1. Séquence d'acides amines choisie dans le groupe constitué de SEQ ID N°1, SEQ ID N°4 et SEQ ID N°8.

2. Protéine recombinante comprenant une séquence d'acides aminés selon la revendication 1 et une protéine, ladite séquence d'acides aminés étant fusionnée à l'extrémité C- ou N-terminale de la protéine.

3. Protéine recombinante selon la revendication 2, dans laquelle la protéine est choisie dans le groupe comprenant une enzyme, un anticorps ou une portion de celui-ci, une protéine rapporteuse, une protéine recombinante, une protéine thérapeutiquement active.

4. Protéine recombinante selon la revendication 2, dans laquelle la protéine est une enzyme, ladite enzyme étant choisie dans le groupe comprenant une glycosidase, les glycosyl transférases, une kinase, une décarboxylase, une épimérase, un transporteur nucléotide-sucre.

5. Séquence d'acide nucléique codant une séquence d'acide aminé selon la revendication 1 ou une protéine recombinante selon la revendication 3 ou 4.

6. Vecteur acides nucléique comprenant la séquence d'acides nucléique selon la revendication 5.

7. Cellule de plante comprenant au moins une séquence d'acide amine selon la revendication 1 et/ou une protéine recombinante selon la revendication 3 ou 4 et/ou un vecteur d'acides nucléiques selon la revendication 6.

8. Cellule de plante selon la revendication 7, dans laquelle ladite cellule de plante comprend au moins une protéine selon la revendication 3 ou 4, ladite protéine étant une protéine hétérologue.

9. Plante comprenant au moins une séquence selon la revendication 1 et/ou une protéine recombinante selon la revendication 3 ou 4 et/ou un vecteur d'acides nucléiques selon la revendication 6.

10. Plante selon la revendication 9, dans laquelle ladite plante comprend au moins une protéine selon la revendication 3 ou 4, ladite protéine étant une protéine hétérologue.

11. Plante selon la revendication 9 ou 10, ladite plante étant choisie dans le groupe comprenant Alfalfa, Arabidospsis thaliana, Nicotiana tabacum, Glycine max, Lycopersicon esculentum, Solanum lycopersicum.

12. Procédé de production d'un polypeptide modifié post-traduction comprenant les étapes de:
- transfection ou transformation d'une cellule de plante avec au moins un vecteur d'acides nucléiques selon la revendication 6, ledit vecteur codant pour une protéine recombinante qui est le polypeptide avant modification post-traductionnelle ou une protéine recombinante différente dudit polypeptide;
- croissance de la cellule de plante transfectée; et
- récupération du polypeptide modifié post-traductionnellement;
dans lequel, lorsque la protéine recombinante est différente dudit polypeptide, le procédé comprend également une étape de transformation de ladite cellule de plante avec au moins un vecteur d'acides nucléiques codant ledit polypeptide.

13. Procédé selon la revendication 12; dans lequel la protéine recombinante est le polypeptide avant modification post-traductionnelle.

14. Procédé selon la revendication 12; dans lequel la protéine recombinante est différente dudit polypeptide, et dans lequel ladite protéine recombinante est un anticorps ou une partie de celui-ci reconnaissant ou liant spécifiquement ledit polypeptide.

15. Procédé selon la revendication 12; dans lequel la protéine recombinante est différente dudit polypeptide, et dans lequel ladite protéine recombinante est une enzyme endogène ou hétérogène impliquée dans la modification post-traductionnelle dudit polypeptide.

16. Procédé selon la revendication 12; dans lequel la protéine recombinante est différente dudit polypeptide, et dans lequel ladite protéine recombinante est un anticorps ou une partie de celui-ci modulant une enzyme impliquée dans la modification post-traductionnelle dudit polypeptide.

17. Procédé selon l'une quelconque des revendications 12 à 16, dans lequel le polypeptide est co-exprimé avec une protéine de stockage.

18. Procédé selon l'une quelconque des revendications 12 à 17, dans lequel la modification post-traductionnel du peptide est réalisée dans le compartiment membranaire du Réticulum Endoplasmique (RE) et/ou l'Appareil de Golgi (AG).

19. Procédé selon l'une quelconque des revendications 12 à 18, dans lequel ledit polypeptide modifié post-traductionnellement est une protéine thérapeutiquement active.

20. Procédé selon l'une quelconque des revendications 12 à 19, dans lequel les cellules de plante sont des cellules de plantes concernant une plante choisie dans le groupe comprenant Medicago sativa, Arabidospsis thaliana, Nicotiana tabacum, Glycine max, Lycopersicon esculentum, Solanum lycopersicum.

21. Utilisation d'une séquence d'acides aminés selon la revendication 1 pour permettre la rétention d'un polypeptide recombinant fusionné avec ladite séquence d'acides aminés sous une forme liée à la membrane dans le réticulum endoplasmique.

22. Utilisation selon la revendication 21, dans lequel le polypeptide recombinant est une protéine membranaire et la séquence d'acides aminés permet l'encrage de la protéine membranaire dans le réticulum endoplasmique.
